(19) Europäisches Patentamt
European Patent Office
Office européen des brevets

(11) EP 4 365 182 A1

(12) **EUROPEAN PATENT APPLICATION**
published in accordance with Art. 153(4) EPC

(43) Date of publication:
**08.05.2024 Bulletin 2024/19**

(21) Application number: **22819640.8**

(22) Date of filing: **10.06.2022**

(51) International Patent Classification (IPC):
***C07H 19/073*** *(2006.01)* ***C07H 1/00*** *(2006.01)*
***A61K 31/7064*** *(2006.01)* ***A61P 35/00*** *(2006.01)*

(52) Cooperative Patent Classification (CPC):
**A61K 31/7064; A61P 35/00; C07H 1/00; C07H 19/073**

(86) International application number:
**PCT/CN2022/098082**

(87) International publication number:
**WO 2022/258043 (15.12.2022 Gazette 2022/50)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **11.06.2021 CN 202110653130**

(71) Applicant: **Ascentawits Pharmaceuticals, Ltd.**
**Shenzhen, Guangdong 518118 (CN)**

(72) Inventors:
- **LI, Anrong**
  **Shenzhen, Guangdong 518118 (CN)**
- **QI, Tianyang**
  **Shenzhen, Guangdong 518118 (CN)**
- **DUAN, Jianxin**
  **Shenzhen, Guangdong 518118 (CN)**
- **MENG, Fanying**
  **San Francisco, California 94121 (US)**
- **MENG, Teng**
  **Shenzhen, Guangdong 518118 (CN)**
- **ZHANG, Mengyun**
  **Shenzhen, Guangdong 518118 (CN)**

(74) Representative: **Studio Torta S.p.A.**
**Via Viotti, 9**
**10121 Torino (IT)**

(54) **GEMCITABINE ANTICANCER DERIVATIVE AND ANTICANCER PHARMACEUTICAL USE**

(57) The present invention provides gemcitabine anticancer derivatives of formula (III), which have the potential to be developed into AKR1C3-activated anticancer drugs, and the anticancer pharmaceutical use thereof,.

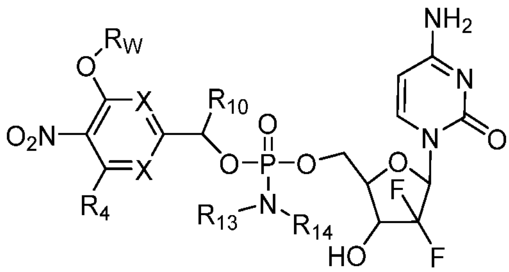

(III)

EP 4 365 182 A1

## Description

### Technical Field

The present invention, belonging to the field of research and development of anticancer drugs, relates to a series of AKR1C3 enzyme-activated gemcitabine anticancer derivatives obtained by further research and development of the compound TH3057 disclosed in Patent Application No. PCT/US2016/025665 entitled NITROBENZYL DERIVATIVES OF ANTI-CANCER AGENTS (Publication No. WO2016/161342A1), which corresponds to Chinese Patent Application No. 201680020013.2 (Publication No. CN108136214A).

### Background Art

The invention by Jian-xin Duan et al., entitled NITROBENZYL DERIVATIVES OF ANTI-CANCER AGENTS with Patent Application No. PCT/US2016/025665 and Publication No. WO2016/161342A1, corresponding to Chinese Patent Application No. 201680020013.2 with Publication No. CN108136214A, discloses a series of nitrobenzyl derivatives:

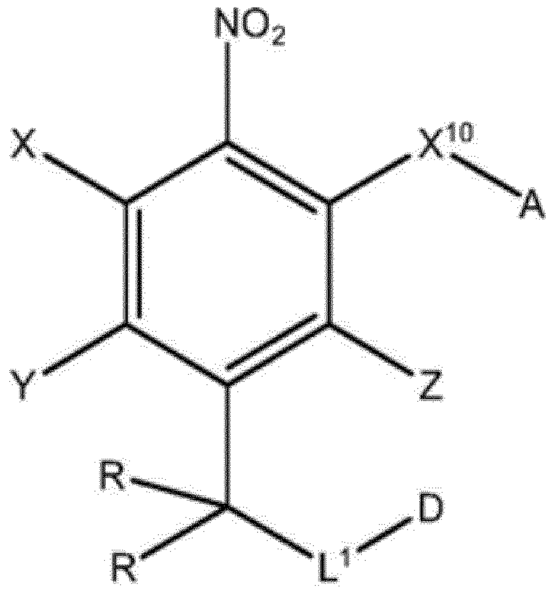

,

which actually is an AKR1C3 activated anticancer drug obtained by bonding a nitrobenzyl structure to an anticancer drug residue by $L^1$, , wherein the linker $L^1$ is defined to have different structures for different anticancer drug residues D.

In particular, in this application, the inventors designed a derivative of gemcitabine combined with a nitrobenzyl structure, which has the following structure:

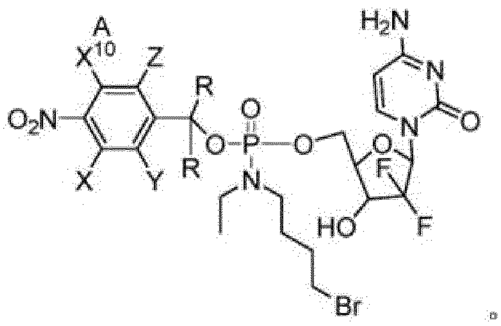

and the three specific compounds:

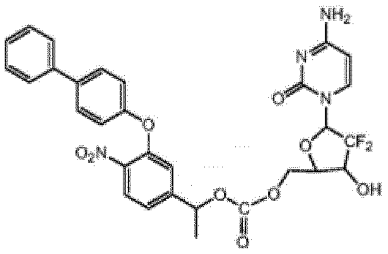,
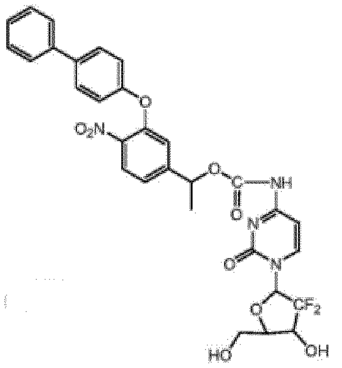 and
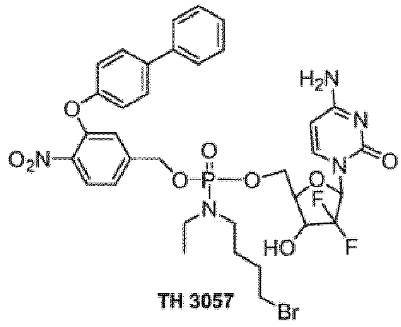
TH 3057
.

The compound TH3057 was further synthesized, and tested for its cell proliferation inhibition activity ($IC_{50}$ values) against the H460 cell line with high expression of AKR1C3 enzyme, without and with the addition of AKR1C3 enzyme inhibitor, with the $IC_{50}$ values being 0.2 $\mu$mol/L and 5 $\mu$mol/L respectively.

## Summary of the Invention

The inventors continued further research and found that not all the derivatives designed in the above invention that combine gemcitabine with a nitrobenzyl structure were AKR1C3 activated anticancer drugs, or compounds similar to TH3057. Not all the compounds obtained from the gemcitabine residue bonded to a nitrobenzyl structure by the linker $L^1$ were AKR1C3 activated anticancer drugs. They were tested to demonstrate that not all of them could be activated by AKR1C3 enzyme or to have extremely low activation ratios. Therefore, based on the compounds designed in the aforementioned invention, the inventors designed and synthesized a series of gemcitabine anticancer derivatives, which were confirmed to have the ability to be activated by AKR1C3 enzyme or have a higher activation ratio. And these compounds may have better potential to be developed as AKR1C3 activated anticancer drugs than TH3057. Therefore, the present invention provides a new possibility or idea for designing and developing AKR1C3 activated anticancer drugs with higher specific activation ratios.

The present invention provides gemcitabine anticancer derivatives with new structures, which are AKR1C3 activated anticancer drugs.

A gemcitabine derivative of formula (III), or a pharmaceutically acceptable salt, a solvate, an isotopic variant, or an isomer thereof:

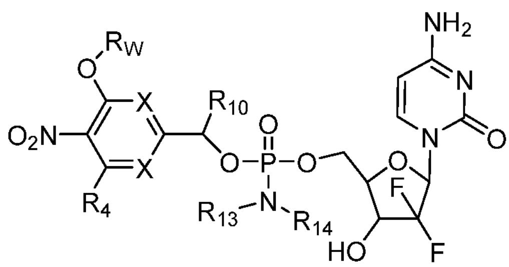
(III)

wherein,

two X's are each independently $CR^{15}$ or N;

$R_{13}$ and $R_{14}$ are each independently hydrogen; $C_1$-$C_6$ alkyl; cycloalkyl; alkenyl; alkynyl; $C_6$-$C_{20}$ aryl; 5-20-membered heterocyclyl; halogen-substituted $C_1$-$C_6$ alkyl, cycloalkyl, alkenyl or alkynyl; halogen-substituted $C_6$-$C_{20}$ aryl; or halogen-substituted 5-20-membered heterocyclyl, and $R_{13}$ and $R_{14}$ are not hydrogen at the same time;

$R_{10}$ is hydrogen; $C_1$-$C_6$ alkyl; cycloalkyl; alkenyl; alkynyl; $C_6$-$C_{20}$ aryl; 5-20-membered heterocyclyl; halogen-substituted $C_1$-$C_6$ alkyl, cycloalkyl, alkenyl or alkynyl; halogen-substituted $C_6$-$C_{20}$ aryl; or halogen-substituted 5-20-membered heterocyclyl;

or $R_{10}$ and $R_{13}$ or $R_{14}$ may be connected to form a 5-9-membered ring under the condition that they meet the above

definitions for $R_{10}$, $R_{13}$ and $R_{14}$;

$R_4$ and $R^{15}$ are each independently hydrogen; halogen; $C_1$-$C_6$ alkyl; cycloalkyl; alkenyl; alkynyl; alkoxy; cyano; 5-20-membered heterocyclyl; $C_6$-$C_{20}$ aryl; or halogen-substituted $C_1$-$C_6$ alkyl, cycloalkyl, alkenyl, alkynyl, alkoxyl, 5-20-membered heterocyclyl or $C_6$-$C_{20}$ aryl;

or $R_{10}$ and $R^{15}$ may form a 4-12-membered cyclic hydrocarbon or heterocycle under the condition that they meet the above definitions for $R_{10}$ and $R^{15}$;

$R_W$ is 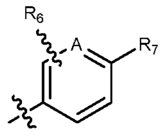 ;

A is $CR^{16}$ or N, and the position of A may be changed on the ring;

$R^{16}$ is hydrogen; $C_1$-$C_6$ alkyl; cycloalkyl; alkenyl; alkynyl; $C_6$-$C_{20}$ aryl; 5-20-membered heterocyclyl; halogen-substituted $C_1$-$C_6$ alkyl, cycloalkyl, alkenyl or alkynyl; halogen-substituted $C_6$-$C_{20}$ aryl; or halogen-substituted 5-20-membered heterocyclyl;

$R_6$ and $R_7$ meet the following conditions:

$R_6$ and $R_7$ are each independently hydrogen; halogen; cyano; hydroxyl; $C_1$-$C_6$ alkyl, cycloalkyl, alkenyl, alkynyl, alkoxyl, 5-20-membered heterocyclyl or $C_6$-$C_{20}$ aryl; or halogen-substituted $C_1$-$C_6$ alkyl, cycloalkyl, alkenyl, alkynyl, alkoxyl, 5-20-membered heterocyclyl or $C_6$-$C_{20}$ aryl; or cyano-substituted $C_1$-$C_6$ alkyl, cycloalkyl, alkenyl, alkynyl, alkoxyl, 5-20-membered heterocyclyl or $C_6$-$C_{20}$ aryl; or hydroxyl-substituted $C_1$-$C_6$ alkyl, cycloalkyl, alkoxyl, 5-20-membered heterocyclyl or $C_6$-$C_{20}$ aryl; or $-CONR^{11}R^{12}$; or $-CH_2NR^{11}R^{12}$;

or $R_6$ and $R_7$ are connected to form

a 5-8-membered single or fused heterocycle containing at least one N or S or O, or two or three of N, S and O at the same time;

or a 5-8-membered single or fused heterocycle containing at least one N or S or O, or two or three of N, S and O at the same time, which is $C_1$-$C_6$ alkyl-substituted;

$R_6$ and $CR^{16}$ may be connected to form a 5-9-membered ring, heterocycle, or aromatic heterocycle;

$R^{11}$ and $R^{12}$ meet the following conditions:
$R^{11}$ and $R^{12}$ are each independently $C_1$-$C_6$ alkyl, or halogen-substituted $C_1$-$C_6$ alkyl, or $R^{11}$ and $R^{12}$ form a 5-7-membered ring with N from $-CONR^{11}R^{12}$, or form a 5-7-membered ring with N from $-CH_2NR^{11}R^{12}$ under the condition that they meet the above definitions for $R^{11}$ and $R^{12}$.

Preferably, the compound with the structural formula of formula (III) includes compounds of formula (I) and formula (II) below:

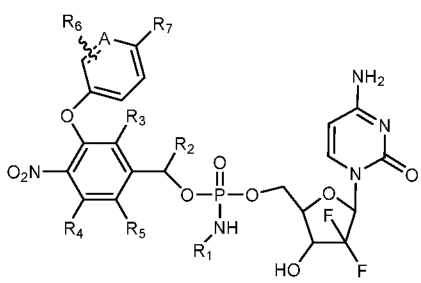 (I)

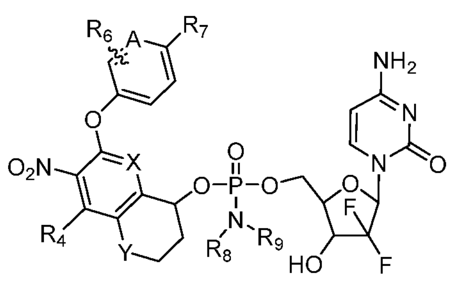 (II)

wherein,

Y is $CR^aR^b$, $NR^a$, or O, and the position of Y may be changed between the 4 positions excluding 2 carbon atoms shared with the benzene ring;

$R^a$ and $R^b$ meet the following conditions:
$R^a$ and $R^b$ are each independently hydrogen; $C_1$-$C_6$ alkyl; cycloalkyl; alkenyl; alkynyl; $C_6$-$C_{20}$ aryl; 5-20-membered heterocyclyl; halogen-substituted $C_1$-$C_6$ alkyl, cycloalkyl, alkenyl oralkynyl; halogen-substituted $C_6$-$C_{20}$ aryl; or halogen-substituted 5-20-membered heterocyclyl;

$R_1$, $R_2$, $R_8$ and $R_9$ meet the following conditions:

$R_1$ is $C_1$-$C_6$ alkyl; cycloalkyl; alkenyl; alkynyl; $C_6$-$C_{20}$ aryl; 5-20-membered heterocyclyl; halogen-substituted $C_1$-$C_6$ alkyl, cycloalkyl, alkenyl or alkynyl; halogen-substituted $C_6$-$C_{20}$ aryl; or halogen-substituted 5-20-membered heterocyclyl;

$R_2$ is hydrogen; $C_1$-$C_6$ alkyl; cycloalkyl; alkenyl; alkynyl; $C_6$-$C_{20}$ aryl; 5-20-membered heterocyclyl; halogen-substituted $C_1$-$C_6$ alkyl, cycloalkyl, alkenyl or alkynyl; halogen-substituted $C_6$-$C_{20}$ aryl; or halogen-substituted 5-20-membered heterocyclyl;

or $R_1$ and $R_2$ may be connected to form a 5-9-membered ring under the condition that they meet the above definitions for $R_1$ and $R_2$;

$R_8$ and $R_9$ are each independently hydrogen; $C_1$-$C_6$ alkyl; cycloalkyl; alkenyl; alkynyl; $C_6$-$C_{20}$ aryl; 5-20-membered heterocyclyl; halogen-substituted $C_1$-$C_6$ alkyl, cycloalkyl, alkenyl or alkynyl; halogen-substituted $C_6$-$C_{20}$ aryl; or halogen-substituted 5-20-membered heterocyclyl, and $R_8$ and $R_9$ are not hydrogen at the same time;

or $R_8$ and $R_9$ may be connected to form a 5-9-membered ring under the condition that they meet the above definitions for $R_8$ and $R_9$;

$R_3$, $R_4$ and $R_5$ meet the following conditions:

$R_3$, $R_4$ and $R_5$ are each independently hydrogen; halogen; $C_1$-$C_6$ alkyl; cycloalkyl; alkenyl; alkynyl; alkoxy; cyano; 5-20-membered heterocyclyl; $C_6$-$C_{20}$ aryl; or halogen-substituted $C_1$-$C_6$ alkyl, cycloalkyl, alkenyl, alkynyl, alkoxyl, 5-20-membered heterocyclyl or $C_6$-$C_{20}$ aryl.

$R_6$ above is connected to the benzene or pyridine ring via a wavy line, and the position of connection is variable and may be one of positions 2/3/5/6 for the specific compound of this structure.

The substitution has a broad meaning. It can be mono-substitution (only one H on a C atom in a benzene ring and the like can be substituted), or multi-substitution, which include multiple substitutions on a certain C atom, i.e., di-substitution and tri-substitution (such as gem-difluoromethyl and gem-trifluoromethyl) or separate substitution on different C atoms in a ring (such as perfluorobenzene).

Alkyl refers to monovalent saturated aliphatic hydrocarbyl groups having more than 1 carbon atom and, in some embodiments, from 1 to 6 carbon atoms. "$C_{x-y}$ alkyl" refers to alkyl groups having from x to y carbon atoms. This term includes, by way of example, linear and branched hydrocarbyl groups (chain alkyl) such as methyl ($CH_3$-), ethyl ($CH_3CH_2$-), *n*-propyl ($CH_3CH_2CH_2$-), isopropyl ($(CH_3)_2CH$-), *n*-butyl ($CH_3CH_2CH_2CH_2$-), isobutyl ($(CH_3)_2CHCH_2$-), *s*-butyl ($(CH_3)(CH_3CH_2)CH$-), *t*-butyl ($(CH_3)_3C$-), *n*-pentyl ($CH_3CH_2CH_2CH_2CH_2$-), and neopentyl ($(CH_3)_3CCH_2$-), etc.

When alkyl and cycloalkyl groups are recited as alternatives, alkyl specifically refers to chain alkyl and is distinguished from cycloalkyl; when alkyl appears alone, its meaning is broad, including chain alkyl and cycloalkyl groups, such as $C_1$-$C_6$ alkyl and halogen-substituted $C_1$-$C_6$ alkyl including $C_1$-$C_6$ chain alkyl and cyclic alkyl, and halogen-substituted $C_1$-$C_6$ chain alkyl and cyclic alkyl, respectively.

Cycloalkyl or cyclic alkyl refers to a saturated or partially saturated cyclic group of more than 3 carbon atoms and no ring heteroatoms and having a single ring or multiple rings including fused, bridged, and spiro ring systems. For multiple ring systems having aromatic and non- aromatic rings that have no ring heteroatoms, the term "cycloalkyl" applies when the point of attachment is at a non-aromatic carbon atom (e.g., 5,6,7,8,-tetrahydronaphthalene-5-yl). Examples of cycloalkyl groups include, for instance, adamantyl, cyclopropyl, cyclobutyl, cyclopentyl, cyclooctyl, and cyclohexenyl. "Cycloalkylene" refers to a divalent cycloalkyl radical having the appropriate hydrogen content.

Alkenyl refers to a linear or branched hydrocarbyl or cyclic hydrocarbyl group having more than 2 carbon atoms and in some embodiments from 1 to 6 carbon atoms and having at least 1 site of vinyl unsaturation (>C=C<). For example,

$C_{x-y}$ alkenyl refers to alkenyl groups having from x to y carbon atoms and is meant to include, for example, ethenyl, propenyl, 1,3-butadienyl, and the like.

Alkynyl refers to a linear monovalent hydrocarbyl radical or a branched monovalent hydrocarbyl radical or cyclic hydrocarbyl radical having more than 2 carbon atoms and in some embodiments from 2 to 6 carbon atoms or 2 to 4 carbon atoms and containing at least one triple bond. For example, $C_2$-$C_6$ alkynyl includes ethynyl, propynyl, and the like.

"$C_6$-$C_{20}$ aryl" refers to an aromatic group of from 6 to 20 carbon atoms and no ring heteroatoms and having a single ring (e.g., phenyl) or multiple condensed (fused) rings (e.g., naphthyl or anthryl), or hydrocarbyl groups (alkyl, cycloalkyl, alkenyl, alkynyl) are attached to the ring so that the total number of carbon atoms including the ring and the hydrocarbyl groups on the ring is 6-20. For multiple ring systems, including fused, bridged, and spiro ring systems having aromatic and non-aromatic rings that have no ring heteroatoms, the term "aryl" or "Ar" applies when the point of attachment is at an aromatic carbon atom (e.g., 5,6,7,8-tetrahydronaphthalene-2-yl is an aryl group as its point of attachment is at the 2- position of the aromatic phenyl ring).

"Halogen" refers to one or more of fluorine, chlorine, bromine, and iodine.

"Cyano" refers to a -C=N group.

"Heteroaryl" refers to an aromatic group of 1 to 6 heteroatoms selected from the group consisting of oxygen, nitrogen, and sulfur and includes single ring (e.g., imidazol-2-yl and imidazol-5-yl) and multiple ring systems (e.g., imidazopyridyl, benzotriazolyl, benzimidazol-2-yl and benzimidazol-6-yl). For multiple ring systems, including fused, bridged, and spiro ring systems having aromatic and non-aromatic rings, the term "heteroaryl" applies if there is at least one ring heteroatom, and the point of attachment is at an atom of an aromatic ring (e.g., 1,2,3,4-tetrahydroquinolin-6-yl and 5,6,7,8-tetrahydroquinolin-3-yl). In some embodiments, the nitrogen and/or the sulfur ring atom(s) of the heteroaryl group are optionally oxidized to provide for the N-oxide (N$\rightarrow$O), sulfinyl, or sulfonyl moieties. The term heteroaryl includes, but is not limited to, acridinyl, azocinyl, benzimidazolyl, benzofuranyl, benzothiofuranyl, benzothiophenyl, benzoxazolyl, benzothiazolyl, benzotriazolyl, benzotetrazolyl, benzisoxazolyl, benzisothiazolyl, benzothienyl, benzimidazolinyl, carbazolyl, NH-carbazolyl, carbolinyl, chromanyl, chromenyl, cinnolinyl, dithiazinyl, furanyl, furazanyl, imidazolidinyl, imidazolinyl, imidazopyridyl, imidazolyl, indazolyl, indolenyl, indolinyl, indolizinyl, indolyl, isobenzofuranyl, isochromanyl, isoindazolyl, isoindolinyl, isoindolyl, isoquinolinyl, isoquinolyl, isothiazolyl, isoxazolyl, naphthyridinyl, octahydroisoquinolinyl, oxadiazolyl, oxazolidinyl, oxazolyl, pyrimidinyl, phenanthridinyl, phenanthrolinyl, phenazinyl, phenothiazinyl, phenoxathiinyl, phenoxazinyl, phthalazinyl, piperazinyl, pteridinyl, purinyl, pyranyl, pyrazinyl, pyrazolidinyl, pyrazolinyl, pyrazolyl, pyridazinyl, pyridooxazolyl, pyridoimidazolyl, pyridothiazolyl, pyridinyl, pyridyl, pyrimidinyl, pyrrolyl, quinazolinyl, quinolinyl, quinoxalinyl, quinuclidinyl, tetrahydroisoquinolinyl, tetrahydroquinolinyl, tetrazolyl, thiadiazinyl, thiadiazolyl, thianthrenyl, thiazolyl, thienyl, thienothiazolyl, thienooxazolyl, thienoimidazolyl, thiophenyl, triazinyl and xanthenyl.

"Heterocyclic" or "heterocycle" or "heterocycloalkyl" or "heterocyclyl" has the same meaning, refers to a saturated or partially saturated cyclic group having from 1 to 14 carbon atoms and from 1 to 6 heteroatoms selected from the group consisting of nitrogen, sulfur, or oxygen and includes single ring and multiple ring systems which include fused, bridged, and spiro ring systems. For multiple ring systems having aromatic and/or non-aromatic rings, the term "heterocyclic", "heterocycle", "heterocycloalkyl", or "heterocyclyl" applies when there is at least one ring heteroatom, and the point of attachment is at an atom of a non-aromatic ring (e.g., 1,2,3,4-tetrahydroquinoline-3-yl, 5,6,7,8-tetrahydroquinoline-6-yl, and decahydroquinolin-6-yl). In some examples, the heterocyclic groups herein are 3-15-membered, 4-14-membered, 5-13-membered, 7-12-membered, or 5-7-membered heterocycles. In some other examples, the heterocycles contain 4 heteroatoms. In some other examples, the heterocycles contain 3 heteroatoms. In another example, the heterocycles contain up to 2 heteroatoms. In some examples, the nitrogen and/or sulfur atom(s) of the heterocyclic group are optionally oxidized to provide for the N-oxide, sulfinyl, or sulfonyl moieties. Heterocyclyl includes, but is not limited to, tetrahydropyranyl, piperidinyl, N-methylpiperidin-3-yl, piperazinyl, N-methylpyrrolidin-3-yl, 3-pyrrolidinyl, 2-pyrrolidon-1-yl, morpholinyl, and pyrrolidinyl. A prefix indicating the number of carbon atoms (e.g., $C_3$-$C_{10}$) refers to the total number of carbon atoms in the portion of the heterocyclyl group exclusive of the number of heteroatoms.

In this application, heterocycles or heterocyclyl groups include the aforementioned "heteroaryl" and "heterocycle", but do not include "aryl". A 5-8-membered heterocycle is a ring composed of 5-8 atoms, including heteroatoms (oxygen, nitrogen, and sulfur) and carbon atoms.

Preferably, $R_1$, $R_8$ and $R_9$ are each independently $C_1$-$C_5$ alkyl, halogen-substituted $C_1$-$C_5$ alkyl, $C_3$-$C_6$ cycloalkyl or $C_6$-$C_{20}$ aryl.

More preferably, $R_1$, $R_8$ and $R_9$ are each independently methyl, ethyl, n-propyl, isopropyl, isobutyl, t-butyl, neopentyl, bromoethyl, 1,1,1-trifluoroethyl, $C_3$-$C_6$ cycloalkyl or benzyl.

Preferably, $R_2$ is hydrogen, methyl, or trifluoromethyl.

Preferably, $R_1$ and $R_2$ in formula (I) are connected to form a 6-membered ring.

Preferably, $R_3$, $R_4$ and $R_5$ are each independently hydrogen.

Preferably, $R_6$ is hydrogen, fluorine, fluorophenyl, or

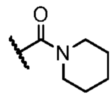 or 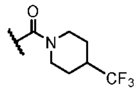.

Preferably, $R_7$ is hydrogen, $-CF_3$, phenyl, chlorophenyl, fluorophenyl, $-CF_3$-substituted phenyl, fluoropyridyl, or

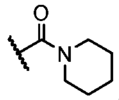, 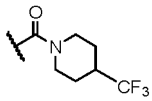 or 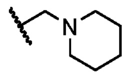.

In chlorophenyl, fluorophenyl, $-CF_3$-substituted phenyl, and fluoropyridyl mentioned above, chlorine, fluorine, and $-CF_3$ may substitute at any substitutable position of the corresponding benzene ring and pyridine, and the number of substitutions may be more than one. For example, for chlorophenyl, it includes o-chlorophenyl, m-chlorophenyl, p-chlorophenyl, 2,3-dichlorophenyl, 2,4-dichlorophenyl, 2,5-dichlorophenyl, 2,6-dichlorophenyl, 2,3,4-trichlorophenyl 2,3,5-trichlorophenyl, 2,3,6-trichlorophenyl, 3,4,5-trichlorophenyl, 3,4,6-trichlorophenyl, 4,5,6-trichlorophenyl, and corresponding tetrachlorophenyl and pentachlorophenyl.

Preferably, $R_6$ and $R_7$ are connected to form a 5-8-membered heterocycle, $C_1$-$C_6$ alkyl-substituted 5-8-membered heterocycle containing N or S or O, or any two or three of N, S and O at the same time; and the 5-8-membered heterocycle shares 2 atoms with the connected benzene ring or pyridine ring to form a fused ring.

Further preferably, $R_6$ and $R_7$ are connected to form a 5-8-membered heterocycle containing 2 N atoms, or $C_1$-$C_6$ alkyl-substituted 5-8-membered heterocycle; more preferably, $R_6$ and $R_7$ are connected to form a pyrimidine, or $C_1$-$C_6$ alkyl-substituted pyrimidine; or $R_6$ and $R_7$ are connected to form a 5-8-membered heterocycle containing 1 N atom, or $C_1$-$C_6$ alkyl-substituted 5-8-membered heterocycle, and more preferably, $R_6$ and $R_7$ are connected to form a pyridine, or $C_1$-$C_6$ alkyl-substituted pyridine.

Preferably, the compound with the structural formula of formula (I) includes compounds with the structures of formulas I-1 to I-5 below:

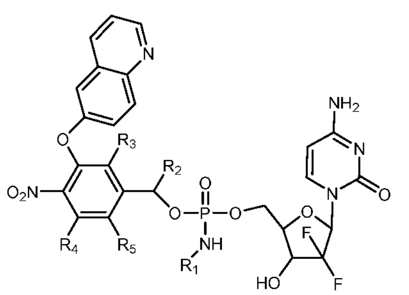 (I-1), 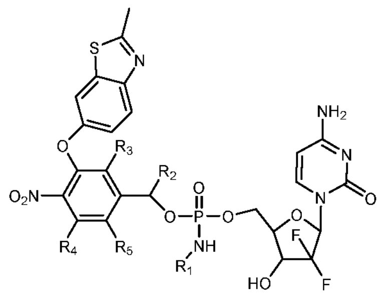 (I-2),

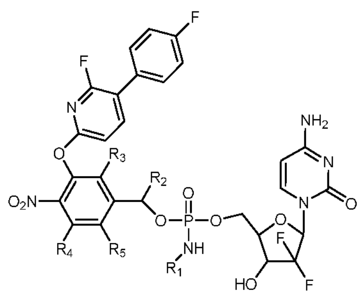 (I-3), 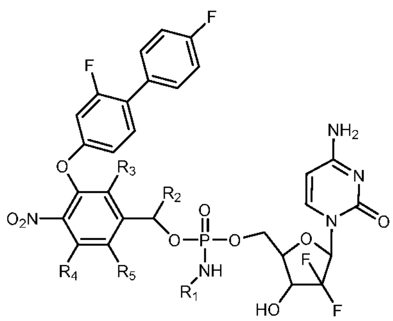 (I-4)

and

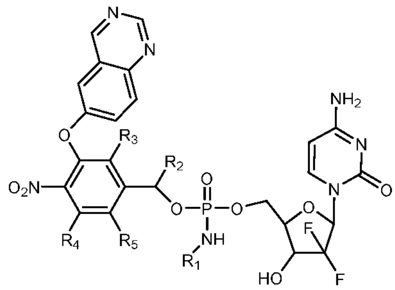

(I-5),

wherein definitions of the substituents are as above.

Preferably, the compound with the structural formula of formula (II) includes compounds with the structures of formulas II-1 to II-6 below:

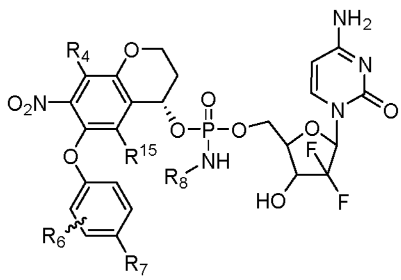

(II-1),

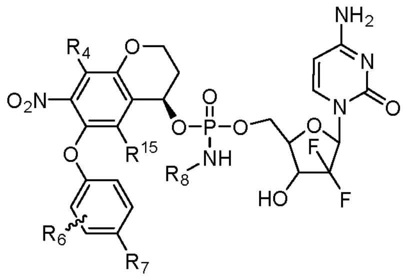

(II-2),

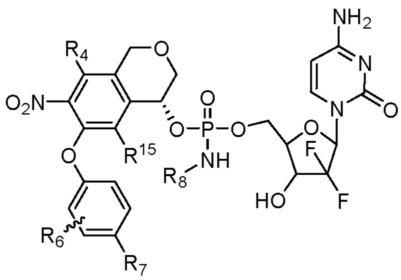

(II-3),

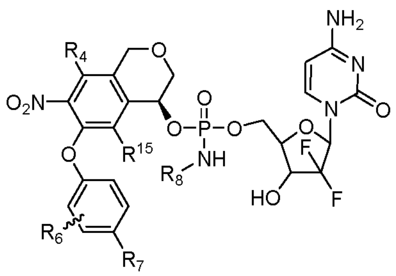

(II-4),

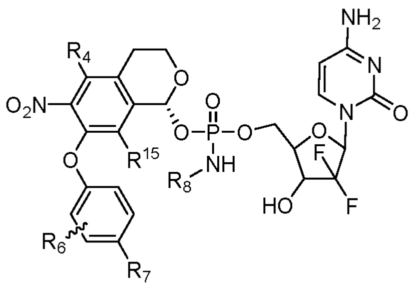

(II-5) and

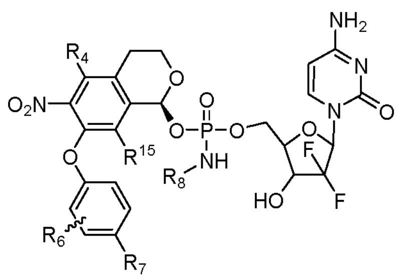

(II-6),

wherein definitions of the substituents are as above.

In this application, the structure of formula (I) substituted by isotope deuterium includes a compound with the structure of formula I-6 below:

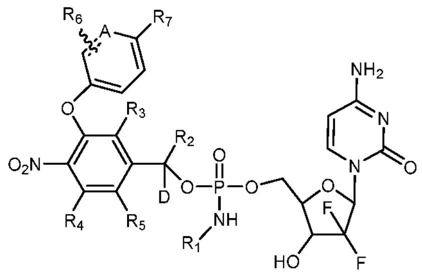

(I-6).

Preferably, the structure of formula (I) substituted by isotope deuterium includes compounds with the structures of formulas I-7 and I-8 below:

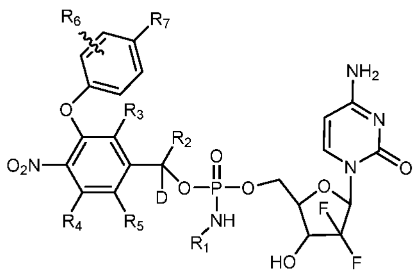

(I-7) and

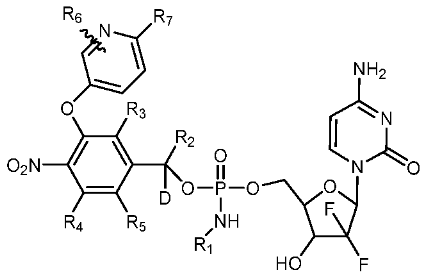

(I-8).

Further preferably, the structure of formula (I) substituted by the isotope deuterium includes compounds with the structures of formulas I-9 to I-13 below:

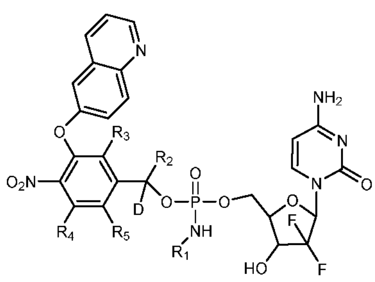

(I-9),

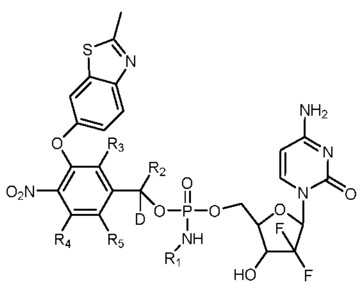

(I-10),

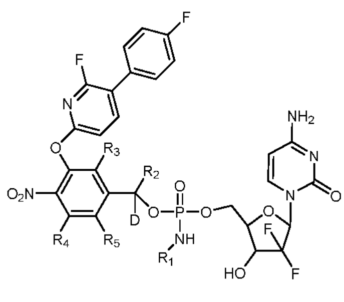

(I-11),

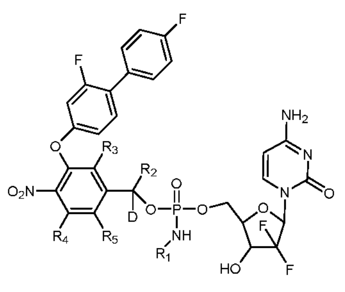

(I-12)

and

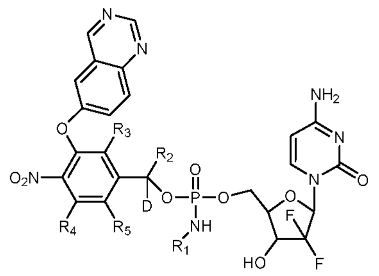
(I-13),

wherein definitions of the substituents are as above.

In this application, the structure of formula (II) substituted by isotope deuterium includes compounds with the structures of formulas II-7 to II-12 below:

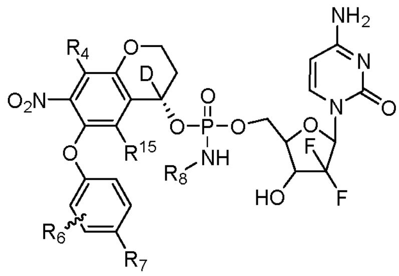
(II-7),

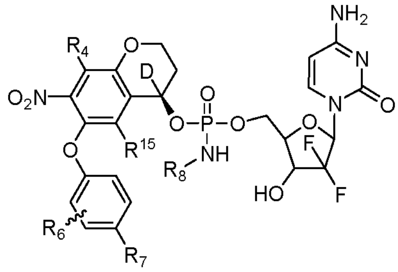
(II-8),

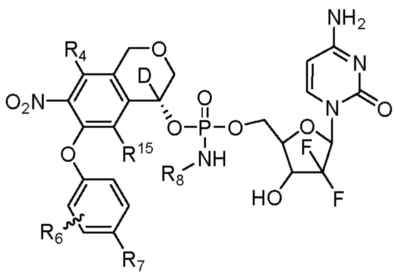
(II-9),

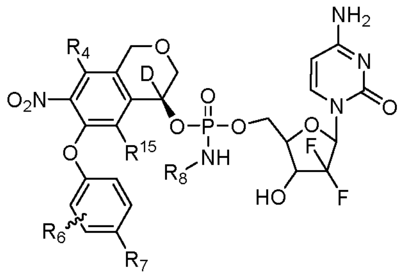
(II-10),

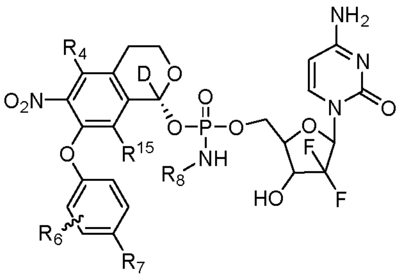
(II-11) and

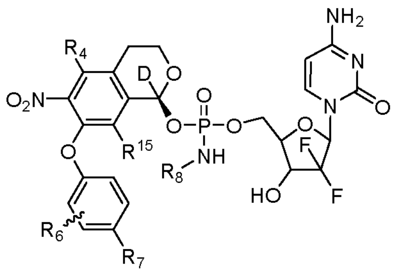
(II-12),

wherein, definitions of the substituents are as above.

Preferably, hydrogen atom(s) in $R_1$ are substituted by at least one deuterium.

Preferably, formula (I) is selected from compounds of the following structures:

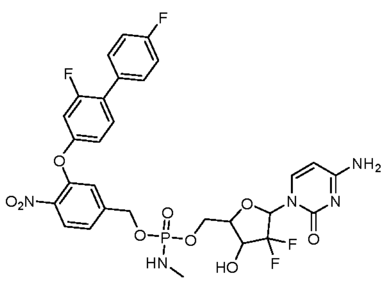,
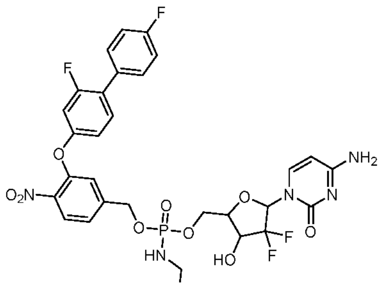,
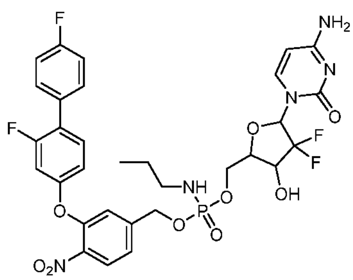,

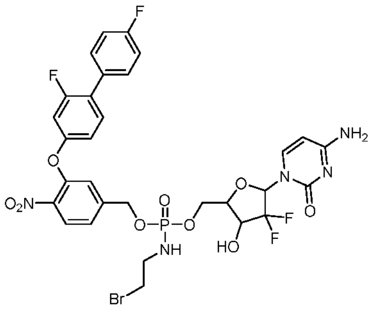,
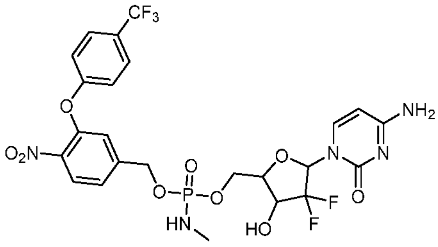,

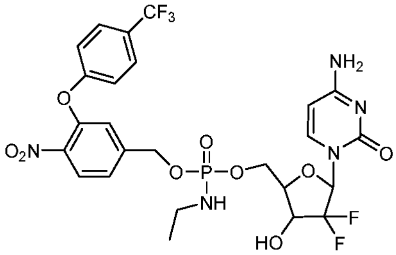,
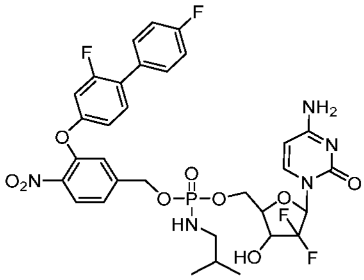,
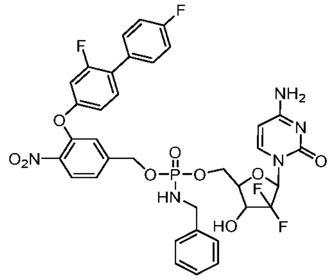,

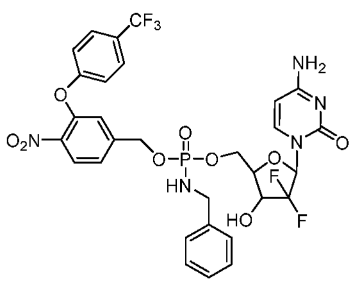,
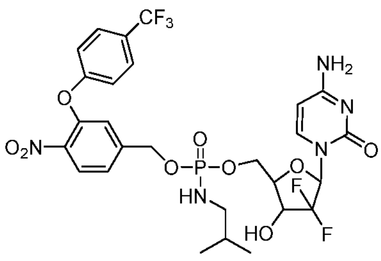,
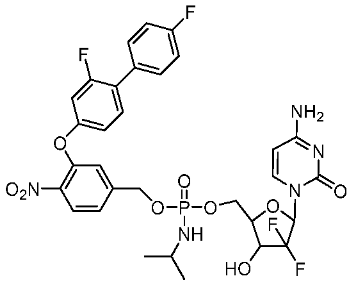,

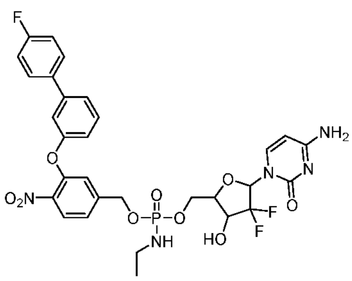,
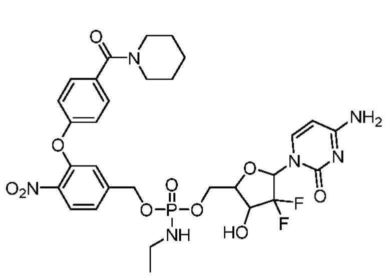,
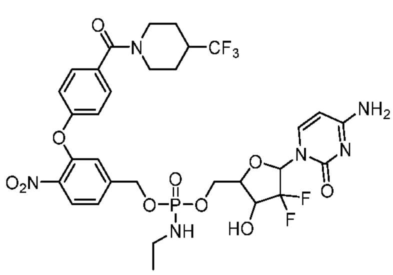,

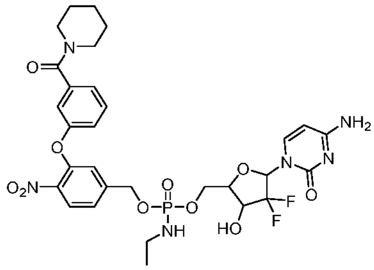,

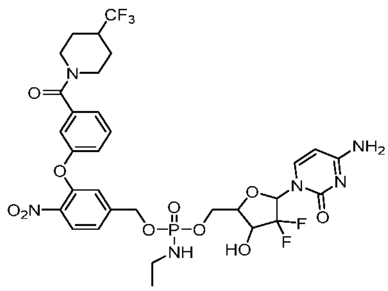,

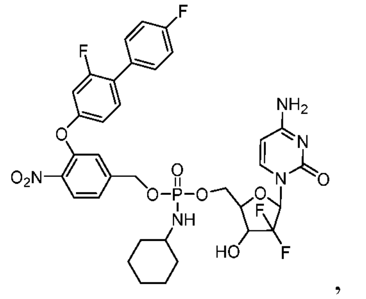,

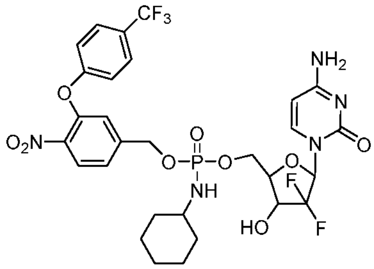,

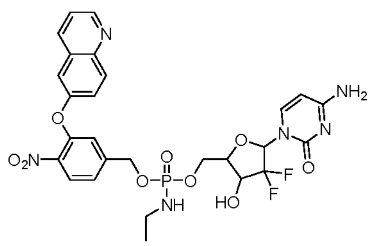,

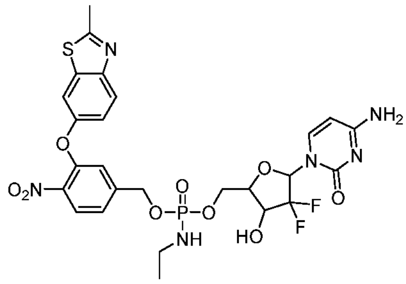,

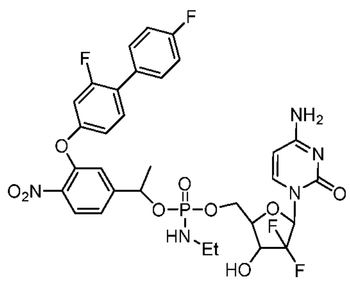,

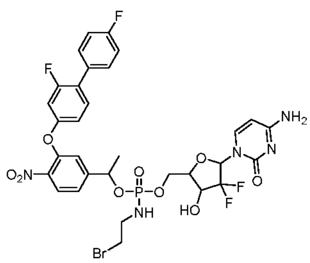,

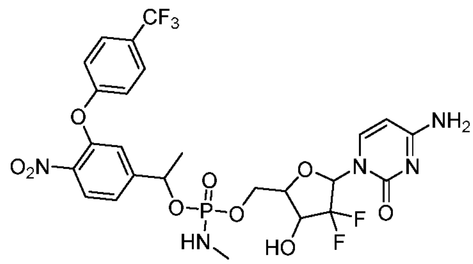,

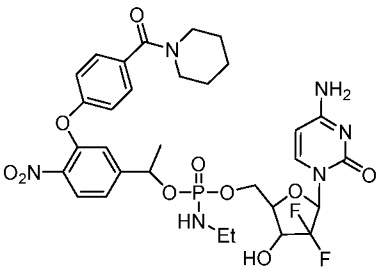,

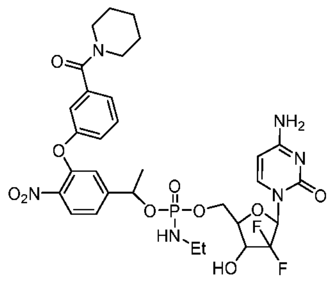,

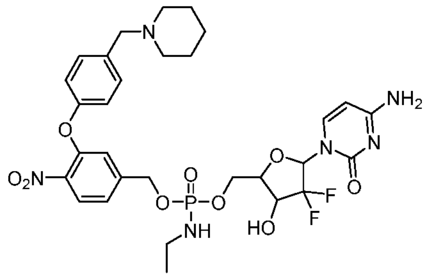,

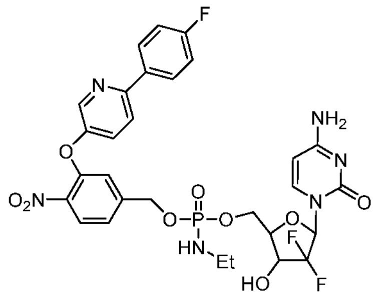,

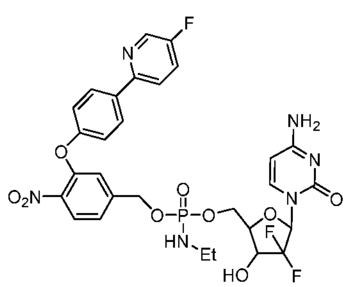,

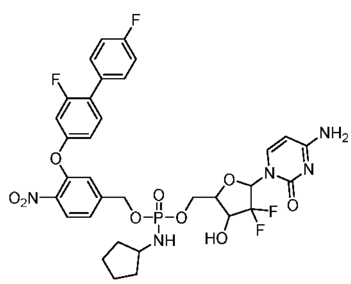,

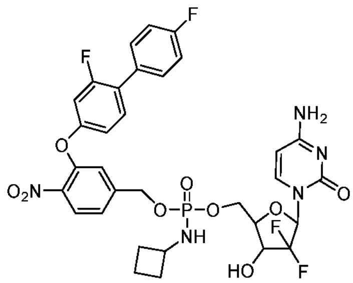,
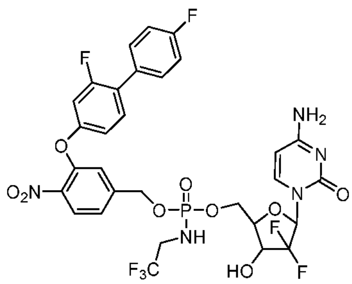,
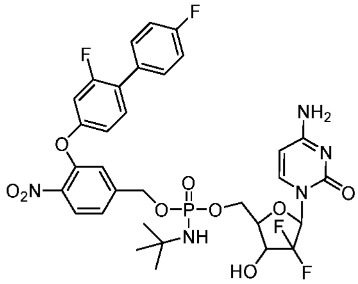,
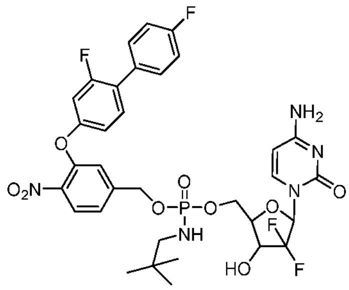,
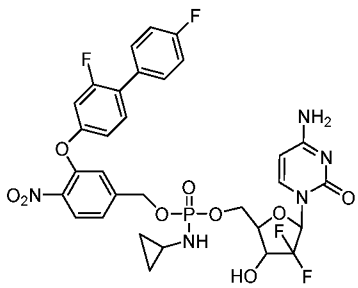,
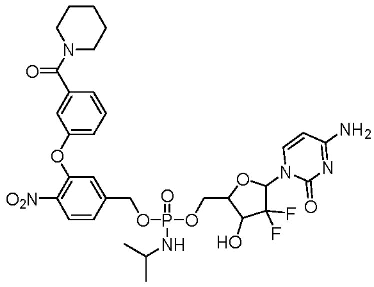,
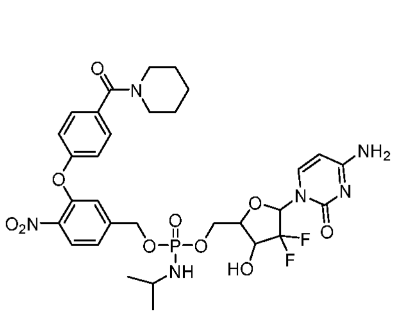,
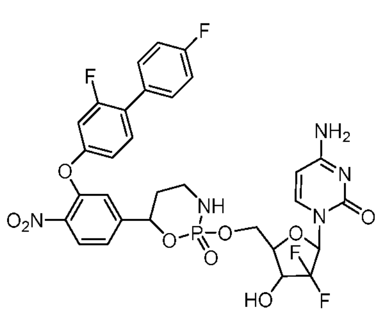,
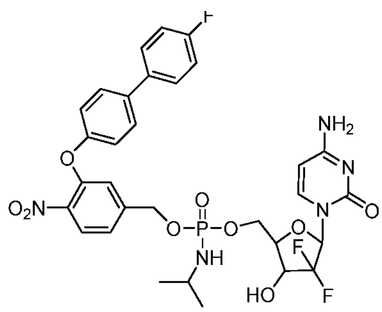,
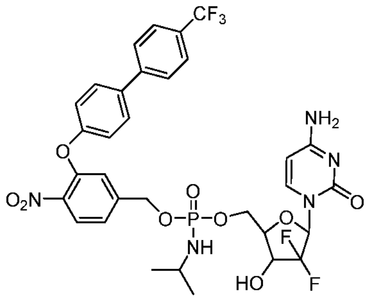,
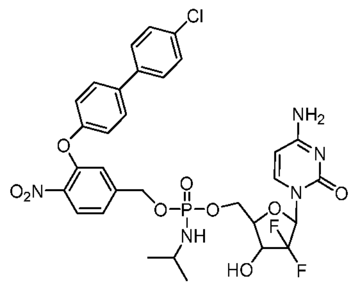,
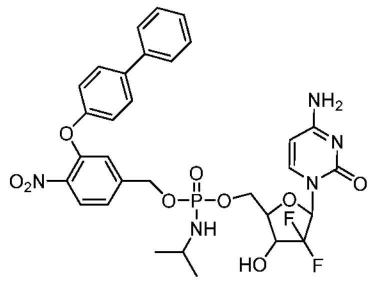,
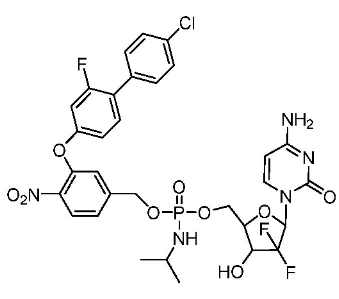,
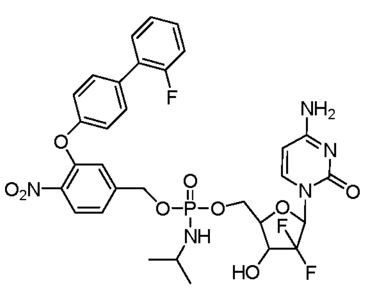 and
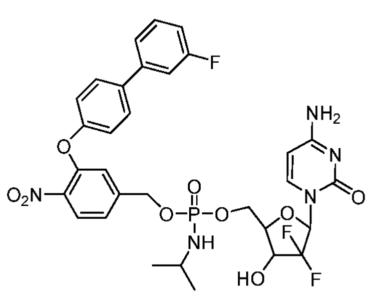.

Preferably, formula (II) is selected from compounds of the following structures:

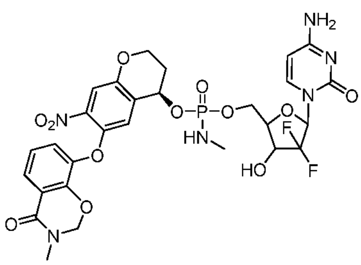 , 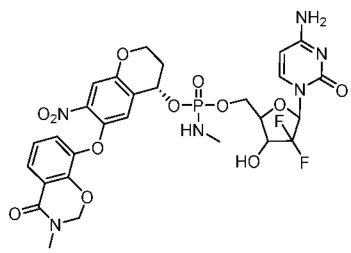 , 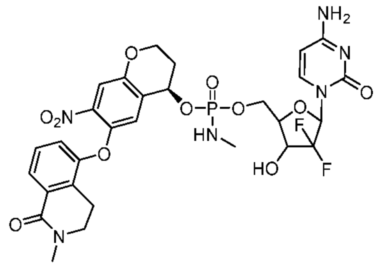 ,

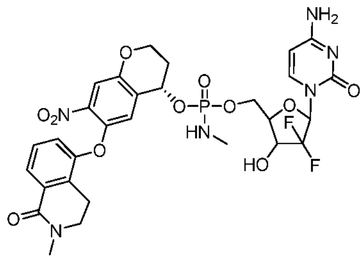 , 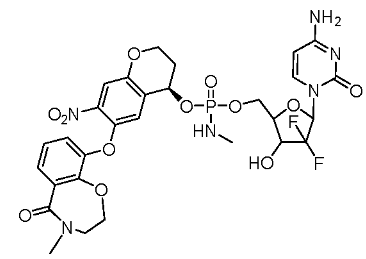 , 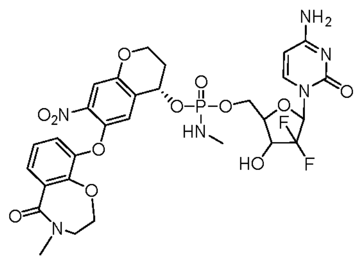 ,

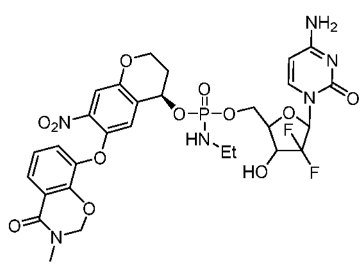 , 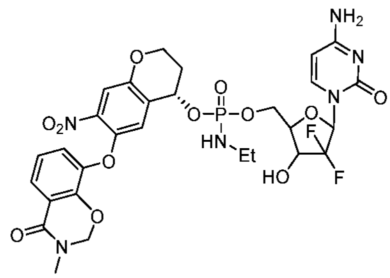 , 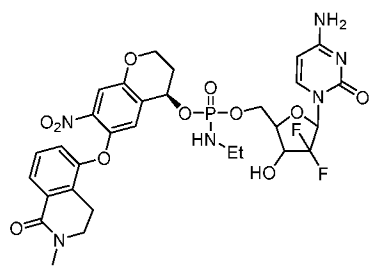 ,

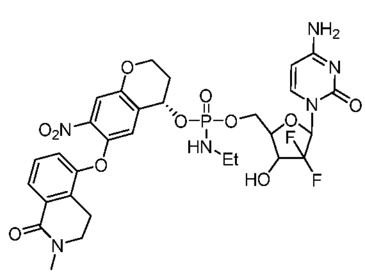 , 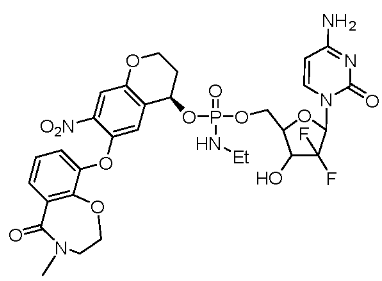 , 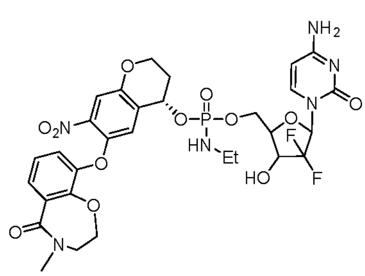 ,

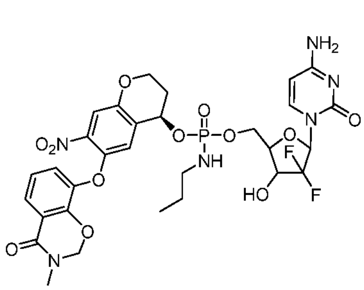 , 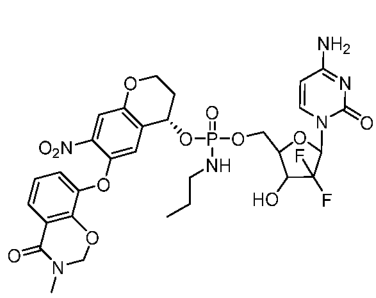 , 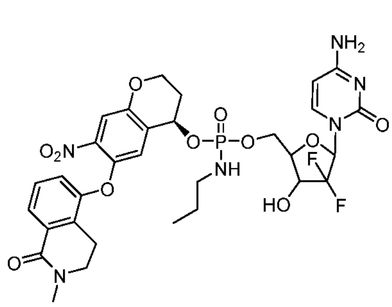 ,

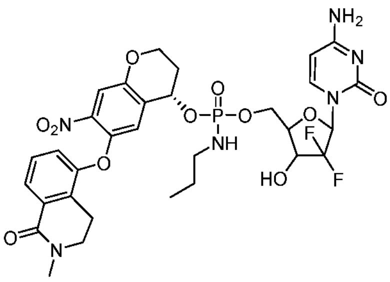, 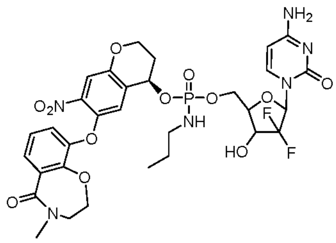, 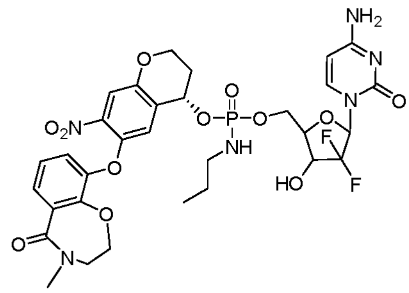,

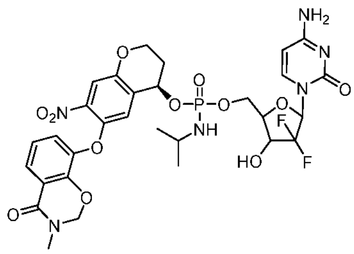, 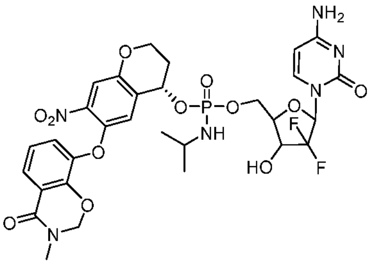, 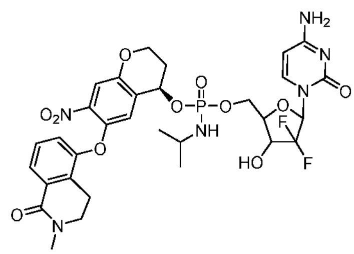,

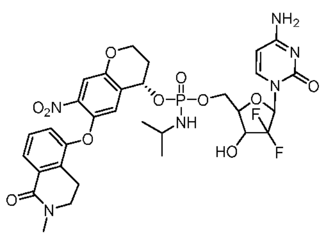, 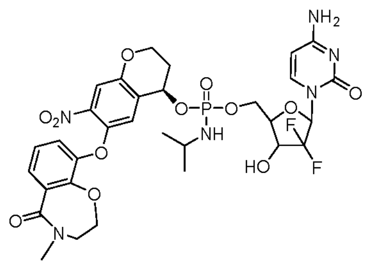, 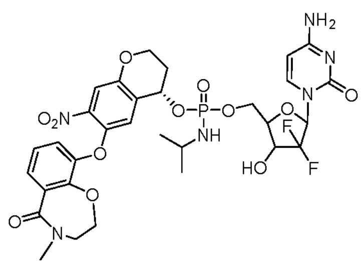,

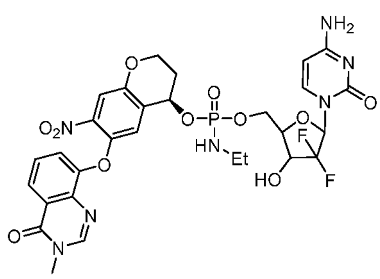, 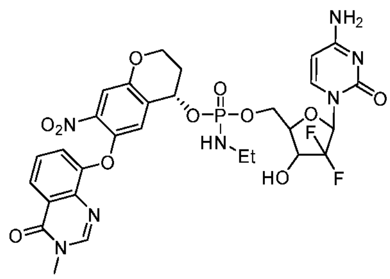,

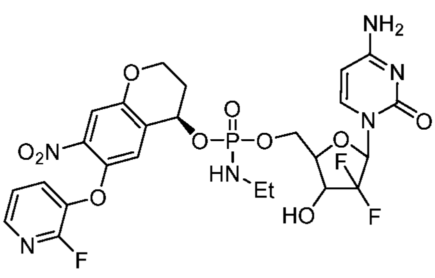, 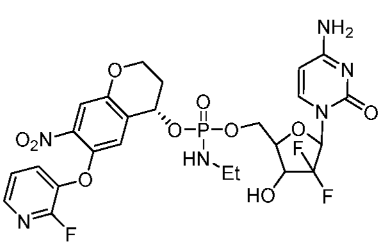,

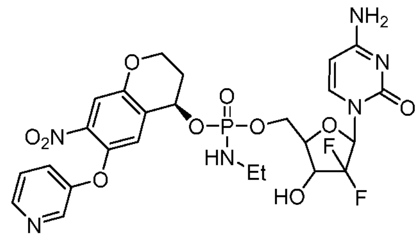 , 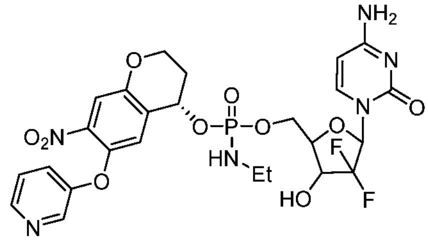 ,

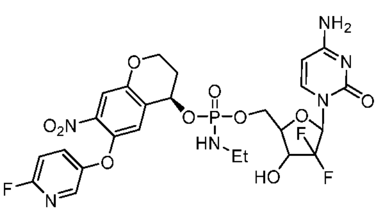 , 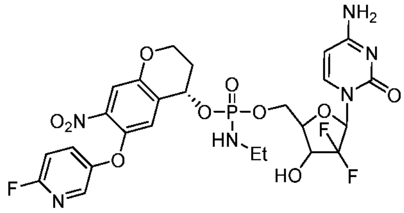 , 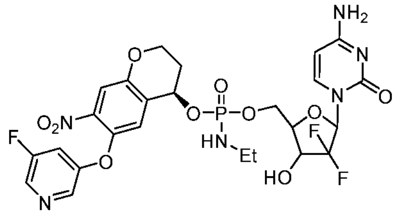 ,

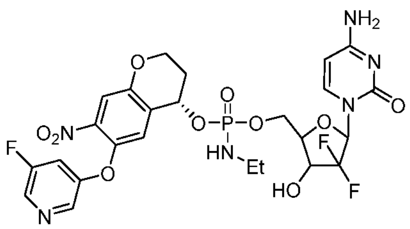 , 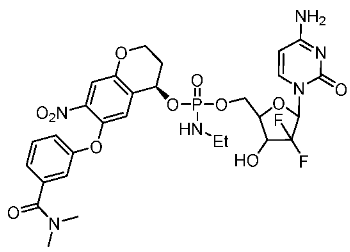 , 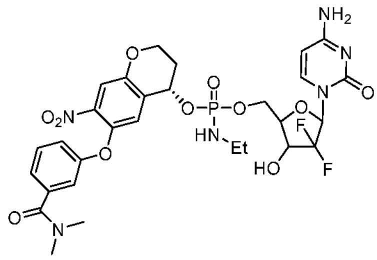 ,

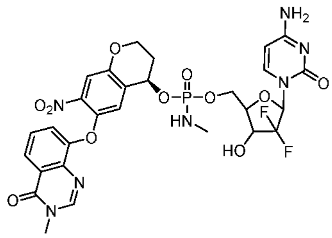 , 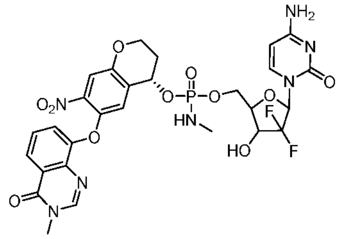 , 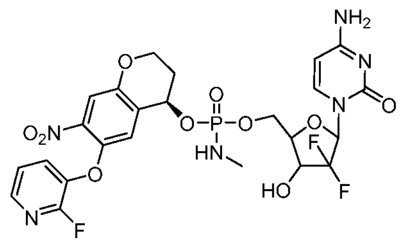 ,

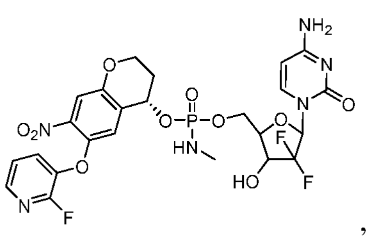 , 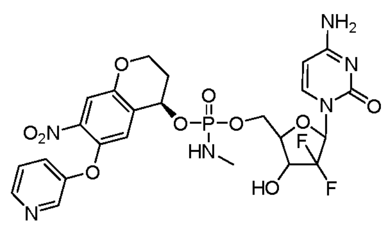 , 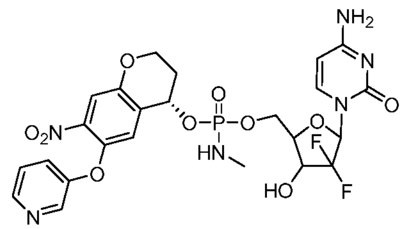 ,

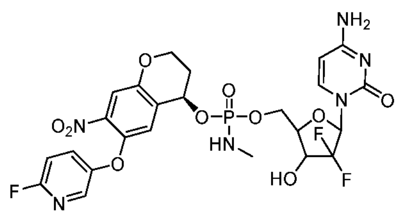 , 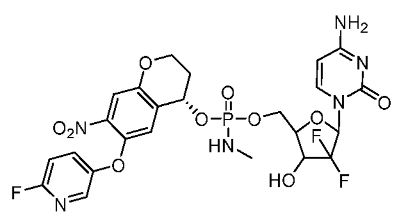 , 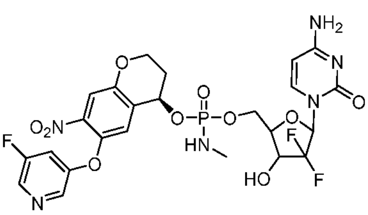 ,

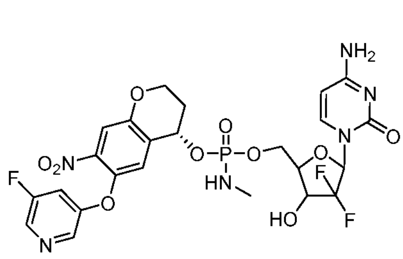,
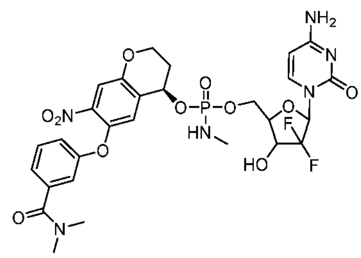,
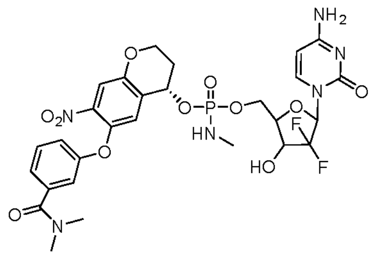,

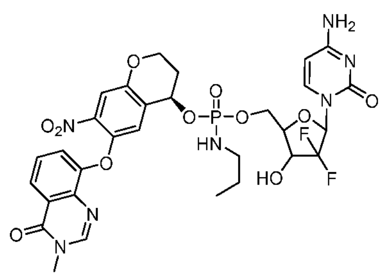,
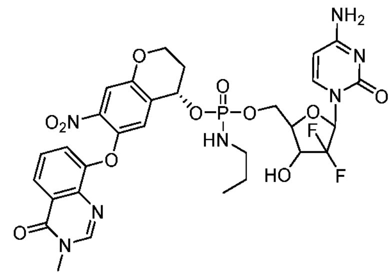,
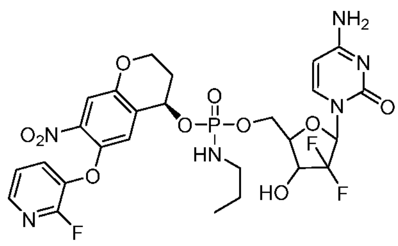,

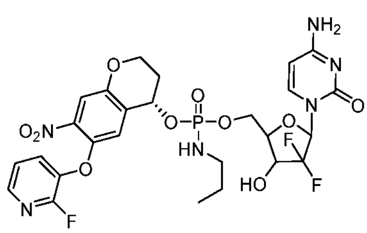,
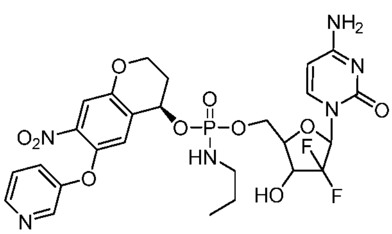,
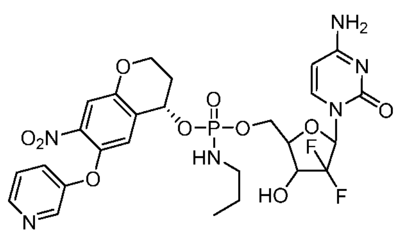,

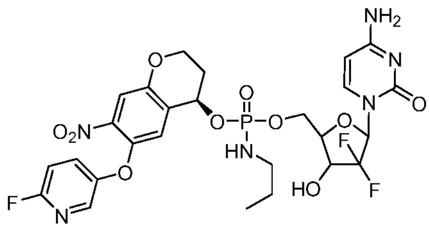,
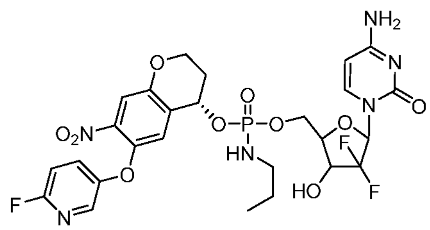,

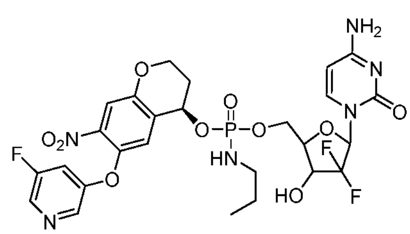,
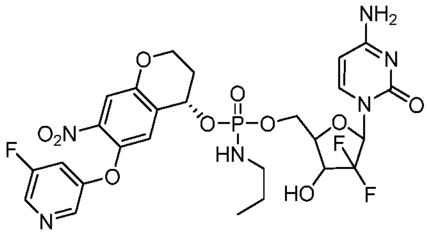,

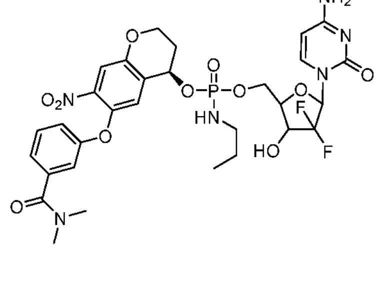,
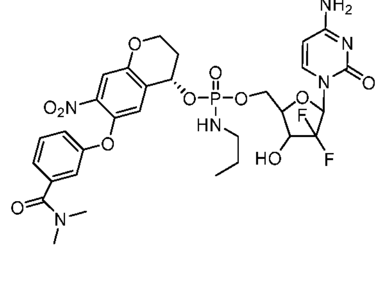,
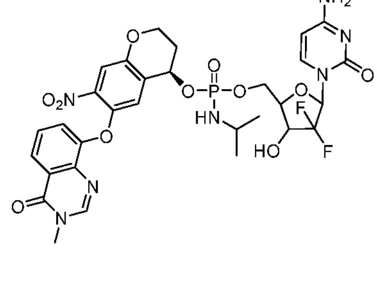,

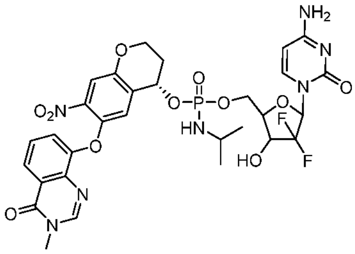,
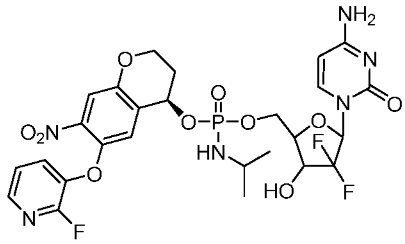,
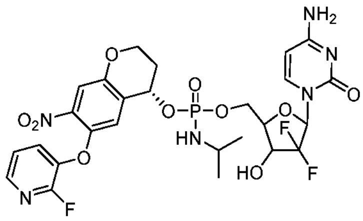,

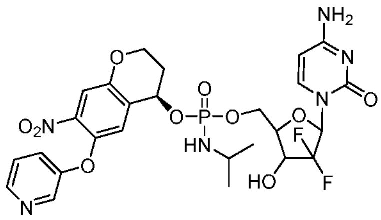,
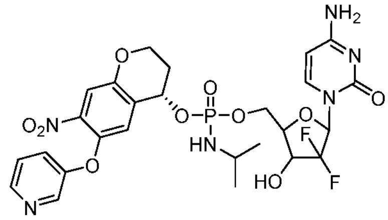,
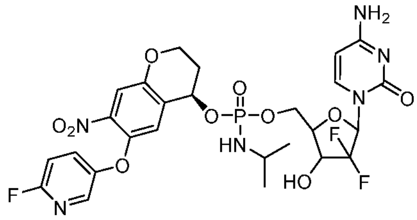,

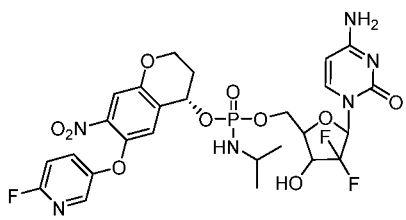,
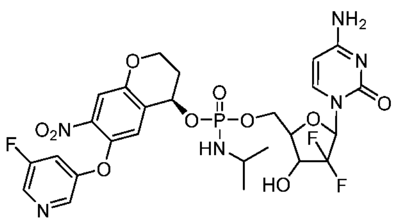,

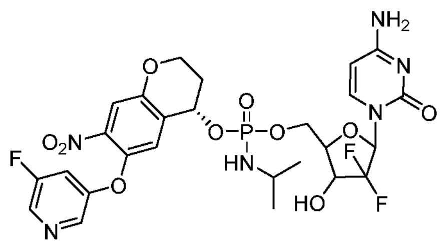,
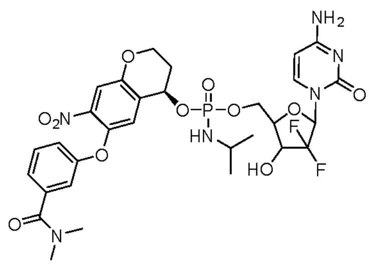

and

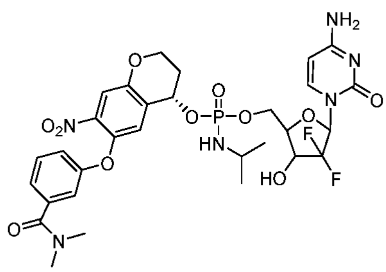.

Further, the salt is a basic salt or an acid salt, and the solvate is a hydrate or alcoholate.

The compound also includes the form of their salts of formula II or formula III; that is, the present invention provides a pharmaceutically acceptable salt of the compound, wherein the salt may be a basic salt, including salts of the compound formed with inorganic bases (such as alkali metal hydroxides, alkaline earth metal hydroxides, and the like), or with organic bases (such as mono-, di-, or triethanolamine, and the like). Alternatively, the salt may be an acid salt including salts of the compound formed with inorganic acids (such as hydrochloric acid, hydrobromic acid, hydroiodic acid, nitric acid, perchloric acid, sulfuric acid, phosphoric acid, or the like), or with organic acids (such as methanesulfonic acid, trifluoromethanesulfonic acid, ethanesulfonic acid, benzenesulfonic acid, *p*-toluenesulfonic acid, fumaric acid, oxalic

acid, maleic acid, citric acid, and the like). The selection and preparation of acceptable salts and solvates of the compound are well known in the art. For gemcitabine and derivatives thereof, preferred salts are hydrochlorides and monophosphates.

The compound described herein may further be used in the form of a solvate, wherein the solvate is a hydrate, an alcoholate and the like, and the alcoholate includes ethanolates.

The present invention also provides application of the compound as described above in the manufacture of a medicament for the treatment of tumors and cancers.

The present invention also provides a medicament or formulation containing the above compound.

Preferably, the medicament or formulation is used for the treatment of tumor and cancer diseases in patients, wherein the tumors and cancers include:

lung cancer, non-small cell lung cancer, liver cancer, pancreatic cancer, stomach cancer, bone cancer, esophagus cancer, breast cancer, prostate cancer, testicular cancer, colon cancer, ovarian cancer, bladder cancer, cervical cancer, melanoma, squamous cell carcinoma, basal cell carcinoma, adenocarcinoma, sweat gland carcinoma, sebaceous gland carcinoma, papillary carcinoma, papillary adenocarcinoma, cystic adenocarcinoma, cystic carcinoma, medullary carcinoma, bronchial carcinoma, osteocyte carcinoma, epithelial carcinoma, cholangiocarcinoma, choriocarcinoma, embryonal carcinoma, seminoma, Wilm's tumor, glioblastoma, astrocytoma, medulloblastoma, craniopharyngioma, ependymoma, pineal tumor, hemocytoblastoma, vocal cord neuroma, meningioma, neuroblastoma, optic neuroblastoma, retinoblastoma, neurofibroma, fibrosarcoma, fibroblastoma, fibroma, fibroadenoma, fibrochondroma, fibrocystoma, fibromyxoma, fibroosteoma, fibromyxosarcoma, fibropapilloma, myxosarcoma, myxocystoma, myxochondroma, myxochondrosarcoma, myxochondrofibrosarcoma, myxadenoma, myxoblastoma, liposarcoma, lipoma, lipoadenoma, lipoblastoma, lipochondroma, lipofibroma, lipoangioma, myxolipoma, chondrosarcoma, chondroma, chondromyoma, chordoma, chorioadenoma, chorioepithelioma, chorioblastoma, osteosarcoma, osteoblastoma, osteochondrofibroma, osteochondrosarcoma, osteochondroma, osteocystoma, osteodentinoma, osteofibroma, fibrosarcoma of bone, angiosarcoma, hemangioma, angiolipoma, angiochondroma, hemangioblastoma, angiokeratoma, angioglioma, angioendothelioma, angiofibroma, angiomyoma, angiolipoma, angiolymphangioma, angiolipoleiomyoma, angiomyolipoma, angiomyoneuroma, angiomyxoma, angioreticuloma, lymphangiosarcoma, lymphogranuloma, lymphangioma, lymphoma, lymphomyxoma, lymphosarcoma, lymphangiofibroma, lymphocytoma, lymphoepithelioma, lymphoblastoma, endothelioma, endothelioblastoma, synovioma, synovial sarcoma, mesothelioma, connective tissue tumor, Ewing's tumor, leiomyoma, leiomyosarcoma, leiomyoblastoma, leiomyofibroma, rhabdomyoma, rhabdomyosarcoma, rhabdomyomyxoma, acute lymphatic leukemia, acute myelogenous leukemia, anemia of chronic disease, polycythemia, lymphoma, endometrial cancer, glioma, colorectal cancer, thyroid cancer, urothelial cancer or multiple myeloma.

The present invention also provides a method for treating cancer or tumor, comprising a step of applying the aforementioned medicament or formulation; and a step of determining an AKR1C3 reductase content or expression level of cancer cells or tissues in a patient, and

administering the aforementioned medicament or formulation to the patient if the measured AKR1C3 reductase content or expression level is equal to or greater than a predetermined value.

Preferably, the AKR1C3 reductase content or expression level may be measured using an AKR1C3 antibody.

The present invention also provides another method for treating cancer or tumor, comprising a step of applying the aforementioned medicament or formulation; and a step of adjusting an AKR1C3 reductase content or expression level, and

administering the aforementioned medicament or formulation to the patient when the AKR1C3 reductase content or expression level is adjusted to be equal to or greater than a predetermined value.

The AKR1C3 reductase content can be determined using methods including ELISA and IHC methods, etc.

Liquid samples such as plasma and blood can be directly tested using a commercially available human aldo-keto reductase 1C3 (AKR1C3) ELISA assay kit. Other samples are tested after being treated.

The immunohistochemical (IHC) method is suitable for testing solid tumor samples.

Detection of AKR1C3 expression level refers to the detection of corresponding mRNA expression levels. Studies show that there is a significant correlation between the AKR1C3 enzyme content and AKR1C3 RNA expression level in cancer cells of different solid tumors, and there is a significant correlation between the AKR1C3 enzyme content and AKR1C3 RNA expression level in different blood cancer cell lines. Therefore, the enzyme content can also be predicted or characterized by detecting ARKR1C3 RNA content (or expression level), thereby providing guidance on using the medicines. The q-PCR technique can be used to detect the RNA (messenger RNA, i.e. mRNA) content or expression level of AKR1C3.

Studies show that that after radiotherapy, the content of AKR1C3 enzyme in the tumor tissue of patients with head and neck cancer is increased. Therefore, it is possible to increase the expression level of AKR1C3 enzyme by irradiating the patients' tumor tissue with radioactive rays used in radiotherapy. The radioactive rays include $\alpha$-, $\beta$-, $\gamma$-rays produced by radioisotopes, and X-rays, electron rays, proton beams and other particle beams produced by various X-ray therapeutic machines or accelerators.

Studies have found that certain chemical components can also promote the expression of AKR1C3 enzyme in the human body, such as kukui (scientific name: *Aleurites moluccana* (L.) Willd.) seed oil (Japanese patent application (Publication No. JP2021145582A) entitled PRODUCTION PROMOTER FOR AKR1C1, AKR1C2 AND AKR1C3).

This method is mainly for the situation where the content of AKR1C3 reductase in a patient is relatively low, and is performed by adjusting the content level of AKR1C3 reductase in the patient to an appropriate level through a certain adjustment treatment/administration process.

Regarding the medicament or formulation described herein, the prepared medicament contains a specific dosage range of the shown compounds or salts or solvates thereof, and/or the prepared medicament is in a specific dosage form and is administered using a specific administration method.

The medicament or formulation herein may also contain pharmaceutically acceptable auxiliaries or excipients. The medicament may be in any dosage form for clinical administration, such as tablets, suppositories, dispersible tablets, enteric-coated tablets, chewable tablets, orally disintegrating tablets, capsules, sugar-coated preparations, granules, dry powders, oral solutions, injections, lyophilized powder for injection, or infusion solutions. According to the specific dosage form and the administration method, the pharmaceutically acceptable auxiliaries or excipients in the medicament may include one or more of the following: diluents, solubilizers, disintegrants, suspensions, lubricants, binding agents, fillers, flavoring agents, sweeteners, antioxidants, surfactants, preservatives, wrapping agents, and pigments.

The medicament should be applied or administered so as to reach a "therapeutically effective amount". The "therapeutically effective amount" refers to an amount of a drug that, when administered to a patient with cancer, will have the intended therapeutic effect, e.g., alleviation, amelioration, palliation, or elimination of one or more manifestations of cancer in the patient. A therapeutic effect does not necessarily occur by administration of one dose, and may occur only after administration of a series of doses. Thus, a therapeutically effective amount may be administered in one or more administrations.

"Treatment" of a condition or patient refers to taking steps to obtain beneficial or desired results, including clinical results. For purposes of this invention, beneficial or desired clinical results include, but are not limited to, alleviation or amelioration of one or more symptoms of cancer; diminishment of extent of disease; delay or slowing of disease progression; amelioration, palliation, or stabilization of the disease state; or other beneficial results. Treatment of cancer may, in some cases, result in partial response or stable disease.

A subject of "treating cancer or tumor" refers to any suitable species: mammals or fish, such as mammals, e.g., murines, dogs, cats, horses, or humans; preferably, the patient is a mammal, more preferably a human.

The definitions of isotopic variants or isomers in this invention are consistent with the definitions of isotopic variants and chiral isomers in Patent Application No. PCT/US2016/062114 (Publication No. WO2017087428), corresponding to Chinese Patent Application No. 2016800446081 (Publication No. CN108290911A).

Terms not explained or illustrated in detail in this application document shall be interpreted in accordance with textbooks of medicinal chemistry, organic chemistry, biochemistry, or pharmacology.

## Brief Description of the Drawings

Figure 1 shows the curves of inhibition rates of gemcitabine at different concentrations on H460 cancer cells in the presence or absence of AKR1C3 inhibitor AST-3021, wherein the abscissa (conc.Log(nM)) represents the logarithm value of the concentration value in nmol/L with base 10, and the ordinate (inhibition%) represents the percent inhibition rate.

Figure 2 shows the curves of inhibition rates of compound H11 at different concentrations on H460 cancer cells in the presence or absence of AKR1C3 inhibitor AST-3021, wherein the abscissa (conc.Log(nM)) represents the logarithm value of the concentration value in nmol/L with base 10, and the ordinate (inhibition%) represents the percent inhibition rate.

Figure 3 shows the curves of inhibition rates of compound H11 at different concentrations on HepG2 cancer cells in the presence or absence of AKR1C3 inhibitor AST-3021, wherein the abscissa (conc.Log(nM)) represents the logarithm value of the concentration value in nmol/L with base 10, and the ordinate (inhibition%) represents the percent inhibition rate.

Figure 4 shows the curves of tumor volume changes with days in the *in vivo* pharmacodynamic experiment of compound H11 and gemcitabine in pancreatic cancer PA1222 model.

Figure 5 shows the curves of weight change rates of mice with days in the *in vivo* pharmacodynamic experiment of

compound H11 and gemcitabine in pancreatic cancer PA1222 model.

## Detailed Description of the Invention

The present invention will be described below with reference to specific examples. Those skilled in the art can understand that these examples are only used for describing the invention and do not in any way limit its scope.

The experimental methods in the following examples are all conventional methods unless otherwise specified. The raw materials of the medicaments, the reagents and the like used in the following examples are all commercially available products unless otherwise specified.

### I. Test of inhibition of H460 cancer cells

*In vitro* human tumor cell line cytotoxicity assay was used.

*In vitro* proliferation data on the H460 non-small cell lung cancer human tumor cell line (cell line which highly expresses AKR1C3) is reported below in the compound table.

$IC_{50}$ values were obtained with the following steps:

Specifically, exponentially growing cells were seeded at a density of 2 × $10^3$ cells/100 μL/well in a 96-well plate and incubated at 37°C in 5% $CO_2$, 95% air and 100% relative humidity for 24 hours. The test compound was administered alone: 24 hours after cell plating, the compound acted alone, 99 μL of growth medium per well was added, and 1 μL of the compound prepared at 200 × was added, and the plate was shaken gently to ensure even mixing, then placed into an incubator of 37°C, 5% $CO_2$. After further cultivation for 72 hours, the number of live cells was detected using CTG. The CTG detection method was as follows: the cell plate to be tested was placed at room temperature to equilibrate for 30 minutes, and 100 μL of medium per well was discarded. 25 μL of CTG reagent (CellTiter-Glo® kit) per well was added, and the plate was placed in a high-speed shaker to oscillate for 2 minutes, and placed at room temperature in the dark for 30 minutes. The chemiluminescence signal value was read using a multimode microplate reader, with a read time of 1000 ms. The drug concentration resulting in growth inhibition of 50% ($IC_{50}$) was calculated using computer software, and the results were listed in Table 1, providing values corresponding to $IC_{50}$ (nM).

Similarly, in order to further verify that the compounds were activated by human AKR1C3 (aldosterone reductase family 1 member C3), the effect of some of the compounds on the proliferation of H460 cancer cells was tested with pretreatment with a specific AKR1C3 enzyme inhibitor AST-3021 (3 μM). The detailed experimental procedure was as follows: 24 hours after cell plating, 98 μL of growth medium per well was added, and 1 μL of 200 × compound AST-3021 was added to each well, and the plate was shaken gently to ensure even mixing, and then placed into an incubator; after 2 hours, 1 μL of the test compound prepared at 200 × was added, and the plate was shaken gently to ensure even mixing, and then placed into an incubator of 37°C, 5% $CO_2$. The inhibitor used was compound 36, i.e.

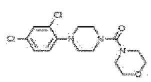,

in Flanagan et al., Bioorganic and Medicinal Chemistry (2014), page 962-977, and is referred to as AST-3021 in this invention. The values measured in this case are values corresponding to +AST-3021 $IC_{50}$ (nM) in Table 1.

The value corresponding to +AST-3021 $IC_{50}$ (nM) divided by the value corresponding to $IC_{50}$ (nM) is the specific activation ratio. The relative magnitude of the specific activation ratio can be used to evaluate whether this compound is a cytotoxic compound activated by human AKR1C3 (aldosterone reductase family 1 member C3): when the specific activation ratio is less than 1, it is considered that the compound is not activated by human AKR1C3 (aldosterone reductase family 1 member C3); when the specific activation ratio is greater than 1, it is considered that the compound is activated by human AKR1C3 (aldosterone reductase family 1 member C3), and the higher the value, the higher the activation degree and the selectivity of AKR1C3.

Table 1: Comparison data of $IC_{50}$ values and specific activation ratios (+AST-3021 $IC_{50}/IC_{50}$) of various compounds ± AST-3021

| Compound No. | Compound structure | ICso(nM) | +AST-3021 ICso (nM) | Comparative compound No. | Comparative compound structure | ICso (nM) | +AST-3021 ICso (nM) |
|---|---|---|---|---|---|---|---|
| H1 | 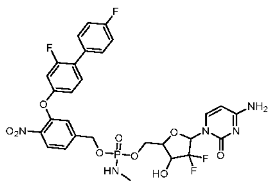 | 29.4 | 711.7 | / | / | | |
| | | specific activation ratio 24.2 | | | | | |
| H2 | 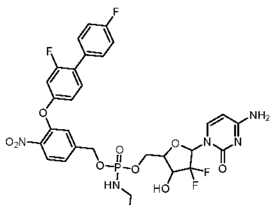 | 19.89 | 951 | D1 | 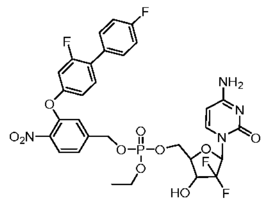 | 774.1 | 1223 |
| | | | | | | specific activation ratio 1.6 | |
| | | specific activation ratio 47.8 | | D2 | 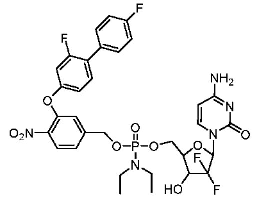 | 96.87 | 4371 |
| | | | | | | specific activation ratio 45.1 | |
| | | | | D3 | 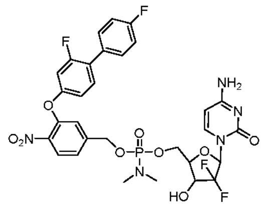 | 19.9 | 787.3 |
| | | | | | | specific activation ratio 39.6 | |
| H3 | 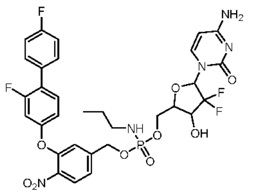 | 32.73 | 1491 | D4 | 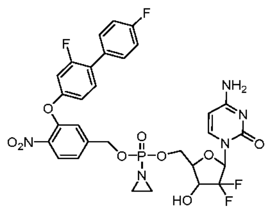 | 17.73 | 19.81 |
| | | specific activation ratio 45.6 | | | | specific activation ratio 1.1 | |

(continued)

| Compound No. | Compound structure | ICso(nM) | +AST-3021 ICso (nM) | Comparative compound No. | Comparative compound structure | ICso (nM) | +AST-3021 ICso (nM) |
|---|---|---|---|---|---|---|---|
| H4 | 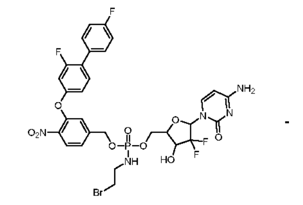 | 5.15 | 31.68 | D4 | 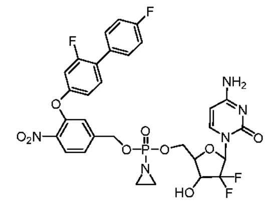 | 17.73 | 19.81 |
| | | specific activation ratio 6.2 | | | | specific activation ratio 1.1 | |
| H5 | 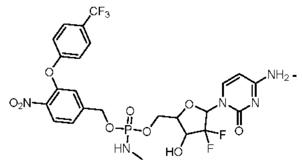 | 117.4 | 498.7 | / | / | | |
| | | specific activation ratio 4.2 | | | | | |
| H6 | 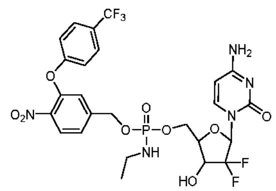 | 178.2 | 2304 | D5 | 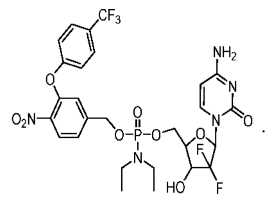 | 167.2 | 1474 |
| | | specific activation ratio 12.9 | | | | specific activation ratio 8.8 | |
| H7 | 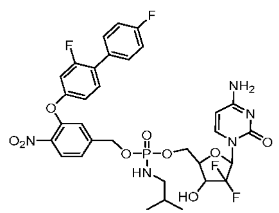 | 26.78 | 1642 | D2 | 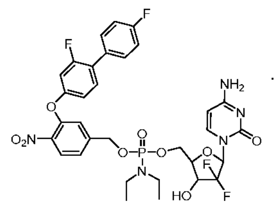 | 96.87 | 4371 |
| | | specific activation ratio 61.3 | | | | specific activation ratio 45.1 | |
| H8 | 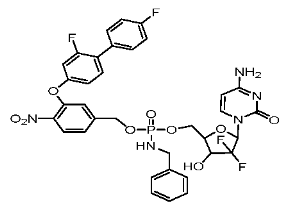 | 67.47 | 3022 | D6 | 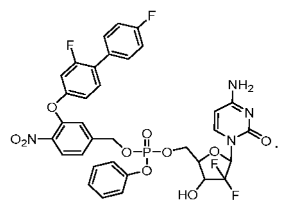 | 20.57 | 11.17 |
| | | specific activation ratio 44.8 | | | | specific activation ratio 0.54 | |

(continued)

| Compound No. | Compound structure | ICso(nM) | +AST-3021 ICso (nM) | Comparative compound No. | Comparative compound structure | ICso (nM) | +AST-3021 ICso (nM) |
|---|---|---|---|---|---|---|---|
| H9 | 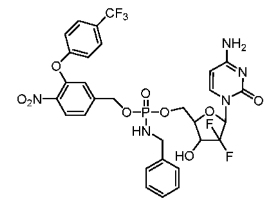 | 78.15 | 1811 | D7 | 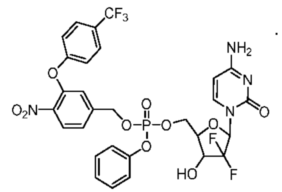 | 50.26 38.91 | |
| | | specific activation ratio 23.2 | | | | specific activation ratio 0.77 | |
| H10 | 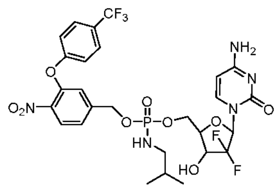 | 104.60 | 2902 | D5 | 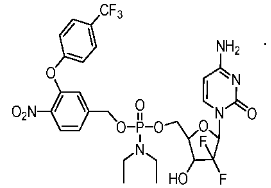 | 167.2 | 1474 |
| | | specific activation ratio 27.7 | | | | specific activation ratio 8.8 | |
| H11 | 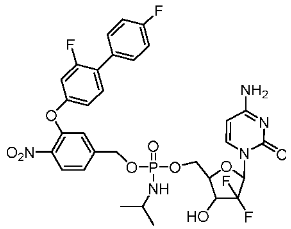 | 15.9 | 1476 | D8 | 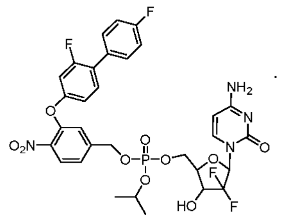 | / | / |
| | | specific activation ratio 92.8 | | | | / | |
| H12 | 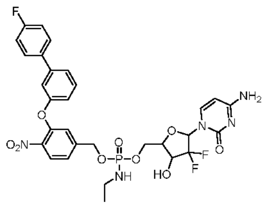 | 73.84 | 716.9 | / | / | | |
| | | specific activation ratio 9.7 | | | | | |

(continued)

| Compound No. | Compound structure | ICso(nM) | +AST-3021 ICso (nM) | Comparative compound No. | Comparative compound structure | ICso (nM) | +AST-3021 ICso (nM) |
|---|---|---|---|---|---|---|---|
| H13 | 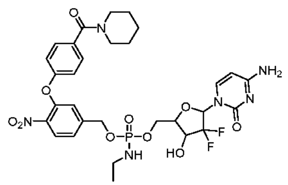 | 392.90 | 3677 | / | / | | |
| | | specific activation ratio 9.4 | | | | | |
| H14 | | 1772 | 3298 | / | / | | |
| | 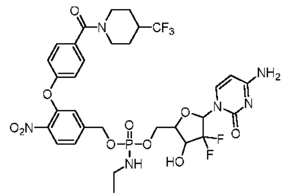 | specific activation ratio 1.9 | | | | | |
| H15 | 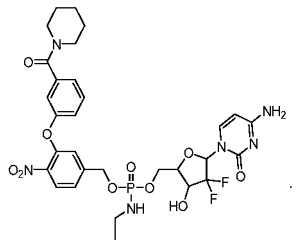 | 485.8 | 2416 | / | / | | |
| | | specific activation ratio 5.0 | | | | | |
| H16 | 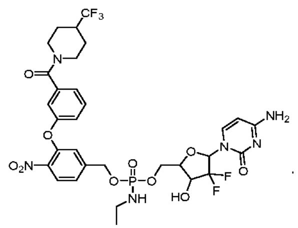 | 376.9 | 3373 | / | / | | |
| | | specific activation ratio 8.9 | | | | | |

(continued)

| Compound No. | Compound structure | ICso(nM) | +AST-3021 ICso (nM) | Comparative compound No. | Comparative compound structure | ICso (nM) | +AST-3021 ICso (nM) |
|---|---|---|---|---|---|---|---|
| H17 | 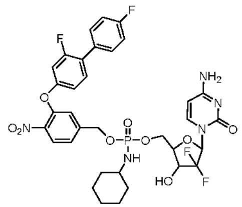 . | 396.9 | 10815 | / | / | | |
| | | specific activation ratio 27.2 | | | | | |
| H18 | 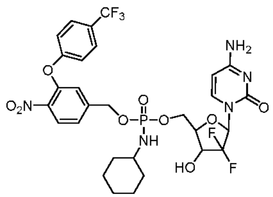 . | 460.8 | 1700 | / | / | | |
| | | specific activation ratio 3.7 | | | | | |
| H19 | 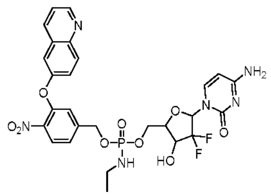 . | 150.1 | 510.3 | / | / | | |
| | | specific activation ratio 3.4 | | | | | |
| H20 | 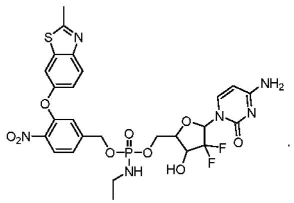 . | 231.5 | 1432 | / | / | | |
| | | specific activation ratio 6.2 | | | | | |

(continued)

| Compound No. | Compound structure | ICso(nM) | +AST-3021 ICso (nM) | Comparative compound No. | Comparative compound structure | ICso (nM) | +AST-3021 ICso (nM) |
|---|---|---|---|---|---|---|---|
| H21 | 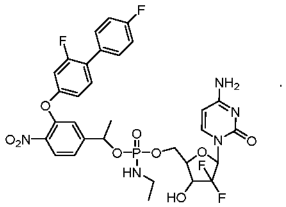 | 126.6 | 4412 | D9 | 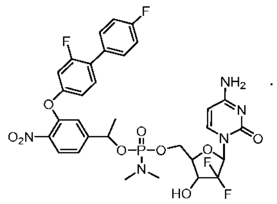 | 67.8 | 3830 |
| | | specific activation ratio 34.8 | | | | specific activation ratio 56.5 | |
| H22 | 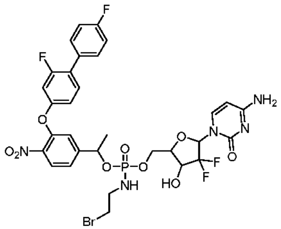 | 123.8 | 2373 | / | / | | |
| | | specific activation ratio 19.2 | | | | | |
| H23 | 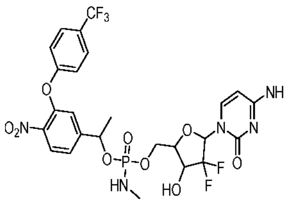 | 379 | 11306 | / | / | | |
| | | specific activation ratio 29.8 | | | | | |
| H24 | 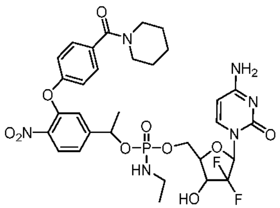 | 1124 | 21445 | / | / | | |
| | | specific activation ratio 19.1 | | | | | |

(continued)

| Compound No. | Compound structure | ICso(nM) | +AST-3021 ICso (nM) | Comparative compound No. | Comparative compound structure | ICso (nM) | +AST-3021 ICso (nM) |
|---|---|---|---|---|---|---|---|
| H25 | 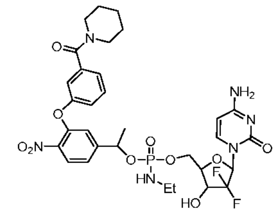 | 514.7 | 4935 | / | / | | |
| | | specific activation ratio 9.6 | | | | | |
| H26 | 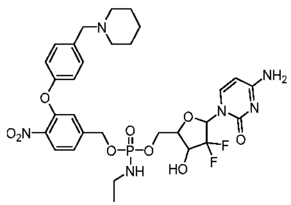 | / | / | / | / | | |
| | | / | | | | | |
| H27 | 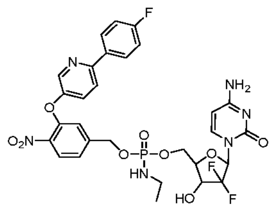 | 95.34 | 4319 | / | / | | |
| | | specific activation ratio 45.3 | | | | | |
| H28 | 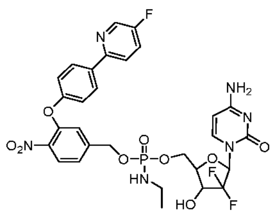 | 233.3 | 4763 | / | / | | |
| | | specific activation ratio 20.4 | | | | | |

(continued)

| Compound No. | Compound structure | ICso(nM) | +AST-3021 ICso (nM) | Comparative compound No. | Comparative compound structure | ICso (nM) | +AST-3021 ICso (nM) |
|---|---|---|---|---|---|---|---|
| H29 | 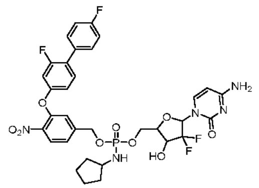 | 82.25 | 3880 | / | / | | |
| | | specific activation ratio 47.17 | | | | | |
| H30 | 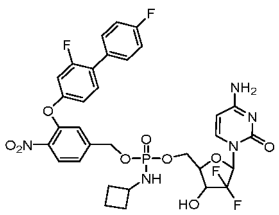 | 52.73 | 1371 | / | / | | |
| | | specific activation ratio 26.0 | | | | | |
| H31 | 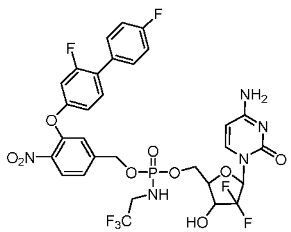 | 763.6 | 5288 | / | / | | |
| | | specific activation ratio 6.9 | | | | | |
| H32 | 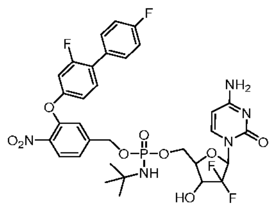 | 74.39 | 2582 | / | / | | |
| | | specific activation ratio 34.7 | | | | | |

(continued)

| Compound No. | Compound structure | ICso(nM) | +AST-3021 ICso (nM) | Comparative compound No. | Comparative compound structure | ICso (nM) | +AST-3021 ICso (nM) |
|---|---|---|---|---|---|---|---|
| H33 | 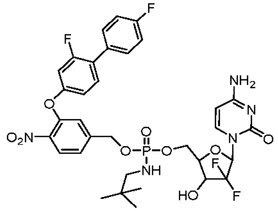 | 230.6 | 8603 | / | / | | |
| | | specific activation ratio 37.3 | | | | | |
| H34 | 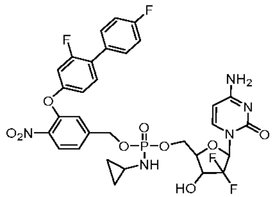 | 55.63 | 1038 | / | / | | |
| | | specific activation ratio 18.7 | | | | | |
| H35 | 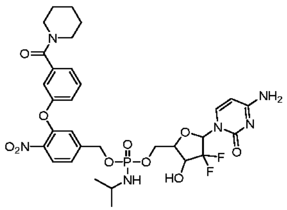 | 453.6 | 3898 | / | / | | |
| | | specific activation ratio 8.6 | | | | | |
| H36 | 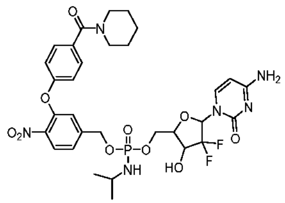 | 381.8 | 1171 | / | / | | |
| | | specific activation ratio 3.1 | | | | | |

(continued)

<table>
<tr><th>Compound No.</th><th>Compound structure</th><th>ICso(nM)</th><th>+AST-3021 ICso (nM)</th><th>Comparative compound No.</th><th>Comparative compound structure</th><th>ICso (nM)</th><th>+AST-3021 ICso (nM)</th></tr>
<tr><td rowspan="2">H37</td><td rowspan="2">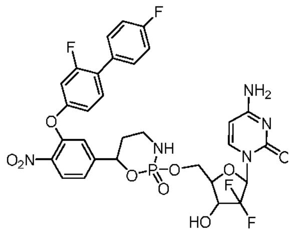</td><td>/</td><td>/</td><td rowspan="2">D10</td><td rowspan="2">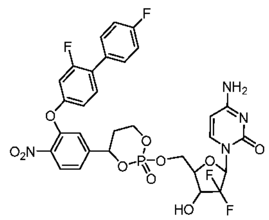</td><td colspan="2" rowspan="2">/</td></tr>
<tr><td colspan="2">/</td></tr>
<tr><td rowspan="2">H38</td><td rowspan="2">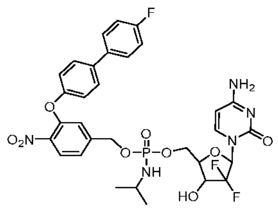</td><td>65.17</td><td>33080</td><td>/</td><td colspan="3" rowspan="2">/</td></tr>
<tr><td colspan="2">specific activation ratio 507.6</td><td>/</td></tr>
<tr><td rowspan="2">H39</td><td rowspan="2">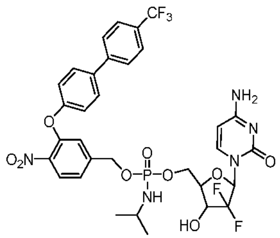</td><td>2183</td><td colspan="2">27739</td><td rowspan="2">/</td><td colspan="2" rowspan="2">/</td></tr>
<tr><td colspan="3">specific activation ratio 12.7</td></tr>
<tr><td rowspan="2">H40</td><td rowspan="2">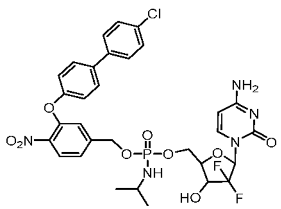</td><td>194.4</td><td colspan="2">16896</td><td rowspan="2">/</td><td colspan="2" rowspan="2">/</td></tr>
<tr><td colspan="3">specific activation ratio 86.9</td></tr>
</table>

(continued)

| Compound No. | Compound structure | ICso(nM) | +AST-3021 ICso (nM) | Comparative compound No. | Comparative compound structure | ICso (nM) | +AST-3021 ICso (nM) |
|---|---|---|---|---|---|---|---|
| H41 | 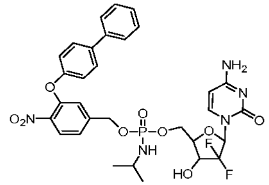 | 52.29 | | 23306 | / | / | |
| | | specific activation ratio 445.7 | | | | | |
| H42 | 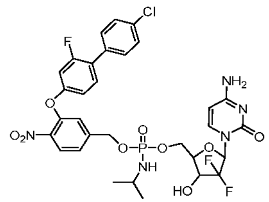 | 57.12 | | 19047 | / | / | |
| | | specific activation ratio 333.5 | | | | | |
| H43 | 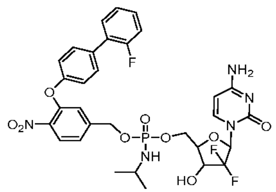 | 59.96 | | 15455 | / | / | |
| | | specific activation ratio 257.8 | | | | | |
| H44 | 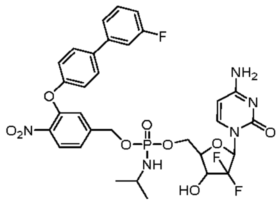 | 51.33 | | 17251 | / | / | |
| | | specific activation ratio 336.1 | | | | | |

The results of the experiment indicate that human AKR1C3 can activate the *in vitro* cytotoxicity of compounds H1~H44 above and compounds with similar structures; in other words, this series of compounds are anticancer gemcitabine prodrugs activated by AKR1C3. Specifically, by comparing the compounds on the left side of the above table with comparative compounds D1 toD10 on the right side, it can be seen that compounds of structural formula (I) where the N atom connected to $R_1$ is an imine structure (-NH-) have higher *in vitro* cytotoxicity compared with compounds of structural formula (comparative I) where the N atom connected to $R_1$ is a tertiary amine group (compared when the numbers of carbon atoms connected to N and the structures are similar): generally, both the values corresponding to $IC_{50}$ (nM) in the table and the values corresponding to +AST-3021 $IC_{50}$ (nM) in the table are lower (i.e. compounds of structural formula (I) have stronger cytotoxicity), and compound (I) has a higher AKR1C3 specific activation ratio than compound (comparative I).

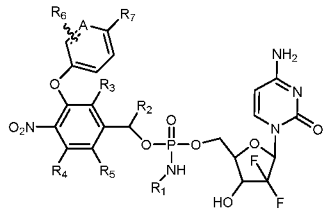 (I), 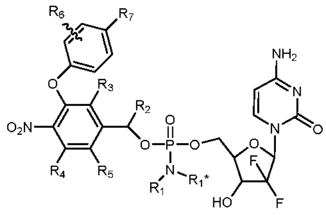 (comparative I),

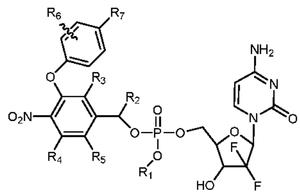 (comparative II).

Further, the experiment also confirmed that compounds of structural formula (comparative II) were not activated by AKR1C3 at all, and might not be substrates of AKR1C3.

That is to say, the present invention corrects the partially inaccurate view as indicated in the patent application entitiled NITROBENZYL DERIVATIVES OF ANTI-CANCER AGENTS with Patent Application No. PCT/US2016/025665 and Publication No. WO2016/161342A1, corresponding to Chinese Patent Application No. 201680020013.2 (Publication No. CN108136214A)), that nitrobenzyl derivatives:

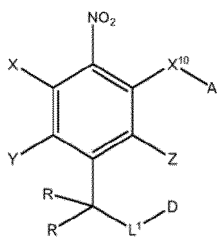

are all AKR1C3 activated anticancer drugs. In other words, the present invention clarifies that for gemcitabine-nitrobenzyl derivatives, compounds of structural formula (comparative II) are not activated by AKR1C3 at all, and finds by comparison of examples that compounds of structural formula (I) have higher cancer cell proliferation inhibition activity than compounds of structural formula (comparative I).

**II. Experiment on AKR1C3 activation dependent inhibitory activity against cell proliferation**

2.1 Test of inhibition by gemcitabine and compound H11 on H460 cancer cells in the presence or absence of the AKR1C3 inhibitor AST-3021

The experimental scheme was as follows:

1) H460 cell suspension was added to a 96-well plate with 100 μL per well at a cell density of 2000/well.

2) Cells were incubated in an incubator of 37°C, 5% $CO_2$ overnight.

3) Compound treatment

Combined administration: 24 hours after cell plating, 98 μL of growth medium per well was added. 1 μL of AST-3021 was added to each well (final concentration 3 μM), and the plate was shaken gently to ensure even mixing, and then placed into an incubator; after 2 hours, 1 μL of the test compound at different concentrations was added, and the plate was shaken gently to ensure even mixing, and then placed into an incubator of 37°C, 5% $CO_2$.

Administration of the drug alone: 24 hours after cell plating, 99 μL of growth medium per well was added. 1 μL of the test compound at different concentrations was added, and the plate was shaken gently to ensure even mixing, and then placed into an incubator of 37°C, 5% $CO_2$.

4) The cell plate was placed into an incubator for 72 hours.

5) The cell plate to be tested was placed at room temperature to equilibrate for 30 minutes, and 100 μL of medium per well was discarded.

6) 25 μL of CTG reagent per well was add, and the plate was placed in a high-speed shaker to oscillate for 2 minutes, and placed at room temperature in the dark for 30 minutes.

7) The chemiluminescence signal value was read using a multimode microplate reader, with a read time of 1000 ms.

8) $IC_{50}$ was calculated using GraphPad Prism 5 software, and the $IC_{50}$ (half inhibitory concentration) of the compound was obtained using the nonlinear fitting formula.

In order to further verify that the compound was activated specifically by human AKR1C3 (aldosterone reductase family 1 member C3), the compound was added following the pretreatment of cells with the specific AKR1C3 enzyme inhibitor AST-3021 (3 μM), and the effect of the presence of AST-3021 on the proliferation of H460 cancer cells was tested

$IC_{50}$ data of inhibitory effect of the compounds gemcitabine and H11 on H460 cancer cells in the presence or absence of the AKR1C3 inhibitor AST-3021 is shown in Table 2 and Table 3. $IC_{50}$ curves corresponding to gemcitabine are shown in Figure 1. $IC_{50}$ curves of compound H11 corresponding to experiment No. 3 are shown in Figure 2.

Table 2: $IC_{50}$ data of inhibitory effect of gemcitabine on H460 cancer cells in the presence or absence of the AKR1C3 inhibitor

| Compound | $IC_{50}$ (nmol/L) | | Specific activation ratio |
|---|---|---|---|
| | AST-3021 not added | AST-3021 added | |
| gemcitabine | 13.19 | 14.21 | 1.08 |

Table 3: $IC_{50}$ data of inhibitory effect of compound H11 on H460 cancer cells in the presence or absence of AKR1C3 inhibitor

| Experiment No. | Compound | $IC_{50}$ (nmol/L) | | Specific activation ratio |
|---|---|---|---|---|
| | | AST-3021 not added | AST-3021 added | |
| 1 | H11 | 16.25 | 1473.00 | 90.65 |
| 2 | H11 | 30.29 | 2348.00 | 77.52 |
| 3 | H11 | 35.70 | 2942.00 | 80.41 |

The test results show that the $IC_{50}$ values of gemcitabine with or without the addition of AST-3021 were close, with a specific activation ratio of only 1.08, indicating that the inhibitory activity of gemcitabine on cancer cells is not dependent on the expression of AKR1C3. However, the $IC_{50}$ values of compound H11 with or without the addition of AST-3021 vary significantly, with specific activation ratios reaching over 75 as shown by all the test results of three different times. This together with the relation between $IC_{50}$ specific activation ratios of gemcitabine and the addition of

AST-3021 demonstrates that compound H11 is an AKR1C3 activation dependent anticancer compound, and its *in vitro* activity is closely related to the expression of AKR1C3 enzyme.

2.2 Test of inhibition by compound H11 on HepG2 cancer cells in the presence or absence of the AKR1C3 inhibitor AST-3021

HepG2 cells with high expression of human AKR1C3 were selected, and $IC_{50}$ data of compound H11 on HepG2 cancer cells in the presence or absence of the AKR1C3 inhibitor AST-3021 was tested using the same test method as 2.1, as shown in Table 4. $IC_{50}$ curves corresponding to experiment No. 5 are shown in Figure 3.

Table 4: $IC_{50}$ data of inhibitory effect of compound H11 on HepG2 cancer cells in the presence or absence of AKR1C3 inhibitor

| Experiment No. | Compound | $IC_{50}$ (nmol/L) | | Specific activation ratio |
|---|---|---|---|---|
| | | AST-3021 not added | AST-3021 added | |
| 4 | H11 | 42.85 | 3350.00 | 78.18 |
| 5 | H11 | 39.82 | 1582.00 | 39.73 |
| 6 | H11 | 80.89 | 1660.00 | 20.52 |

The test results show that the $IC_{50}$ values of compound H11 with or without the addition of AST-3021 vary significantly, with specific activation ratios reaching over 20 as shown by all the test results of three different times, demonstrating that compound H11 is an AKR1C3 activation dependent anticancer compound for HepG2 cancer cells, and its *in vitro* activity is closely related to the expression of AKR1C3 enzyme.

**III. Comparison of compound H11 and gemcitabine in the *in vivo* pharmacodynamic experiment in pancreatic cancer PA1222 model**

Description of the experiment: Tumor tissue was collected from tumor bearing mice of HuPrime® pancreatic cancer xenograft model PA1222 (pancreatic cancer PDX model with high expression of AKR1C3), cut into tumor blocks with a diameter of 2-3 mm and inoculated subcutaneously at the right anterior scapula of BALB/c nude mice. When the average volume of tumor was about 102 $mm^3$, mice were randomly divided into groups according to the size of the tumor. Table 5 shows the design of the experiment on antitumor effect of test drugs in HuPrime® pancreatic cancer PA1222 tumor model.

Table 5: Design of the experiment on antitumor effect of test drugs in HuPrime® pancreatic cancer PA1222 tumor model

| **Group** | **Number of animals** | **Administration** | **Dosage (mg/kg)** | **Administration method** | **Administration cycle** |
|---|---|---|---|---|---|
| 1 | 5 | 10% anhydrous ethanol + 10% polyoxyl 35 castor oil + 80% glucose injection D5W (pH 7.7-8.0) | - | tail vein injection (i.v.) | Q2D × 3 weeks |
| 2 | 5 | gemcitabine | 80 | intraperitoneal injection (i.p.) | Q3D × 4 |
| 3 | 5 | H11 | 240 | intragastric administration (p.o.) | QD × 21 |
| 4 | 5 | H11 | 160 | intraperitoneal injection (i.p.) | QD × 5, 2 days off, 2 weeks off; QD × 5 |

(continued)

| Group | Number of animals | Administration | Dosage (mg/kg) | Administration method | Administration cycle |
|---|---|---|---|---|---|
| 5 | 5 | H11 | 160 | tail vein injection (i.v.) | Q2D × 3 weeks |
| Note: 1. The dosage volume was 10 μL/g. 2. Q3D × 4 means performing administration once every three days for four consecutive administrations; Q2D × 3 means performing administration once every two days for three consecutive weeks; QD × 21 means performing administration once a day for 21 consecutive days; QD × 5, 2 days off, 2 weeks off; QD × 5 means performing administration once a day for 5 consecutive days, stopping administration for 2 days, and for 2 weeks, and then performing administration continuously for 5 days, once a day. | | | | | |

Group 2, Group 3, Group 4, and Group 5 were ended on the 32nd day after the first administration, i.e. Day 32. Tumors were collected, weighed, photographed, and quick-frozen. On Day 57, Group 1 was ended, and tumors were collected, weighed, photographed, and quick-frozen. Efficacy evaluation was performed according to the relative tumor growth inhibition (TGI%), and safety evaluation was performed according to changes in animal weight and mortality.

Standard for efficacy evaluation and calculation methods were as follows:

The relative tumor proliferation rate, T/C %, is the percent ratio of relative tumor volume or weight between the treatment group and the control group at a certain time point. The calculation formula is as follows: T/C % = $T_{RTV}/C_{RTV}$ × 100% ($T_{RTV}$: average RTV of the treatment group; $C_{RTV}$: average RTV of the solvent control group; RTV = $V_t/V_0$, $V_0$ is the tumor volume of the animal at the time of grouping, and $V_t$ is the tumor volume of the animal after treatment);

The relative tumor growth inhibition, TGI (%), is calculated as follows: TGI % = (1 - T/C) × 100% (T and C are the average relative tumor volume (RTV) of the treatment group and the control group at a specific time point, respectively).

The efficacy of each group in the pancreatic cancer PA1222 model was analyzed on the 32nd day after the first administration, and the results are shown in Table 6. The average volumes of tumors on different days were recorded and averaged as shown in Table 7. The average tumor volumes in the first 32 days after the start of administration were plotted into curves, as shown in Figure 4.

Table 6: Pharmacodynamic analysis of each group in the HuPrime® pancreatic cancer PA1222 model

| Group of the experiment | The 32nd day after the first administration (i.e. Day 32, 10/4/2021) | | | | |
|---|---|---|---|---|---|
| | Tumor volume ($\bar{x}$ ± S) | Relative tumor - volume ($\bar{x}$ ± S) | TGI (%) | T/C (%) | P-value (vs. the control group) |
| Group 1 | 629.85 ± 136.87 | 6.21 ± 1.36 | - | - | - |
| Group 1 | 1247.07 ± 307.89 **(Day 56)** | 12.15 ± 2.89 | - | - | - |
| Group 2 | 6.79 ± 4.33 | 0.06 ± 0.04 | 99.03% | 0.97% | < 0.001 |
| Group 3 | 44.61 ± 22.14 | 0.47 ± 0.27 | 92.35% | 7.65% | < 0.001 |
| Group 4 | 202.58 ± 45.40 | 2.01 ± 0.41 | 67.62% | 32.38% | < 0.05 |
| Group 5 | 0.00 ± 0.00 | 0.00 ± 0.00 | 100.00% | 0.00% | < 0.001 |
| Note: 1. The data is represented as "mean ± standard error"; 2. T/C % = $T_{RTV}/C_{RTV}$ × 100%; TGI % = (1 - T/C) × 100%. | | | | | |

Table 7: Tumor volume change of mice of each group in the HuPrime® pancreatic cancer PA1222 model with treatment time

| **Tumor volume (mm$^3$)** ($\bar{x}$ ± S) | | | | | |
|---|---|---|---|---|---|
| **Time** | Group 1 | Group 2 | Group 3 | Group 4 | Group 5 |
| the 0th day of administration | 102.69 ± 6.88 | 102.95 ± 6.86 | 102.83 ± 6.64 | 101.91 ± 5.96 | 102.11 ± 6.00 |
| the 4th day of administration | 144.96 ± 13.68 | 104.07 ± 9.22 | 123.16 ± 3.48 | 105.94 ± 8.67 | 98.46 ± 3.72 |
| the 7th day of administration | 177.74 ± 24.98 | 74.43 ± 5.22 | 90.07 ± 4.65 | 96.63 ± 11.34 | 79.97 ± 3.69 |
| the 11th day of administration | 243.12 ± 34.25 | 38.92 ± 4.90 | 54.28 ± 5.75 | 116.07 ± 22.87 | 40.67 ± 3.20 |
| the 14th day of administration | 299.90 ± 51.57 | 24.66 ± 3.94 | 41.73 ± 7.84 | 139.61 ± 22.61 | 22.37 ± 2.70 |
| the 18th day of administration | 358.17 ± 54.38 | 17.66 ± 4.82 | 34.60 ± 7.08 | 153.86 ± 27.60 | 11.52 ± 4.90 |
| the 21st day of administration | 399.99 ± 56.79 | 12.13 ± 3.15 | 29.52 ± 12.59 | 188.69 ±33.45 | 11.56 ± 3.08 |
| the 25th day of administration | 490.19 ± 78.73 | 10.23 ± 3.05 | 30.71 ± 15.00 | 198.68 ± 40.97 | 6.73 ± 2.81 |
| the 28th day of | 540.46 ± | 7.97 ± | 30.59 ± | 195.90 ± | 0.00 ± 0.00 |
| administration | 97.48 | 3.70 | 12.12 | 41.71 | |
| the 32nd day of administration | 629.85 ± 136.87 | 6.79 ± 4.33 | 44.61 ± 22.14 | 202.58 ± 45.40 | 0.00 ± 0.00 |
| the 35th day of administration | 669.64 ± 145.73 | / | / | / | / |
| the 39th day of administration | 799.46 ± 184.86 | / | / | / | / |
| the 42nd day of administration | 912.51 ± 196.70 | / | / | / | / |
| the 46th day of administration | 1004.17 ± 295.28 | / | / | / | / |
| the 49th day of administration | 1064.65 ± 273.52 | / | / | / | / |
| the 53rd day of administration | 1154.21 ± 310.62 | / | / | / | / |
| the 56th day of administration | 1247.07 ± 307.89 | / | / | / | / |

In order to verify the effect of test drugs on the weight change of experimental animals, according to the above experimental method, the weight change of experimental animals were recorded simultaneously at different time points, as shown in Table 8. The weight growth rate was calculated and plotted as curves, as shown in Figure 5.

Table 8: Average weight (g) of mice on different days

| Day | 0 | 1 | 2 | 3 | 4 | 5 | 6 | 7 | 8 | 9 | 10 | 11 | 12 | 13 | 14 | 15 | 16 | 17 | 18 | 19 |
|---|---|---|---|---|---|---|---|---|---|---|---|---|---|---|---|---|---|---|---|---|
| Group 1 | 22.2 | / | 22.4 | / | 22.4 | / | / | 22.9 | 22.9 | / | 22.6 | 22.9 | 23.0 | / | 22.4 | / | / | / | 22.8 | / |
| Group 2 | 21.1 | / | / | 21.2 | 21.0 | / | 21.2 | 21.4 | / | 21.5 | / | 21.3 | / | / | 21.9 | / | / | / | 22.1 | / |
| Group 3 | 21.8 | 21.5 | 21.5 | 21.6 | 21.9 | 22.3 | 21.7 | 21.6 | 21.7 | 22.5 | 21.6 | 21.9 | 22.0 | 21.9 | 21.6 | 21.6 | 22.1 | 21.8 | 21.9 | 21.8 |
| Group 4 | 21.4 | 22.0 | 21.5 | 22.0 | 21.7 | / | / | 22.3 | / | / | / | 22.5 | / | / | 22.9 | / | / | / | 22.7 | / |
| Group 5 | 21.6 | / | 21.8 | / | 21.4 | / | / | 20.9 | 21.6 | / | 21.3 | 22.1 | 22.2 | / | 22.0 | / | 22.3 | / | 22.1 | / |
| Day | 20 | 21 | 22 | 23 | 24 | 25 | 26 | 28 | 32 | 33 | 35 | 39 | 40 | 42 | 46 | 47 | 49 | 53 | 56 | 57 |
| Group 1 | 22.6 | 23.0 | / | / | / | 23.6 | / | 23.1 | 23.4 | 23.5 | 23.3 | 23.7 | 24.1 | 23.6 | 24.1 | 24.2 | 23.5 | 24.2 | 23.7 | 22.9 |
| Group 2 | / | 22.6 | / | / | / | 22.3 | / | 22.1 | 23.1 | / | / | / | / | / | / | / | / | / | / | / |
| Group 3 | 21.9 | 21.8 | / | / | / | 22.6 | / | 22.4 | 22.9 | / | / | / | / | / | / | / | / | / | / | / |
| Group 4 | / | 22.9 | 22.6 | 22.5 | 22.6 | 22.5 | / | 22.6 | 23.3 | / | / | / | / | / | / | / | / | / | / | / |
| Group 5 | 22.1 | 22.3 | / | / | / | 22.6 | / | 22.5 | 23.2 | / | / | / | / | / | / | / | / | / | / | / |

The above experimental data indicates that:
Test drugs gemcitabine (80 mg/kg, Group 2), H11 240 mg/kg (Group 3, QD × 21) and H11 160 mg/kg, i.v. (Group 5, Q2D × 3 weeks) treatment groups showed extremely significant antitumor effect on the 32nd day after the first administration (Day 32), with relative tumor growth inhibition (TGI (%)) of 99.03%, 92.35%, and 100%, and complete tumor inhibition rates of 60%, 20%, and 100%, respectively. H11 160 mg/kg, i.p. (Group 4, QD × 5, 2 days off, 2 weeks off; QD × 5) treatment group showed significant antitumor effect on the 32nd day after the first administration (Day 32), with a relative tumor growth inhibition TGI (%) of 67.62%. Meanwhile, the antitumor effect of test drug H11 160 mg/kg, i.v. (Group 5, Q2D × 3 weeks) was better than that of H11 160 mg/kg, i.p. (Group 4, QD × 5, 2 days off, 2 weeks off; QD × 5), and there was a significant difference in statistics between the two groups (Group 5 and Group 4) ($P < 0.001$). The antitumor effect of test drug H11 160 mg/kg, i.v. (Group 5, Q2D × 3 weeks) was better than that of H11 240 mg/kg, p.o. (Group 3, QD × 21), and there was a significant difference in statistical comparison between the two groups (Group 5 and Group 3) ($P < 0.05$). The antitumor effect of test drug H11 160 mg/kg, i.v. (Group 5, Q2D × 3 weeks), H11 240 mg/kg, p.o. (Group 3, QD × 21) was not significantly different from that of the test drug gemcitabine (80 mg/kg, Group 2) treatment group, and there was no statistically significant difference between any two of the groups (Group 5 vs Group 2 and Group 3 vs Group 2) (*P*-values greater than 0.05).
The mice in the test drug gemcitabine 80 mg/kg treatment group, H11 240 mg/kg (Group 3, QD × 21) treatment group, H11 160 mg/kg, i.p. (Group 4, QD × 5, 2 days off, 2 weeks off; QD × 5) treatment group, and test drug H11 160 mg/kg, i.v. (Group 5, Q2D × 3 weeks) treatment group and the control group did not show any significant weight loss, and tolerated well during the treatment period.

**IV. Experiment on evaluation of metabolic stability**

4.1 Study on metabolic stability of compound H11 and the positive control verapamil in liver microsomes

Experimental scheme:

(I) Solution preparation
Main solutions were prepared according to Table 9 below:

Table 9: Preparation of main solutions

| Reagent | Material concentration | Volume | Final concentration |
|---|---|---|---|
| phosphate buffer | 100 mM | 216.25 μL | 100 mM |
| microsome | 20 mg/mL | 6.25 μL | 0.5 mg/mL |

(II) Two separate experiments were performed

a) With cofactor (NADPH): 25 μL of 10 mM NADPH was added to the culture broth. The final concentration of liver microsomes was 0.5 mg/mL, and the final concentration of NADPH was 1 mM.

b) Without cofactor (NADPH): 25 μL of 100 mM phosphate buffer was added. The final concentration of liver microsomes was 0.5 mg/mL. The mixture was preheated at 37°C for 10 minutes.

(III) 25 μL of 100 mM control compound or test compound solution was added to start the reaction. This study used verapamil as a positive control. The final concentrations of the test compound and control compound were both 1 μM. The culture broth was cultured in water at 37°C.
(IV) Aliquots of 30 μL were collected from the reaction solution at 0.5, 5, 15, 30, and 60 minutes, respectively. 5 volumes of cold acetonitrile and IS (internal standard, containing 100 nM of alprazolam, 200 nM of caffeine, and 100 nM of tolylbutanamide) were added to stop the reaction. Samples were centrifuged at a speed of 3,220 g for 40 minutes. 100 μL of supernatant was taken and mixed with 100 μL of ultrapure water, equally divided and analyzed with LC-MS/MS.
(V) Data analysis
All calculations were conducted using Microsoft Excel. The peak area was determined from the extracted ion chromatogram. The slope value k was determined by linear regression of the natural logarithm of the remaining percentage of the parent drug vs. incubation time curve.

(1) The *in vitro* half-life (*in vitro* $t_{1/2}$) was determined by the slope value: *in vitro* $t_{1/2}$ = -0.693/k.
(2) *In vitro* $t_{1/2}$ (min) was converted into *in vitro* intrinsic clearance (*in vitro* $CL_{int}$, μL/min/1 × $10^6$ cells), using the following formula (average of repeated measurements): $CL_{int}$ = -kV/N, wherein V = culture volume (0.2 mL) and N = number of liver cells per well (0.1 × $10^6$ cells).
(3) Scale-up $CL_{int}$ (mL/min/kg), Predicted Hepatic $CL_H$ (mL/min/kg), and Hepatic Extraction Ratio (ER) were calculated using the following formulas:

Scale-up$CL_{int}$ = (0.693/$T_{1/2}$) × (1/(liver cell concentration (0.5 × $10^6$ cells/mL))) × Scaling Factor (Table 10);

$$\text{Predicted Hepatic } CL_H = (QH \times \text{Scale-up } CL_{int} \times fub)/(QH + \text{Scale-up } CL_{int} \times fub);$$

$$ER = \text{Predicted Hepatic } CL_H/QH;$$

wherein, QH is the hepatic blood flow (mL/min/kg) in Table 10, and fub is the fraction of drug unbound in plasma, and assumed as 1.

Table 10: Scaling Factors for Intrinsic Clearance Prediction in human, monkey, dog, rat, and mouse hepatocytes

| Species | Liver microsome protein per gram of liver | Liver weight per kilogram of body weight | Scaling Factor | Hepatic Blood Flow (mL/min/kg) |
|---|---|---|---|---|
| human | 40 | 25.7 | 1028 | 21 |
| monkey | 40 | 30 | 1200 | 44 |
| dog | 55 | 32 | 1760 | 31 |
| rat | 61 | 40 | 2440 | 68 |
| mouse | 47 | 88 | 4136 | 90 |
| Note: Scaling Factor = (liver microsome protein per gram of liver) × (liver weight per kilogram of body weight). | | | | |

**Results of the experiment:**

The above experimental scheme was used to detect and calculate the metabolic stability data of compound H11 and the positive control verapamil in human, monkey, dog, rat, and mouse liver microsomes. Table 11 shows the change of the remaining percentage of compound H11 and verapamil in human, monkey, dog, rat, and mouse liver microsomes over time. Table 12 shows the metabolic stability data of compound H11 and verapamil in human, monkey, dog, rat, and mouse liver microsomes.

Table 11: Change of the remaining percentage of compound H11 and verapamil in human, monkey, dog, rat, and mouse liver microsomes over time

| **Compound** | **Species** | **Cofactor added or not** | **Remaining percentage (%)** | | | | |
|---|---|---|---|---|---|---|---|
| | | | **0.5 minutes** | **5 minutes** | **15 minutes** | **30 minutes** | **60 minutes** |
| verapamil | human | NADPH added | 100.00 | 40.59 | 9.10 | 2.75 | BLOD |
| | | NADPH not added | 100.00 | - | - | - | 104.85 |
| | monkey | NADPH added | 100.00 | 5.34 | BLOD | BLOD | BLOD |
| | | NADPH not added | 100.00 | - | - | - | 104.77 |
| | dog | NADPH added | 100.00 | 31.34 | 5.52 | 0.66 | BLOD |
| | | NADPH not added | 100.00 | - | - | - | 93.24 |
| | rat | NADPH added | 100.00 | 27.74 | 2.94 | BLOD | BLOD |
| | | NADPH not added | 100.00 | - | - | - | 103.79 |
| | mouse | NADPH added | 100.00 | 10.79 | BLOD | BLOD | BLOD |
| | | NADPH not added | 100.00 | - | - | - | 98.85 |
| H11 | human | NADPH added | 100.00 | 86.00 | 63.82 | 43.87 | 26.14 |
| | | NADPH not added | 100.00 | - | - | - | 100.86 |
| | monkey | NADPH added | 100.00 | 46.01 | 13.27 | 0.59 | 0.09 |
| | | NADPH not added | 100.00 | - | - | - | 105.87 |
| | dog | NADPH added | 100.00 | 90.20 | 71.99 | 52.53 | 32.12 |
| | | NADPH not added | 100.00 | - | - | - | 92.14 |
| | rat | NADPH added | 100.00 | 85.67 | 56.17 | 29.55 | 13.20 |
| | | NADPH not added | 100.00 | - | - | - | 103.20 |
| | mouse | NADPH added | 100.00 | 85.55 | 55.03 | 36.05 | 16.85 |
| | | NADPH not added | 100.00 | - | - | - | 93.32 |
| Note: BLOD indicates that the result is below the detection limit. | | | | | | | |

Table 12: Metabolic stability data of compound H11 and verapamil in human, monkey, dog, rat, and mouse liver microsomes

| Compound | Species | *In vitro* half-life $t_{1/2}$ (min) | *In vitro* intrinsic clearance $CL_{int}$ (μL/min/mg) | Scale-up $CL_{int}$ (mL/min/kg) | Predicted Hepatic $CL_H$ (mL/min/kg) | Hepatic Extraction Ratio (ER) |
|---|---|---|---|---|---|---|
| verapamil | human | 3.46 | 400.78 | 412.00 | 19.98 | 0.95 |
| | monkey | 1.06 | 1304.60 | 1565.52 | 42.80 | 0.97 |
| | dog | 2.69 | 515.70 | 907.63 | 29.98 | 0.97 |
| | rat | 2.43 | 569.96 | 1390.69 | 64.83 | 0.95 |
| | mouse | 1.40 | 989.53 | 4092.70 | 88.06 | 0.98 |

(continued)

| Compound | Species | *In vitro* half-life $t_{1/2}$ (min) | *In vitro* intrinsic clearance $CL_{int}$ (μL/min/mg) | Scale-up $CL_{int}$ (mL/min/kg) | Predicted Hepatic $CL_H$ (mL/min/kg) | Hepatic Extraction Ratio (ER) |
|---|---|---|---|---|---|---|
| H11 | human | 31.01 | 44.71 | 45.97 | 14.41 | 0.69 |
| | monkey | 5.06 | 274.95 | 329.94 | 38.81 | 0.88 |
| | dog | 36.33 | 38.16 | 67.17 | 21.21 | 0.68 |
| | rat | 20.11 | 68.99 | 168.33 | 48.43 | 0.71 |
| | mouse | 23.30 | 59.52 | 246.18 | 65.90 | 0.73 |

**Conclusion of the experiment:**

During the incubation process, the remaining percentage of the positive control verapamil significantly decreased with the prolongation of incubation time (Table 12), which is consistent with the characteristics of this positive drug. Therefore, the activity of liver microsomes in this experiment was normal, and the experimental results of test drug H11 are reliable.

The results show that the intrinsic clearances of H11 calculated after incubation with human, monkey, dog, rat, and mouse liver microsomes were 44.71 μL/min/$10^6$, 274.95 μL/min/$10^6$ 38.16 μL/min/$10^6$, 68.99 μL/min/$10^6$, and 59.52 μL/min/$10^6$ cells, respectively. H11 has a high clearance and poor stability in monkey liver microsomes; and medium clearances and certain stability in human, dog, rat, and mouse liver microsomes.

4.2 Study on plasma stability of compound H11

Experimental scheme:

(I) Solution preparation

1 mM working solution of the test compound was prepared in DMSO. 1 mM working solution of propantheline was prepared in acetonitrile. 1 mM working solution of lovastatin (mevinolin) was prepared in DMSO. Propantheline was used as a positive control for human, monkey, dog, and mouse plasma stability tests. Lovastatin was a positive control for rat plasma stability test.

(II) Experimental process

A. 398 μL of plasma per well was added to the culture plate, and the plate was preheated at 37°C for 15 minutes.

B. After preincubation, 2 μL of working solution (test solution or control solution) was added to the 398 μL plasma mentioned above, resulting in a final concentration of 5 μM. The final concentration of organic solvents was 0.5%. The test was conducted in duplicate.

C. The reaction sample was incubated at 37°C.

D. 50 μL was extracted from the reaction sample at 0, 15, 30, 60, and 120 minutes, respectively. 450 μL of cold acetonitrile with internal standard was added to stop the reaction.

E. All samples were vortexed for 10 minutes, followed by centrifugation at 3220 g for 30 minutes to precipitate the protein. 100 μL of supernatant was taken and transferred to a new plate. The supernatant was diluted with ultrapure water according to the LC-MS signal response and peak shape.

(III) Data analysis

Samples were analyzed with LC-MS/MS. All calculations were conducted using Microsoft Excel. The peak area ratio was determined from the extracted ion chromatogram.

(1) The percentage of the remaining compound at each time point was calculated with the following formula:

$$\text{remaining percentage t min (\%)} = \text{peak area ratio t min/peak area ratio 0 min} \times 100,$$

wherein the peak area ratio t min is the peak area ratio of the control compound to the test compound at t min, and the peak area ratio 0 min is the peak area ratio of the control compound to the test compound at zero time point.

(2) The *in vitro* half-life (*in vitro* $t_{1/2}$) was determined by the slope value:

$$t_{1/2} = -0.693/k,$$

wherein the slope value k was determined by linear regression of the natural logarithm of the remaining percentage of the parent drug vs. incubation time curve.

Results of the experiment:

The above experimental scheme was used to detect and calculate the metabolic stability data of compound H11 and the positive controls propantheline and lovastatin (mevinolin) in human, monkey, dog, rat, and mouse plasma, respectively. Table 13 shows the change of the remaining percentage of compound H11 and the positive controls propantheline and lovastatin in human, monkey, dog, rat, and mouse plasma over time.

Table 13: Change of the remaining percentage of compound H11 and propantheline and lovastatin in plasma over time

| Compound | Species | Remaining percentage (%) | | | | | $t_{1/2}$ (min) |
|---|---|---|---|---|---|---|---|
| | | 0 min | 15 min | 30 min | 60 min | 120 min | |
| propantheline | human | 100.00 | 81.24 | 59.99 | 32.76 | 7.75 | 36.64 |
| | monkey | 100.00 | 90.53 | 77.57 | 70.39 | 49.22 | 121.02 |
| | dog | 100.00 | 101.04 | 87.98 | 85.64 | 66.64 | 202.71 |
| lovastatin (mevinolin) | rat | 100.00 | 5.27 | 0.52 | 0.45 | 0.34 | 3.53 |
| propantheline | mouse | 100.00 | 69.89 | 43.11 | 15.28 | 2.03 | 21.83 |
| H11 | human | 100.00 | 95.74 | 94.17 | 102.77 | 99.46 | ∞ |
| | monkey | 100.00 | 100.61 | 92.99 | 103.66 | 98.48 | ∞ |
| | dog | 100.00 | 104.14 | 103.37 | 102.42 | 103.04 | ∞ |
| | rat | 100.00 | 108.59 | 105.39 | 100.74 | 109.53 | ∞ |
| | mouse | 100.00 | 102.19 | 96.83 | 104.70 | 97.53 | ∞ |

**Conclusion of the experiment:**

The experimental results of the positive controls propantheline and lovastatin are consistent with the characteristics of these positive drugs; therefore the activity of different plasma used in this experiment was normal, and the experimental results of test drug H11 are reliable.

Research data shows that H11 is stable and has no significant metabolism when incubated at 37°C in mouse, rat, dog, monkey, and human plasma for at least 2 hours.

V. **Maximal tolerance dose (MTD) test**

Experimental process:

According to the research scheme in Table 14, mice were grouped and administered at the concentrations and dosage levels shown in Table 14. On the 1st to 5th day and the 14th to 19th day, intraperitoneal injection of a solvent control or compound H11 at different dosage levels was performed, with a dose volume of 10mL/kg. Animals in Groups

1, 3, and 4 were put to death and autopsied on the 22nd day, and animals in Group 2 were put to death and autopsied on the 16th day.

Table 14: Research scheme

| Group | Treatment drug | Dosage level of administration (mg/kg/day) | Concentration (mg/mL) | Number of mice (male/female) |
|---|---|---|---|---|
| 1 | blank vehicle[a] | / | / | 2/2 |
| 2 | H11 | 80 | 8 | 3/3 |
| 3 | H11 | 160 | 16 | 3/3 |
| 4 | H11 | 320 | 32 | 3/3 |
| Note: a, conventional 10% polyoxyl (35) castor oil and 10% ethanol dissolved in 5% glucose injection. | | | | |

The evaluated parameters included cage-side observation, detailed clinical observation, and post-administration observation (approximately 1-2 hours after dosing), body weight and weight change, food intake, and gross pathology.

Experimental results:

(1) Mortality of animals
During the experiment, all experimental animals survived until the planned autopsy.

(2) Clinical symptoms
No abnormal compound-related clinical manifestation was observed.

(3) The impact on animal weight and food intake
No abnormal compound-related change in body weight or food intake was observed.

(4) Pathological examination results
Results of the compound-related pathological examination mainly include enlargement of the spleen visible at ≥160 mg/kg, and a change to white in the renal capsule visible in the 320 mg/kg dose group, and no other abnormalities.

Experimental conclusion:

Under the conditions of this experiment, mice were administered with compound H11 once a day for 5 consecutive days through two rounds of intraperitoneal injection. The safety to animals was good, and no abnormalities were observed except for spleen enlargement and changes in the renal capsule of the 320mg/kg dose group as observed through autopsy. Therefore, the maximal tolerance dose under the conditions of this experiment can reach 320 mg/kg (intraperitoneal injection once a day for 5 consecutive days). However, the MTD of gemcitabine reported by literature is 120 mg/kg (intraperitoneal injection, once every 3 days, for four consecutive doses) (Cancer Chemotherapy and Pharmacology 38 (4): 335-42). It is evident that as a prodrug of gemcitabine, H11 has a greater tolerance dose and is safer than gemcitabine.

**VI. Synthesis of compounds**

Description of reagents and instruments used is as follows:
THF stands for tetrahydrofuran; DCM stands for dichloromethane; EA or EtOAC stands for ethyl acetate; TEA stands for triethylamine; MTBE stands for methyl tert-butyl ether; DMAP stands for 4-dimethylaminopyridine; DBAD stands for di-tert-butyl azodicarboxylate; TFA stands for trifluoroacetic acid; EtOH stands for ethanol; t-BuOH stands for tert-butanol; DMF stands for dimethylformamide; PE stands for petroleum ether; TBAF stands for tetrabutylammonium fluoride; DIPEA stands for N,N-diisopropylethylamine; HPLC stands for high performance liquid chromatography; and LCMS stands for liquid chromatography mass spectrometry.
In the synthesis process, all the chemical reagents and drugs whose sources were not indicated were analytically

pure or chemically pure, and purchased from commercial reagent companies. Other English abbreviations mentioned herein are subject to the interpretation in the field of organic chemistry.

**Synthesis of compound H11**

Under $N_2$ protection, H11-A1 (6.0 g, 12.9 mmol) and $POCl_3$ (1.2 mL, 12.9 mmol) were added to dry DCM (60 mL), and a DCM (10 mL) solution of TEA (2.2 mL, 15.5 mmol) was dropwise added at -10°C and stirred. H11-A1 was purchased or synthesized with reference to other research literature or patents (Patent Application No. PCT/US2016/025665 (Publication No. WO2016/161342A1), which corresponds to Chinese Patent Application No. 201680020013.2 (Publication No. CN108136214A)).

After a period of time, the reaction was complete. A DCM solution of H11-A2 (4.6 g, 12.9 mmol, synthesized according to literature or purchased) and TEA (2.2 mL, 15.5 mmol) was added dropwise, and the mixture stirred.

A tetrahydrofuran solution of isopropylamine (2 M, 13.2 mL, 25.9 mmol) was added dropwise to the system, then TEA (5.4 mL, 10.1 mmol) was added, and the mixture was stirred at room temperature. Saturated $NH_4Cl$ solution was added dropwise, and the mixture was filtered, the filtrate separated, and the aqueous phase extracted with DCM. After washing with brine, the organic phase was dried with $Na_2SO_4$ and concentrated, followed by reversed-phase flash column chromatography to yield H11-D (2.0 g (above 85% content) and 2.0 g (50% content), with an overall yield of 25.2%), which was a light yellow solid. MS: Calculated 923.8, found 924.2 ($[M+H]^+$).

Under $N_2$ protection, H11-D (1.5 g, 1.62 mmol) was added to DCM (15 mL) and TFA (18.7 g, 165.2 mmol) was added. After the mixture was stirred at room temperature, the reaction was monitored till completion. The mixture was adjusted to pH = 8 with saturated $NaHCO_3$ aqueous solution and extracted with DCM. After washing with brine, the organic phase was dried with $Na_2SO_4$ and concentrated, followed by neutral preparative high performance liquid chromatography (using a mobile phase adjusted to neutral with acid or base) to isolate H11 (550 mg, yield 47.0%), which was a white solid.

$^1H$-NMR (400 MHz, $CD_3OD$) $\delta$ 8.04 (dd, $J$ = 8.4, 3.6 Hz, 1H), 7.60-7.48 (m, 4H), 7.39 (d, $J$ = 8.8 Hz, 1H), 7.31 (s, 1H), 7.18 (t, $J$ = 8.8 Hz, 2H), 6.94-6.91 (m, 2H), 6.26-6.13 (m, 1H), 5.84 (dd, $J$ = 7.6, 4.4 Hz, 1H), 5.12-5.09 (m, 2H), 4.34-4.17 (m, 3H), 4.04-4.01 (m, 1H), 3.37-3.31 (m, 1H), 1.15-1.12 (m, 6H). MS: Calculated MS, 723.2, found, 724.0 ($[M+H]^+$).

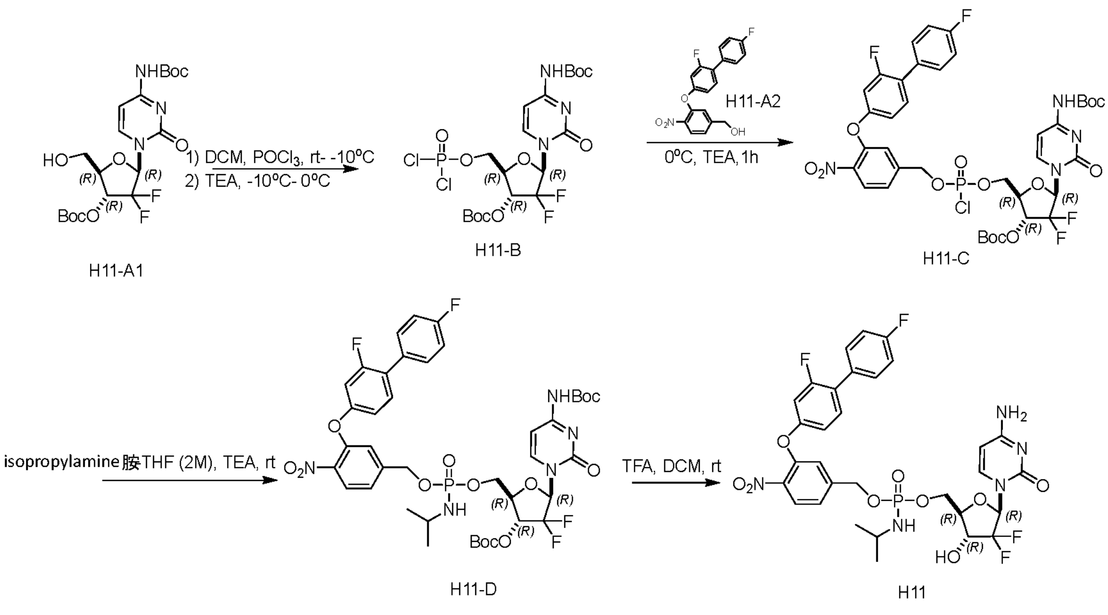

H11-A1
H11-B
H11-C
H11-D
H11

**Synthesis of other compounds**

The method was similar to the synthesis of compound H11, except that isopropylamine was changed to an organic amine or alcohol with other structures, such as methylamine, ethylamine, n-propylamine, 2-bromoethylamine, isobutylamine, 2-methyl-2-propylamine, 2,2-dimethyl-1-propylamine, benzylamine, cyclohexylamine, cyclopentamine, cyclobutylamine, cyclopropylamine, and 2,2,2-trifluoroethylamine; and H11-A2 was changed to other corresponding materials with structural formula as follows:

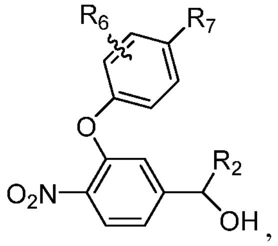

wherein, substituents $R_2$, $R_6$, and $R_7$ are defined as above;
With reference to the synthesis steps of compound H11 mentioned above, other similar compounds could be obtained. The spectral data of other compounds and H11 is listed below:

**Compound H1**

The product (9.3 mg, 6.0%) was isolated with neutral preparative high performance liquid chromatography as a white solid.

$^1$H-NMR (400 MHz, $CD_3OD$) $\delta$ 8.06-8.03 (m, 1H), 7.60-7.40 (m, 5H), 7.32 (s, 1H), 7.18 (t, $J$ = 8.8 Hz, 2H), 6.95-6.91 (m, 2H), 6.23-6.18 (m, 2H), 5.86-5.84 (m, 1H), 5.11 (d, $J$ = 8.4 Hz, 3H), 4.35-4.16 (m, 3H), 4.04-4.02 (m, 1H), 2.57-2.54 (m, 3H). MS: Calculated 695.1, found 696.2 ($[M+H]^+$).

**Compound H2**

The product (27.6 mg, 11.7%) was isolated with neutral preparative high performance liquid chromatography as a white solid.

$^1$H-NMR (400 MHz, $CD_3OD$) $\delta$ 8.06-8.03 (m, 1H), 7.55-7.49 (m, 4H), 7.42-7.39 (m, 1H), 7.31 (s, 1H), 7.21-7.15 (m, 2H), 6.95-6.91 (m, 2H), 6.23-6.17 (m, 1H), 5.85-5.83 (m, 1H), 5.12-5.10 (m, 2H), 4.34-4.16 (m, 3H), 4.04-4.02 (m, 1H), 2.97-2.89 (m, 2H), 1.13-1.08 (m, 3H). MS: Calculated 709.5, found 710.2 ($[M+H]^+$).

**Compound H3**

The product (71 mg, 29.2%) was isolated with neutral preparative high performance liquid chromatography as a white solid.

$^1$H-NMR (400 MHz, $CD_3OD$) $\delta$ 8.06-8.03 (m, 1H), 7.56-7.41 (m, 5H), 7.31 (s, 1H), 7.18 (t, $J$ = 8.8 Hz, 2H), 6.94-6.91 (m, 2H), 6.23-6.17 (m, 2H), 5.85-5.82 (m, 2H), 5.12-5.10 (m, 2H), 4.34-4.21 (m, 4H), 4.03-4.02 (m, 1H), 2.87-2.81 (m, 2H), 1.51-1.45 (m, 2H), 0.90-0.86 (m, 3H). MS: Calculated 723.2, found 724.0 ($[M+H]^+$).

**Compound H4**

The product (53.2 mg, 16.7%) was isolated with neutral preparative high performance liquid chromatography as a white solid.

$^1$H-NMR (400 MHz, $CD_3OD$) $\delta$ 8.04 (dd, $J$ = 8.4, 3.2 Hz, 1H), 7.61-7.46 (m, 4H), 7.42-7.40 (m, 1H), 7.32 (s, 1H), 7.20-7.16 (m, 2H), 6.94-6.91 (m, 2H), 6.24-6.20 (m, 1H), 5.87 (d, $J$ = 7.6 Hz, 1H), 5.14 (d, $J$ = 8.4 Hz, 2H), 4.38-4.18 (m, 3H), 4.06-4.03 (m, 1H), 3.42-3.38 (m, 2H), 3.28-3.25 (m, 2H). MS: Calculated 787.1, found 788.0 and 790.0 ($[M+H]^+$).

**Compound H5**

The product (26.3 mg, 11.1%) was isolated with neutral preparative high performance liquid chromatography as a white solid.

$^1$H-NMR (400 MHz, $CD_3OD$) $\delta$ 8.07 (dd, $J$ = 8.4, 4.0 Hz, 1H), 7.69 (d, $J$ = 8.8 Hz, 2H), 7.65-7.62 (m, 1H), 7.45 (d, $J$ = 8.4 Hz, 1H), 7.31 (s, 1H), 7.15 (d, $J$ = 7.6 Hz, 2H), 6.24-6.19 (m, 1H), 5.88-5.85 (m, 1H), 5.11 (d, $J$ = 8.4 Hz, 2H), 4.33-4.22 (m, 3H), 4.06-4.05 (m, 1H), 2.55 (dd, $J$ = 12.8, 2.0 Hz, 3H). MS: Calculated 651.1, found 652.0 ($[M+H]^+$).

**Compound H6**

The product (17.1 mg, 13.2%) was isolated with preparative high performance liquid chromatography as a white solid.

$^1$H-NMR (300 MHz, $CD_3OD$) $\delta$ 8.07 (d, $J$ = 8.7 Hz, 1H), 7.69 (d, $J$ = 8.7 Hz, 2H), 7.61-7.58 (m, 1H), 7.45 (d, $J$

= 8.2 Hz, 1H), 7.30 (s, 1H), 7.16 (d, $J$ = 8.1 Hz, 2H), 6.23-6.22 (m, 1H), 5.85 (d, $J$ = 7.5 Hz, 1H), 5.11 (d, $J$ = 8.4 Hz, 2H), 4.32-4.20 (m, Hz, 3H), 4.06-4.05 (m, 1H), 2.96-2.90 (m, 2H), 1.11 (t, $J$ = 6.7 Hz, 3H). MS: Calculated 665.1, found 666.0 ($[M+H]^+$).

**Compound H7**

The product (50.0 mg, 15.7%) was isolated with neutral preparative high performance liquid chromatography as a white solid.

$^1$H-NMR (400 MHz, $CD_3OD$) $\delta$ 8.03 (dd, $J$ = 8.4, 3.2 Hz, 1H), 7.59-7.41 (m, 5H), 7.32 (s, 1H), 7.18 (t, $J$ = 8.8 Hz, 2H), 6.93-6.91 (m, 2H), 6.21-6.16 (m, 1H), 5.86-5.84 (m, 1H), 5.11 (d, $J$ = 8.4 Hz, 2H), 4.30-4.24 (m, 3H), 4.05-4.0 (s, 1H), 2.72-2.68 (m, 2H), 1.74-1.61 (m, 1H), 0.88-0.86 (m, 6H). MS: Calculated 737.2, found 738.2 ($[M+H]^+$).

**Compound H8**

The product (49.5 mg, 15.3%) was isolated with neutral preparative high performance liquid chromatography as a white solid.

$^1$H-NMR (400 MHz, $CD_3OD$) $\delta$ 8.00 (dd, $J$ = 8.4, 3.2 Hz, 1H), 7.56-7.43 (m, 4H), 7.33-7.14 (m, 9H), 6.92-6.88 (m, 2H), 6.21-6.16 (m, 1H), 5.80-5.78 (m, 1H), 5.05-5.02 (m, 2H), 4.34-4.00 (m, 6H). MS: Calculated 771.2, found 772.1 ($[M+H]^+$).

**Compound H9**

The product (80.0 mg, yield 25.5%) was isolated with neutral preparative high performance liquid chromatography as a white solid.

$^1$H-NMR (400 MHz, $CD_3OD$) $\delta$ 8.03 (dd, $J$ = 8.4, 3.2 Hz, 1H), 7.67 (d, $J$ = 8.4 Hz, 2H), 7.54 (dd, $J$ = 16.0, 7.6 Hz, 1H), 7.37-7.35 (m, 1H), 7.31-7.20 (m, 6H), 7.12 (d, $J$ = 8.0 Hz, 2H), 6.22-6.17 (m, 1H), 5.80 (t, $J$ = 8.0 Hz, 1H), 5.08-5.02 (m, 2H), 4.35-4.10 (m, 3H), 4.10-4.01 (m, 3H). MS: Calculated MS, 727.1, found, 728.1 ($[M+H]^+$).

**Compound H10**

The product (65.1 mg, 21.3%) was isolated with neutral preparative high performance liquid chromatography as a white solid.

$^1$H-NMR (400 MHz, $CD_3OD$) $\delta$ 8.06 (dd, $J$ = 8.4, 3.2 Hz, 1H), 7.69 (d, $J$ = 8.7 Hz, 2H), 7.61-7.60 (m, 1H), 7.44 (d, $J$ = 8.5 Hz, 1H), 7.31 (s, 1H), 7.15 (d, $J$ = 8.4 Hz, 2H), 6.23-6.19 (m, 1H), 5.86 (dd, $J$ = 7.6, 2.4 Hz, 1H), 5.11 (d, $J$ = 8.0 Hz, 2H), 4.35-4.1 6 (m, 3H), 4.05-4.04 (m, 1H), 2.72-267 (m, 2H), 1.73-1.62 (m, 1H), 0.89-0.86 (m, 6H). MS: Calculated 693.2, found 694.1 ($[M+H]^+$).

**Compound H11**

The product (550 mg, 47.0%) was isolated with neutral preparative high performance liquid chromatography as a white solid.

$^1$H-NMR (400 MHz, $CD_3OD$) $\delta$ 8.04 (dd, $J$ = 8.4, 3.6 Hz, 1H), 7.60-7.48 (m, 4H), 7.39 (d, $J$ = 8.8 Hz, 1H), 7.31 (s, 1H), 7.18 (t, $J$ = 8.8 Hz, 2H), 6.94-6.91 (m, 2H), 6.26-6.13 (m, 1H), 5.84 (dd, $J$ = 7.6, 4.4 Hz, 1H), 5.12-5.09 (m, 2H), 4.34-4.17 (m, 3H), 4.04-4.01 (m, 1H), 3.37-3.31 (m, 1H), 1.15-1.12 (m, 6H). MS: Calculated MS, 723.2, found, 724.0 ($[M+H]^+$).

**Compound H12**

The product (24.6 mg, 8.3%) was isolated with neutral preparative high performance liquid chromatography as a white solid.

$^1$H-NMR (400 MHz, $CD_3OD$) $\delta$ 8.00 (dd, $J$ = 8.4, 2.8 Hz, 1H), 7.64-7.55 (m, 3H), 7.50-7.46 (m, 2H), 7.31-7.29 (m, 2H), 7.18-7.14 (m, 3H), 7.04-6.98 (m, 1H), 6.21-6.16 (m, 1H), 5.84-5.81 (m, 1H), 5.06 (d, $J$ = 8.0 Hz, 2H), 4.30-4.13 (m, 3H), 4.01-3.96 (m, 1H), 2.90-2.82 (m, 2H), 1.05 (t, $J$ = 6.8 Hz, 3H). MS: Calculated MS, 691.2, found, 692.2 ($[M+H]^+$).

**Compound H13**

The product (107.6 mg, yield 34.5%) was isolated with neutral preparative high performance liquid chromatography as a white solid.

$^1$H-NMR (400 MHz, $CD_3OD$) $\delta$ 8.03 (dd, J = 8.4, 3.2 Hz, 1H), 7.60 (dd, J = 7.4, 3.0 Hz, 1H), 7.44 (d, *J* = 8.4 Hz, 2H), 7.42-7.38 (m, 1H), 7.26 (s, 1H), 7.09 (dd, *J* = 8.6, 1.0 Hz, 2H), 6.24-6.19 (m, 1H), 5.84 (dd, *J* = 7.6, 2.8 Hz, 1H), 5.09 (d, *J* = 8.0 Hz, 2H), 4.36-4.15 (m, 3H), 4.05-4.02 (m, 1H), 3.77-3.64 (m, 2H), 3.55-3.37 (m, 2H), 2.97-2.89 (m, 2H), 1.75-1.51 (m, 6H), 1.13 -1.09 (m, 3H). MS: Calculated MS, 708.2, found, 709.2 ($[M+H]^+$).

**Compound H14**

The product (71.5 mg, yield 18.1%) was isolated with preparative high performance liquid chromatography as a white solid.

$^1$H-NMR (400 MHz, $CD_3OD$) $\delta$ 8.04 (dd, *J* = 8.4, 3.2 Hz, 1H), 7.61-7.58 (m, 1H), 7.48 (dd, *J* = 6.8, 1.6 Hz, 2H), 7.40 (dd, *J* = 8.4, 0.8 Hz, 1H), 7.27 (s, 1H), 7.10 (dd, *J* = 8.4, 1.6 Hz, 2H), 6.24-6.18 (m,1H), 5.84 (dd, *J* = 7.6, 2.8 Hz, 1H), 5.13-5.08 (m, 2H), 4.80-4.60 (m, 1H), 4.33- 4.14 (m, 3H), 4.05-4.03 (m, 1H), 4.03-3.70 (m, 1H), 3.23-3.13 (m, 1H), 2.97 -2.89 (m, 3H), 2.60-2.45 (m, 1H), 2.08-1.84 (m, 2H), 1.62-1.48 (m, 2H), 1.14-1.09 (m, 3H). MS: Calculated MS, 776.2, found, 777.2 ($[M+H]^+$).

**Compound H15**

The product (25.2 mg, yield 13.2%) was isolated with preparative high performance liquid chromatography as a white solid.

$^1$H-NMR (400 MHz, $CD_3OD$) $\delta$ 8.02 (dd, *J* = 8.4, 4.0 Hz,, 1H), 7.60 (dd, *J* = 7.6, 3.2 Hz, 1H), 7.49 (t, *J* = 8.0 Hz, 1H), 7.38 (d, *J* = 8.8 Hz, 1H), 7.26-7.18 (m, 2H), 7.15-7.13 (m, 1H), 7.02-7.01 (m, 1H), 6.25-6.15 (m, 1H), 5.84 (dd, J = 7.6, 2.4 Hz, 1H), 5.08 (d, *J* = 8.0 Hz, 2H), 4.33-4.15 (m, 3H), 4.05-4.01 (m, 1H), 3.72-3.65 (m, 2H), 3.40-3.30 (m, 2H), 2.95-2.85 (m, 2H), 1.75-1.45 (m, 6H), 1.13-1.08 (m, 3H). MS: Calculated MS, 708.2, found, 709.2 ($[M+H]^+$).

**Compound H16**

The product (110 mg, yield 33.7%) was isolated with preparative high performance liquid chromatography as a white solid.

$^1$H-NMR (400 MHz, $CD_3OD$) $\delta$ 8.02 (dd, *J* = 8.4, 4.0 Hz, 1H), 7.60 (dd, *J* = 7.6, 3.6 Hz, 1H), 7.50 (t, *J* = 8.0 Hz, 1H), 7.38 (d, *J* = 8.4 Hz, 1H), 7.24-7.20 (m, 2H), 7.17-7.12 (m, 1H), 7.05 (d, *J* = 1.2 Hz,1H), 6.26-6.18(m, 1H), 5.83(dd, *J* = 7.6, 2.8 Hz, 1H), 5.08 (d, *J* = 8.4 Hz, 2H), 4.77-4.63 (m, 1H), 4.33-4.15 (m, 3H), 4.05-4.02 (m, 1H), 3.82-3.73 (m, 1H), 3.18-3.10 (m, 1H), 2.97-2.80 (m, 3H), 2.60-2.42 (m, 1H), 2.05-1.83 (m, 2H), 1.60-1.40 (m, 2H), 1.14-1.08 (m, 3H). MS: Calculated MS, 776.2, found, 777.2 ($[M+H]^+$).

**Compound H17**

The product (39.6 mg, yield 11.1%) was isolated with preparative high performance liquid chromatography as a white solid.

$^1$H-NMR (400 MHz, $CD_3OD$) $\delta$ 8.04 (dd, *J* = 8.4, 2.4 Hz, 1H), 7.62-7.45 (m, 4H), 7.40 (dd, *J* = 8.4, 1.6 Hz, 1H), 7.32 (s, 1H), 7.18 (t, *J* = 8.8 Hz, 2H), 6.95-6.91 (m, 2H), 6.26-6.16 (m, 1H), 5.84 (dd, *J* = 7.6, 1.6 Hz, 1H), 5.11 (d, *J* = 8.4 Hz, 2H), 4.35-4.16 (m, 3H), 4.05-4.00 (m, 1H), 2.96-2.85 (m, 1H), 1.91-1.85 (m, 2H), 1.76-1.64 (m, 2H), 1.58-1.50 (m, 1H), 1.30-1.07 (m, 5H). MS: Calculated MS, 763.2, found, 764.2 ($[M+H]^+$).

**Compound H18**

The product (36.1 mg, yield 15.9%) was isolated with preparative high performance liquid chromatography as a white solid.

$^1$H-NMR (400 MHz, $CD_3OD$) $\delta$ 8.07 (dd, *J* = 8.4, 2.4 Hz, 1H), 7.69 (d, *J* = 8.8 Hz, 2H), 7.61 (d, *J* = 7.2 Hz, 1H), 7.44 (d, *J* = 8.0 Hz,1H), 7.31 (s, 1H), 7.15 (d, *J* = 8.4 Hz, 2H), 6.26-6.17 (m, 1H), 5.86 (d, *J* = 7.6 Hz, 1H), 5.11 (d, *J* = 8.0 Hz, 2H), 4.33-4.15 (m, 3H), 4.06-4.00 (m, 1H), 2.95-2.87 (m, 1H), 1.90-1.82 (m, 2H), 1.75-1.62 (m, 2H), 1.60-1.51 (m, 1H), 1.30-1.09 (m, 5H). MS: Calculated MS, 719.2, found, 720.2 ($[M+H]^+$).

**Compound H19**

The product (5.4 mg, yield 2.8%) was isolated with preparative high performance liquid chromatography as a white solid.

$^1$H-NMR (400 MHz, $CD_3OD$) $\delta$ 8.81 (dd, *J* = 4.4, 1.6 Hz, 1H), 8.28 (d, *J* = 8.0 Hz, 1H), 8.12-8.03 (m, 2H), 7.62-7.51 (m, 3H), 7.47-7.45 (m, 1H), 7.41 (d, *J* = 8.4 Hz, 1H), 7.29 (s, 1H), 6.20-6.11 (m, 1H), 5.83 (t, *J* = 7.6 Hz, 1H),

5.09 (d, $J$ = 8.4 Hz, 2H), 4.29-4.12 (m, 3H), 3.97-3.90 (m, 1H), 2.90-2.80 (m, 2H), 1.06-1.00 (m, 3H). MS: Calculated MS, 648.2, found, 649.2 ($[M+H]^+$).

**Compound H20**

The product (23 mg, yield 11.5%) was isolated with preparative high performance liquid chromatography as a light yellow solid.

$^1$H-NMR (400 MHz, $CD_3OD$) $\delta$ 8.01 (dd, $J$ = 8.4, 2.4 Hz, 1H), 7.91 (d, $J$ = 8.8 Hz, 1H), 7.63 (t, $J$ = 2.4 Hz, 1H), 7.55 (dd, $J$ = 10.8, 7.6 Hz, 1H), 7.31 (d, $J$ = 8.4 Hz, 1H), 7.24 (dd, $J$ = 8.8, 1.2 Hz, 1H), 7.15 (s, 1H), 6.24-6.13 (m, 1H), 5.81 (t, $J$ = 7.8 Hz, 1H), 5.09-5.01 (m, 2H), 4.28-4.10 (m, 3H), 3.98-3.90 (m, 1H), 2.88-2.78 (m, 5H), 1.04 (t, $J$ = 6.4 Hz, 3H). MS: Calculated MS, 668.1, found, 669.1 ($[M+H]^+$).

**Compound H21**

The product (16.8 mg, yield 16.5%) was isolated with preparative high performance liquid chromatography as a white solid.

$^1$H-NMR (400 MHz, $CD_3OD$) $\delta$ 8.08-8.01 (m, 1H), 7.69-7.37 (m, 5H), 7.31-7.30 (m, 1H), 7.18 (t, $J$ = 9.6 Hz, 2H), 6.95-6.87 (m, 2H), 6.27-6.11 (m, 1H), 5.90-5.82 (m, 1H), 5.53-5.42 (m, 1H), 4.38-3.92 (m, 4H), 2.97-2.73 (m, 2H), 1.64-1.59 (m, 3H), 1.15-1.00 (m, 3H). MS: Calculated MS, 723.2, found, 724.2 ($[M+H]^+$).

**Compound H22**

The product (54.0 mg, yield 22.5%) was isolated with preparative high performance liquid chromatography as a light yellow solid.

$^1$H-NMR (400 MHz, $CD_3OD$) $\delta$ 8.08-8.01 (m, 1H), 7.63-7.40 (m, 5H), 7.33-7.30 (m, 1H), 7.18 (t, $J$ = 8.8 Hz, 2H), 6.95-6.90 (m, 2H), 6.25-6.10 (m, 1H), 5.90-5.82 (m, 1H), 5.59-5.50 (m, 1H), 4.40-3.92 (m, 4H), 3.49-3.12 (m, 4H), 1.67-1.59 (m, 3H). MS: Calculated MS, 801.1, 803.1, found, 802.0, 804.0 ($[M+H]^+$).

**Compound H23**

The product (5.7 mg, yield 5.0%) was isolated with preparative high performance liquid chromatography as a white solid.

$^1$H-NMR (400 MHz, $CD_3OD$) $\delta$ 8.10-8.03 (m, 1H), 7.75-7.45 (m, 4H), 7.34-7.30 (m, 1H), 7.16-7.11 (m, 2H), 6.27-6.13 (m, 1H), 5.89-5.82 (m, 1H), 5.55-5.46 (m, 1H), 4.38-3.95 (m, 4H), 2.60-2.40(m, 3H), 1.65-1.60 (m, 3H). MS: Calculated MS, 665.1, found, 666.2 ($[M+H]^+$).

**Compound H24**

The product (6.3 mg, yield 5.0%) was isolated with preparative high performance liquid chromatography as a white solid.

$^1$H-NMR (400 MHz, $CD_3OD$) $\delta$ 8.08-8.01 (m, 1H), 7.69-7.52 (m, 1H), 7.47-7.40 (m, 3H), 7.26 (s, 1H), 7.13-7.05 (m, 2H), 6.28-6.15 (m, 1H), 5.91-5.82 (m, 1H), 5.52-5.42 (m, 1H), 4.35-3.95 (m, 4H), 3.76-3.68 (m, 2H), 3.45-3,32 (m, 2H), 2.98-2.75 (m, 2H), 1.78-1.50 (m, 9H), 1.17-1.02 (m, 3H). MS: Calculated MS, 722.2, found, 723.2 ($[M+H]^+$).

**Compound H25**

The product (4.7 mg, yield 3.8%) was isolated with preparative high performance liquid chromatography as a white solid.

$^1$H-NMR (400 MHz, $CD_3OD$) $\delta$ 8.06-7.98 (m, 1H), 7.67-7.35 (m, 3H), 7.27-7.10 (m, 3H), 7.01 (s, 1H), 6.28-6.13 (m, 1H), 5.90-5.80 (m, 1H), 5.52-5.42 (m, 1H), 4.38-3.95 (m, 4H), 3.73-3.63 (m, 2H), 3.42-3.32 (m, 2H), 2.97-2.73 (m, 2H), 1.76-1.48 (m, 9H), 1.17-1.00 (m, 3H). MS: Calculated MS, 722.2, found, 723.3 ($[M+H]^+$).

**Compound H27**

The product (64.3 mg, yield 29.6%) was isolated with preparative high performance liquid chromatography as a white solid.

$^1$H-NMR (400 MHz, $CD_3OD$) $\delta$ 8.41 (t, $J$ = 2.0 Hz, 1H), 8.07 (dd, $J$ = 8.4, 3.2 Hz, 1H), 8.03-7.98 (m, 2H), 7.88 (d, $J$ = 8.8 Hz, 1H), 7.60 (t, $J$ = 8.0 Hz, 1H), 7.57-7.52 (m, 1H), 7.42 (dd, $J$ = 8.4, 1.6 Hz, 1H), 7.30 (d, $J$ = 1.6 Hz, 1H),

7.21 (t, $J$ = 8.8 Hz, 2H), 6.25-6.15 (m, 1H), 5.88-5.83 (m, 1H), 5.16-5.07 (m, 2H), 4.35-4.15 (m, 3H), 4.07-3.98 (m, 1H), 2.96-2.85 (m, 2H), 1.13-1.05 (m, 3H). MS: Calculated MS, 692.2, found, 692.7 ($[M+H]^+$).

**Compound H28**

The product (23.8 mg, yield 19.2%) was isolated with preparative high performance liquid chromatography as a white solid.

$^1$H-NMR (400 MHz, $CD_3OD$) $\delta$ 8.51 (d, $J$ = 2.8 Hz, 1H), 8.05-8.01 (m, 3H), 7.90 (dd, $J$ = 8.8, 4.0 Hz, 1H), 7.70-7.62 (m, 1H), 7.59 (dd, $J$ = 9.8, 7.6 Hz, 1H), 7.34 (d, $J$ = 8.4 Hz, 1H), 7.23 (s, 1H), 7.165 (dd, $J$ = 8.8, 2.0 Hz, 2H), 6.25-6.15 (m, 1H), 5.83 (t, $J$ = 6.8 Hz, 1H), 5.14-5.03 (m, 2H), 4.31-4.13 (m, 3H), 4.05-3.97 (m, 1H), 2.94-2.85 (m, 2H), 1.12-1.05 (m, 3H). MS: Calculated MS, 692.2, found, 692.7 ($[M+H]^+$).

**Compound H29**

The product (3 mg, yield 1.5%) was isolated with preparative high performance liquid chromatography as a white solid.

$^1$H-NMR (400 MHz, $CD_3OD$) $\delta$ 8.04 (dd, $J$ = 8.4, 3.2 Hz, 1H), 7.60-7.47 (m, 4H), 7.39 (d, $J$ = 8.4 Hz, 1H), 7.32 (s, 1H), 7.18 (t, $J$ = 8.8 Hz, 2H), 6.95-6.91 (m, 2H), 6.27-6.17 (m, 1H), 5.83 (dd, $J$ = 7.6, 4.4 Hz, 1H), 5.10 (d, $J$ = 8.0 Hz, 2H), 4.38-4.18 (m, 3H), 4.07-4.01 (m, 1H), 3.53-3.43 (m, 1H), 1.90-1.78 (m, 2H), 1.75-1.63 (m, 2H), 1.58-1.38 (m, 4H). MS: Calculated MS, 749.2, found, 750.2 ($[M+H]^+$).

**Compound H30**

The product (45 mg, yield 26.0%) was isolated with preparative high performance liquid chromatography as a white solid.

$^1$H-NMR (400 MHz, $CD_3OD$) $\delta$ 8.04 (dd, $J$ = 8.4, 3.2 Hz, 1H), 7.65-7.46 (m, 4H), 7.39 (d, $J$ = 8.4 Hz, 1H), 7.30 (s, 1H), 7.18 (t, $J$ = 8.8 Hz, 2H), 6.93 (dd, $J$ = 10.8, 2.0 Hz, 2H), 6.27-6.17 (m, 1H), 5.90-5.83 (m, 1H), 5.15-5.04 (m, 2H), 4.30-4.14 (m, 3H), 4.07-4.02 (m, 1H), 3.68-3.52 (m, 1H), 2.25-2.10 (m, 2H), 1.97-1.83 (m, 2H), 1.67-1.43 (m, 2H). MS: Calculated MS, 735.2, found, 736.1 ($[M+H]^+$).

**Compound H31**

The product (79 mg, yield 37.1%) was isolated with preparative high performance liquid chromatography as a white solid.

$^1$H-NMR (400 MHz, $CD_3OD$) $\delta$ 8.04 (dd, $J$ = 8.4, 4.8 Hz, 1H), 7.65-7.46 (m, 4H), 7.41 (d, $J$ = 8.4 Hz, 1H), 7.32 (s, 1H), 7.18 (t, $J$ = 8.8 Hz, 2H), 6.93-6.88 (m, 2H), 6.27-6.17 (m, 1H), 5.90-5.81 (m, 1H), 5.19-5.10 (m, 2H), 4.40-4.19 (m, 3H), 4.08-4.02 (m, 1H), 3.68-3.52 (m, 2H). MS: Calculated MS, 763.1, found, 764.0 ($[M+H]^+$).

**Compound H32**

The product (15.4 mg, yield 13.9%) was isolated with preparative high performance liquid chromatography as a white solid.

$^1$H-NMR (400 MHz, $CD_3OD$) $\delta$ 8.03 (dd, $J$ = 8.4, 5.2 Hz,1H), 7.60-7.48 (m, 4H), 7.43-7.38 (m, 1H), 7.31 (s, 1H), 7.18 (t, $J$ = 8.8 Hz, 2H), 6.95-6.88 (m, 2H), 6.24-6.16 (m, 1H), 5.86-5.82 (m, 1H), 5.17-5.10 (m, 2H), 4.37-4.18 (m, 3H), 4.08-4.00 (m, 1H), 1.27 (s, 9H). MS: Calculated MS, 737.2, found, 738.1 ($[M+H]^+$).

**Compound H33**

The product (5.5 mg, yield 2.7%) was isolated with preparative high performance liquid chromatography as a white solid.

$^1$H-NMR (400 MHz, $CD_3OD$) $\delta$ 8.04 (dd, $J$ = 8.4, 3.6 Hz, 1H), 7.67-7.45 (m, 4H), 7.40 (d, $J$= 8.4 Hz, 1H), 7.32 (s, 1H), 7.18 (t, $J$ = 8.8 Hz, 2H), 6.92 (dd, $J$ = 10.4, 1.6 Hz, 2H), 6.25-6.15 (m, 1H), 5.85 (dd, $J$ = 7.6, 4.4 Hz, 1H), 5.18-5.13 (m, 2H), 4.38-4.18 (m, 3H), 4.08-4.00 (m, 1H), 2.68 (d, $J$ = 10.4 Hz, 2H), 0.89-0.84 (m, 9H). MS: Calculated MS, 751.2, found, 752.2 ($[M+H]^+$).

**Compound H34**

The product (35.8 mg, yield 24.9%) was isolated with preparative high performance liquid chromatography as

a white solid.

$^1$H-NMR (400 MHz, $CD_3OD$) $\delta$ 8.04 (dd, $J$ = 8.4, 3.2 Hz, 1H), 7.66-7.45 (m, 4H), 7.40 (d, $J$ = 8.4 Hz, 1H), 7.32 (s, 1H), 7.18 (t, $J$ = 8.8 Hz, 2H), 6.99-6.91 (m, 2H), 6.28-6.15 (m, 1H), 5.84 (t, $J$ = 8.0 Hz, 1H), 5.20-5.10 (m, 2H), 4.40-4.18 (m, 3H), 4.08-4.00 (m, 1H), 2.38-2.27 (m, 1H), 0.58-0.45 (m, 4H). MS: Calculated MS, 721.2, found, 722.1 ($[M+H]^+$).

**Compound H35**

The product (36.7 mg, yield 12.3%) was isolated with preparative high performance liquid chromatography as a white solid.

$^1$H-NMR (400 MHz, $CD_3OD$) $\delta$ 8.02 (dd, $J$ = 8.4, 4.4 Hz, 1H), 7.60 (d, $J$ = 7.6 Hz, 1H), 7.49 (t, $J$ = 8.0 Hz, 1H), 7.37 (d, $J$ = 8.4 Hz, 1H), 7.26-7.18 (m, 2H), 7.14 (dd, $J$ = 8.0, 2.4 Hz, 1H), 7.02 (d, $J$ = 1.6 Hz, 1H), 6.28-6.21 (m, 1H), 5.90-5.83 (m, 1H), 5.08 (d, $J$ = 8.0 Hz, 2H), 4.38-4.25 (m, 3H), 4.08-4.02 (m, 1H), 3.74-3.65 (m, 2H), 3.40-3.34 (m, 3H), 1.78-1.45 (m, 6H), 1.19-1.12 (m, 6H). MS: Calculated MS, 722.3, found, 723.2 ($[M+H]^+$).

**Compound H36**

The product (26.6 mg, yield 8.9%) was isolated with preparative high performance liquid chromatography as a white solid.

$^1$H-NMR (400 MHz, $CD_3OD$) $\delta$ 8.03 (dd, $J$ = 8.4, 3.6 Hz, 1H), 7.60 (dd, $J$ = 7.6, 2.4 Hz, 1H), 7.44 (d, $J$ = 8.8 Hz, 2H), 7.39 (d, $J$ = 8.4 Hz, 1H), 7.26 (s, 1H), 7.09 (dd, $J$ = 8.8, 1.2 Hz, 2H), 6.28-6.16 (m, 1H), 5.84 (dd, $J$ = 7.6, 2.8 Hz, 1H), 5.08 (d, $J$ = 8.0 Hz, 2H), 4.35-4.15 (m, 3H), 4.08-4.02 (m, 1H), 3.76-3.62 (m, 2H), 3.50-3.40 (m, 2H), 3.38-3.32 (m, 1H), 1.78-1.49 (m, 6H), 1.18-1.12 (m, 6H). MS: Calculated MS, 722.2, found, 723.2 ($[M+H]^+$).

**Compound H38**

The product (17.6 mg, yield 20.7%) was isolated with preparative high performance liquid chromatography as a white solid.

$^1$H-NMR (400 MHz, DMSO-$d_6$) $\delta$ 8.11 (dd, $J$ = 8.4, 1.2 Hz, 1H), 7.73-7.67 (m, 4H), 7.51 (d, $J$ = 7.2 Hz, 1H), 7.45-7.37 (m, 3H), 7.29 (t, $J$ = 8.8 Hz, 2H), 7.24-7.21 (m, 1H), 7.15 (d, $J$ = 8.0 Hz, 2H), 6.42-6.36 (m, 1H), 6.20-6.11 (m, 1H), 5.72 (dd, $J$ = 7.6, 2.0 Hz, 1H), 5.23-5.14 (m, 1H), 5.00 (d, $J$ = 8.0 Hz, 2H), 4.20-4.08 (m, 3H), 3.97-3.92 (m, 1H), 3.20-3.10 (m, 1H), 1.00-0.93 (m, 6H). MS: Calculated MS, 705.2, found, 706.2 ($[M+H]^+$).

**Compound H39**

The product (17.6 mg, yield 20.7%) was isolated with preparative high performance liquid chromatography as a white solid.

$^1$H-NMR (400 MHz, DMSO-$d_6$) $\delta$ 8.13 (dd, $J$ = 8.4, 0.8 Hz, 1H), 7.91-7.86 (m, 2H), 7.84-7.78 (m, 4H), 7.51 (d, $J$ = 7.6 Hz, 1H), 7.45-7.37 (m, 3H), 7.27 (d, $J$ = 2.0 Hz, 1H), 7.19 (d, $J$ = 8.4 Hz, 2H), 6.42-6.36 (m, 1H), 6.20-6.10 (m, 1H), 5.73 (dd, $J$ = 7.6, 2.0 Hz, 1H), 5.23-5.12 (m, 1H), 5.02 (d, $J$ = 8.0 Hz, 2H), 4.20-4.08 (m, 3H), 3.98-3.92 (m, 1H), 3.20-3.10 (m, 1H), 1.02-0.95 (m, 6H). MS: Calculated MS, 755.2, found, 756.2 ($[M+H]^+$).

**Compound H40**

The product (48 mg, yield 30.2%) was isolated with preparative high performance liquid chromatography as a white solid.

$^1$H-NMR (400 MHz, DMSO-$d_6$) $\delta$ 8.12 (dd, $J$ = 8.4, 1.2 Hz, 1H), 7.77-7.66 (m, 4H), 7.60-7.35 (m, 6H), 7.25-7.22 (m, 1H), 7.16 (d, $J$ = 8.4 Hz, 2H), 6.40 (t, $J$ = 6.8 Hz, 1H), 6.20-6.10 (m, 1H), 5.74 (dd, $J$ = 7.6, 2.0 Hz, 1H), 5.23-5.12 (m, 1H), 5.01 (d, $J$ = 8.0 Hz, 2H), 4.20-4.08 (m, 3H), 3.98-3.92 (m, 1H), 3.20-3.10 (m, 1H), 1.02-0.95 (m, 6H). MS: Calculated MS, 721.2, 723.2, found, 721.9, 723.9 ($[M+H]^+$).

**Compound H41**

The product (6.5 mg, yield 4.2%) was isolated with preparative high performance liquid chromatography as a white solid.

$^1$H-NMR (400 MHz, DMSO-$d_6$) $\delta$ 8.11 (dd, $J$ = 8.4, 0.8 Hz, 1H), 7.71 (dd, $J$ = 8.8, 0.8 Hz, 2H), 7.68-7.63 (m, 2H), 7.58-7.34 (m, 7H), 7.23 (s, 1H), 7.16 (d, $J$ = 8.4 Hz, 2H), 6.43-6.38 (m, 1H), 6.20-6.10 (m, 1H), 5.73 (dd, $J$ = 7.6, 2.4 Hz, 1H), 5.23-5.15 (m, 1H), 5.01 (d, $J$ = 7.6 Hz, 2H), 4.25-4.10 (m, 3H), 3.98-3.92 (m, 1H), 3.20-3.10 (m, 1H), 1.12-0.96 (m, 6H). MS: Calculated MS, 687.2, found, 687.8 ($[M+H]^+$).

**Compound H42**

The product (61 mg, yield 20.4%) was isolated with preparative high performance liquid chromatography as a white solid.

$^1$H-NMR (400 MHz, DMSO-$d_6$) $\delta$ 8.15 (dd, *J* = 8.4, 1.6 Hz, 1H), 7.60-7.50 (m, 6H), 7.49-7.38 (m, 3H), 7.34 (s, 1H), 7.14 (dd, *J* = 11.6, 1.2 Hz, 1H), 6.98 (dd, *J* = 8.8, 2.4 Hz, 1H), 6.40 (t, *J* = 6.8 Hz, 1H), 6.20-6.10 (m, 1H), 5.74 (dd, *J* = 7.6, 2.4 Hz, 1H), 5.24-5.13 (m, 1H), 5.04 (d, *J* = 8.0 Hz, 2H), 4.25-4.10 (m, 3H), 3.98-3.92 (m, 1H), 3.20-3.10 (m, 1H), 1.04-0.95 (m, 6H). MS: Calculated MS, 739.1, 741.1, found, 739.9, 741.9 ($[M+H]^+$).

**Compound H43**

The product (54 mg, yield 23.1%) was isolated with preparative high performance liquid chromatography as a white solid.

$^1$H-NMR (400 MHz, DMSO-$d_6$) $\delta$ 8.12 (dd, *J* = 8.4, 1.2 Hz, 1H), 7.60 (d, *J* = 7.6 Hz, 2H), 7.61-7.37 (m, 6H), 7.36-7.28 (m, 3H), 7.17 (d, *J* = 8.8 Hz, 2H), 6.40 (t, *J* = 6.8 Hz, 1H), 6.20-6.10 (m, 1H), 5.74 (dd, *J* = 7.6, 2.8 Hz, 1H), 5.25-5.15 (m, 1H), 5.03 (d, *J* = 7.6 Hz, 2H), 4.25-4.09 (m, 3H), 3.99-3.95 (m, 1H), 3.20-3.09 (m, 1H), 1.02-0.95 (m, 6H). MS: Calculated MS, 705.2, found, 706.0 ($[M+H]^+$).

**Compound H44**

The product (55 mg, yield 18.6%) was isolated with preparative high performance liquid chromatography as a white solid.

$^1$H-NMR (400 MHz, DMSO-$d_6$) $\delta$ 8.12 (dd, *J* = 8.4, 1.2 Hz, 1H), 7.76 (d, *J* = 8.4 Hz, 2H), 7.55-7.34 (m, 7H), 7.24 (s, 1H), 7.23-7.10 (m, 3H), 6.41 (t, *J* = 6.4 Hz, 1H), 6.20-6.10 (m, 1H), 5.73 (dd, *J* = 7.6, 2.8 Hz, 1H), 5.20-5.10 (m, 1H), 5.02 (d, *J* = 7.6 Hz, 2H), 4.23-4.08 (m, 3H), 3.99-3.95 (m, 1H), 3.20-3.08 (m, 1H), 1.03-0.94 (m, 6H). MS: Calculated MS, 705.2, found, 705.9 ($[M+H]^+$).

**Compound D1**

The product (15 mg, yield 9.6%) was isolated with preparative high performance liquid chromatography as a white solid.

$^1$H-NMR (400 MHz, $CD_3OD$) $\delta$ 8.05 (dd, *J* = 8.4, 6.4 Hz, 1H), 7.61-7.50 (m, 4H), 7.45-7.40 (m, 1H), 7.30 (d, *J* = 2.0 Hz, 1H), 7.18 (t, *J* = 8.8 Hz, 2H), 6.96-6.90 (m, 2H), 6.27-6.16 (m, 1H), 5.88-5.83 (m, 1H), 5.19 (dd, *J* = 8.8, 2.4 Hz, 2H), 4.43-4.30 (m, 2H), 4.28-4.13 (m, 3H), 4.07-4.02 (m, 1H), 1.38-1.27 (m, 3H). MS: Calculated MS, 710.1, found, 710.7 ($[M+H]^+$).

**Compound D2**

The product (4.5 mg, 4.1%) was isolated with neutral preparative high performance liquid chromatography as a white solid.

$^1$H-NMR (400 MHz, $CD_3OD$) $\delta$ 8.06-8.03 (m, 1H), 7.56-7.39 (m, 5H), 7.28 (s, 1H), 7.17 (t, *J* = 8.8 Hz, 2H), 6.95-6.92 (m, 2H), 6.23-6.15 (m, 1H), 5.85 (t, *J* = 7.2Hz, 1H), 5.12-5.05 (m, 2H), 4.31-4.14 (m, 3H), 4.05-4.03 (m, 1H), 3.12-3.06 (m, 4H), 1.11-107 (m, 6H). MS: Calculated 737.2, found 738.0 ($[M+H]^+$).

**Compound D3**

The product (6.6 mg, yield 21.1%) was isolated with neutral preparative high performance liquid chromatography as a white solid.

$^1$H-NMR (300 MHz, $CD_3OD$) $\delta$ 8.05 (d, *J* = 7.8 Hz, 1H), 7.61-7.39 (m, 5H), 7.30 (s, 1H), 7.18 (t, *J* = 8.7 Hz, 2H), 6.95-6.92 (m, 2H), 6.25-6.16 (m, 1H), 5.86-5.82 (m, 1H), 5.10 (d, *J* = 8.7 Hz, 2H), 4.28-4.14 (m, 3H), 4.04 (m, 1H), 2.68 (d, *J* = 10.2 Hz, 6H). MS: Calculated 709.2, found 710.0 ($[M+H]^+$).

**Compound D4**

The product (14.2 mg, 6.9%) was isolated with neutral preparative high performance liquid chromatography as a white solid.

$^1$H-NMR (400 MHz, $CD_3OD$) $\delta$ 8.05 (t, *J* = 8.0 Hz, 1H), 7.60-7.47 (m, 4H), 7.40 (t, *J* = 7.2 Hz, 1H), 7.30 (d, *J* = 4.4 Hz, 1H), 7.18 (t, *J* = 8.8 Hz, 2H), 6.96-6.92 (m, 2H), 6.22-6.20 (m, 1H), 5.87-5.84 (m, 1H), 5.24-5.20 (m, 2H), 4.55-4.33

(m, 2H), 4.24-4.15 (m, 1H), 4.05-4.03 (m, 1H), 2.22 (d, $J$ = 16.0 Hz, 4H). MS: Calculated 707.2, found 707.9 ($[M+H]^+$).

**Compound D5**

The product (53.5 mg, 26.7%) was isolated with neutral preparative high performance liquid chromatography as a white solid.

$^1$H-NMR (400 MHz, $CD_3CD_3OD$) $\delta$ 8.07 (dd, $J$ = 8.4, 3.2 Hz, 1H), 7.70 (d, $J$ = 8.8 Hz, 2H), 7.60 (t, $J$ = 7.2 Hz, 1H), 7.45 (d, $J$ = 8.4 Hz, 1H), 7.29 (s, 1H), 7.17 (d, $J$ = 7.6 Hz, 2H), 6.25-6.18 (m, 1H), 5.87-5.84 (m, 1H), 5.12-5.08 (m, 2H), 4.30-4.23 (m, 3H), 4.06-4.05 (m, 1H), 2.70-2.66 (m, 6H). MS: Calculated 665.1, found 666.0 ($[M+H]^+$).

**Compound D6**

The product (45.0 mg, 19.1%) was isolated with neutral preparative high performance liquid chromatography as a white solid.

$^1$H-NMR (400 MHz, $CD_3OD$) $\delta$ 8.04-8.00 (m, 1H), 7.55-7.16 (m, 13H), 6.92-6.97 (m, 2H), 6.24-6.17 (m, 1H), 5.80-5.76 (m, 1H), 5.32-5.28 (m, 2H), 4.56-4.42 (m, 2H), 4.23-4.15 (m, 1H), 4.07-4.05 (m, 1H). MS: Calculated 758.1, found 759.1 ($[M+H]^+$).

**Compound D7**

The product (92.0 mg, 29.3%) was isolated with neutral preparative high performance liquid chromatography as a white solid.

$^1$H-NMR (400 MHz, $CD_3OD$) $\delta$ 8.04 (dd, $J$ = 8.4, 2.8 Hz, 1H), 7.67 (d, $J$ = 8.8 Hz, 2H), 7.43-7.33 (m, 4H), 7.25-7.17 (m, 4H), 7.12 (d, $J$ = 8.0 Hz, 2H), 6.23-6.16 (m, 1H), 5.78 (t, $J$ = 8.0 Hz, 1H), 5.30 (dd, $J$ = 9.2, 6.4 Hz, 2H), 4.56-4.43 (m, 2H), 4.23-4.16 (m, 1H), 4.07-4.06 (m, 1H). MS: Calculated 714.1, found 715.0 ($[M+H]^+$).

**Compound D9**

The product (4.0 mg, yield 3.6%) was isolated with neutral preparative high performance liquid chromatography as a white solid.

$^1$H NMR (400 MHz, $CD_3OD$) $\delta$ 8.10-8.02 (m, 1H), 7.69-7.32 (m, 5H), 7.30 (s, 1H), 7.18 (t, $J$ = 8.8 Hz, 2H), 6.97-6.90 (m, 2H), 6.28-6.10 (m, 1H), 5.92-5.80 (m, 1H), 5.50-5.43 (m, 1H), 4.30-3.92 (m, 4H), 2.70 (t, $J$ = 10.2 Hz, 3H), 2.56 (d, $J$ = 10.4 Hz, 3H), 1.63-1.59 (m, 3H). MS: Calculated MS, 723.2, found, 724.2 ($[M+H]^+$).

## Claims

1. A gemcitabine derivative of formula (III), or a pharmaceutically acceptable salt, a solvate, an isotopic variant, or an isomer thereof:

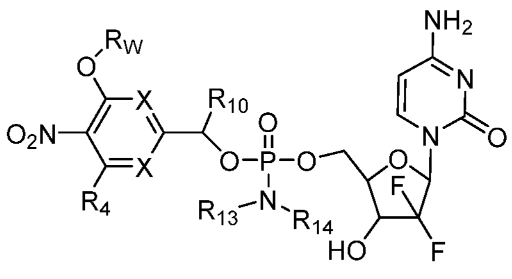

(III)

wherein,

two X's are each independently $CR^{15}$ or N;

$R_{13}$ and $R_{14}$ are each independently hydrogen; $C_1$-$C_6$ alkyl; cycloalkyl; alkenyl; alkynyl; $C_6$-$C_{20}$ aryl; 5-20-membered heterocyclyl; halogen-substituted $C_1$-$C_6$ alkyl, cycloalkyl, alkenyl or alkynyl; halogen-substituted $C_6$-$C_{20}$ aryl; or halogen-substituted 5-20-membered heterocyclyl, and $R_{13}$ and $R_{14}$ are not hydrogen at the same time;

$R_{10}$ is hydrogen; $C_1$-$C_6$ alkyl; cycloalkyl; alkenyl; alkynyl; $C_6$-$C_{20}$ aryl; 5-20-membered heterocyclyl; halogen-

substituted $C_1$-$C_6$ alkyl, cycloalkyl, alkenyl or alkynyl; halogen-substituted $C_6$-$C_{20}$ aryl; or halogen-substituted 5-20-membered heterocyclyl;

or $R_{10}$ and $R_{13}$ or $R_{14}$ may be connected to form a 5-9-membered ring under the condition that they meet the above definitions for $R_{10}$, $R_{13}$ and $R_{14}$;

$R_4$ and $R^{15}$ are each independently hydrogen; halogen; $C_1$-$C_6$ alkyl; cycloalkyl; alkenyl; alkynyl; alkoxy; cyano; 5-20-membered heterocyclyl; $C_6$-$C_{20}$ aryl; or halogen-substituted $C_1$-$C_6$ alkyl, cycloalkyl, alkenyl, alkynyl, alkoxyl, 5-20-membered heterocyclyl or $C_6$-$C_{20}$ aryl;

or $R_{10}$ and $R^{15}$ may form a 4-12-membered cyclic hydrocarbon or heterocycle under the condition that they meet the above definitions for $R_{10}$ and $R^{15}$;

$R_W$ is

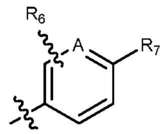

;

A is $CR^{16}$ or N, and the position of A may be changed on the ring;

$R^{16}$ is hydrogen; $C_1$-$C_6$ alkyl; cycloalkyl; alkenyl; alkynyl; $C_6$-$C_{20}$ aryl; 5-20-membered heterocyclyl; halogen-substituted $C_1$-$C_6$ alkyl, cycloalkyl, alkenyl or alkynyl; halogen-substituted $C_6$-$C_{20}$ aryl; or halogen-substituted 5-20-membered heterocyclyl;

$R_6$ and $R_7$ meet the following conditions:

$R_6$ and $R_7$ are each independently hydrogen; halogen; cyano; hydroxyl; $C_1$-$C_6$ alkyl, cycloalkyl, alkenyl, alkynyl, alkoxyl, 5-20-membered heterocyclyl or $C_6$-$C_{20}$ aryl; or halogen-substituted $C_1$-$C_6$ alkyl, cycloalkyl, alkenyl, alkynyl, alkoxyl, 5-20-membered heterocyclyl or $C_6$-$C_{20}$ aryl; or cyano-substituted $C_1$-$C_6$ alkyl, cycloalkyl, alkenyl, alkynyl, alkoxyl, 5-20-membered heterocyclyl or $C_6$-$C_{20}$ aryl; or hydroxyl-substituted $C_1$-$C_6$ alkyl, cycloalkyl, alkoxyl, 5-20-membered heterocyclyl or $C_6$-$C_{20}$ aryl; or $-CONR^{11}R^{12}$; or $-CH_2NR^{11}R^{12}$;

or $R_6$ and $R_7$ are connected to form

a 5-8-membered single or fused heterocycle containing at least one N or S or O, or two or three of N, S and O at the same time;

or a 5-8-membered single or fused heterocycle containing at least one N or S or O, or two or three of N, S and O at the same time, which is $C_1$-$C_6$ alkyl-substituted;

$R_6$ and $CR^{16}$ may be connected to form a 5-9-membered ring, heterocycle, or aromatic heterocycle;

$R^{11}$ and $R^{12}$ meet the following conditions:

$R^{11}$ and $R^{12}$ are each independently $C_1$-$C_6$ alkyl or halogen-substituted $C_1$-$C_6$ alkyl, or $R^{11}$ and $R^{12}$ form a 5-7-membered ring with N from $-CONR^{11}R^{12}$, or form a 5-7-membered ring with N from $-CH_2NR^{11}R^{12}$ under the condition that they meet the above definitions for $R^{11}$ and $R^{12}$.

**2.** The compound according to claim 1, wherein the compound with the structural formula of formula (III) includes compounds of formula (I) and formula (II) below:

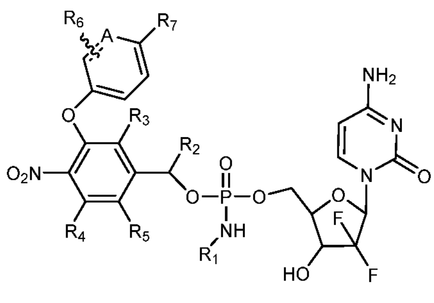

(I)

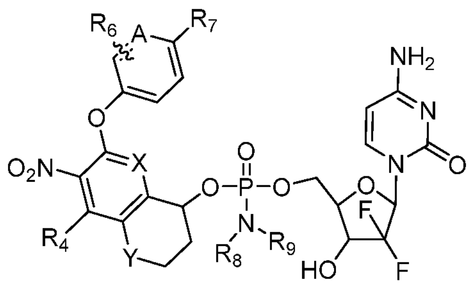

(II)

wherein,

Y is $CR^aR^b$, $NR^a$, or O, and the position of Y may be changed between the 4 positions excluding 2 carbon atoms shared with the benzene ring;

$R^a$ and $R^b$ meet the following conditions:

$R^a$ and $R^b$ are each independently hydrogen; $C_1$-$C_6$ alkyl; cycloalkyl; alkenyl; alkynyl; $C_6$-$C_{20}$ aryl; 5-20-mem-

bered heterocyclyl; halogen-substituted $C_1$-$C_6$ alkyl, cycloalkyl, alkenyl or alkynyl; halogen-substituted $C_6$-$C_{20}$ aryl; or halogen-substituted 5-20-membered heterocyclyl;

$R_1$, $R_2$, $R_8$ and $R_9$ meet the following conditions:

$R_1$ is $C_1$-$C_6$ alkyl; cycloalkyl; alkenyl; alkynyl; $C_6$-$C_{20}$ aryl; 5-20-membered heterocyclyl; halogen-substituted $C_1$-$C_6$ alkyl, cycloalkyl, alkenyl or alkynyl; halogen-substituted $C_6$-$C_{20}$ aryl; or halogen-substituted 5-20-membered heterocyclyl;

$R_2$ is hydrogen; $C_1$-$C_6$ alkyl; cycloalkyl; alkenyl; alkynyl; $C_6$-$C_{20}$ aryl; 5-20-membered heterocyclyl; halogen-substituted $C_1$-$C_6$ alkyl, cycloalkyl, alkenyl or alkynyl; halogen-substituted $C_6$-$C_{20}$ aryl; or halogen-substituted 5-20-membered heterocyclyl;

or $R_1$ and $R_2$ may be connected to form a 5-9-membered ring under the condition that they meet the above definitions for $R_1$ and $R_2$;

$R_8$ and $R_9$ are each independently hydrogen; $C_1$-$C_6$ alkyl; cycloalkyl; alkenyl; alkynyl; $C_6$-$C_{20}$ aryl; 5-20-membered heterocyclyl; halogen-substituted $C_1$-$C_6$ alkyl, cycloalkyl, alkenyl or alkynyl; halogen-substituted $C_6$-$C_{20}$ aryl; or halogen-substituted 5-20-membered heterocyclyl, and $R_8$ and $R_9$ are not hydrogen at the same time;

or $R_8$ and $R_9$ may be connected to form a 5-9-membered ring under the condition that they meet the above definitions for $R_8$ and $R_9$;

$R_3$, $R_4$ and $R_5$ meet the following conditions:

$R_3$, $R_4$ and $R_5$ are each independently hydrogen; halogen; $C_1$-$C_6$ alkyl; cycloalkyl; alkenyl; alkynyl; alkoxy; cyano; 5-20-membered heterocyclyl; $C_6$-$C_{20}$ aryl; or halogen-substituted $C_1$-$C_6$ alkyl, cycloalkyl, alkenyl, alkynyl, alkoxyl, 5-20-membered heterocyclyl or $C_6$-$C_{20}$ aryl.

**3.** The compound according to claim 2, wherein,
$R_1$, $R_8$ and $R_9$ are each independently $C_1$-$C_5$ alkyl, halogen-substituted $C_1$-$C_5$ alkyl, $C_3$-$C_6$ cycloalkyl or $C_6$-$C_{20}$ aryl.

**4.** The compound according to claim 2, wherein,

$R_1$, $R_8$ and $R_9$ are each independently methyl, ethyl, n-propyl, isopropyl, isobutyl, t-butyl, neopentyl, bromoethyl, 1,1,1-trifluoroethyl, $C_3$-$C_6$ cycloalkyl or benzyl;

$R_2$ is hydrogen, methyl, or trifluoromethyl.

**5.** The compound according to claim 2, wherein, $R_1$ and $R_2$ in formula (I) are connected to form a 6-membered ring.

**6.** The compound according to claim 2, wherein, $R_3$, $R_4$ and $R_5$ are each independently hydrogen.

**7.** The compound according to claim 2, wherein,
$R_6$ is hydrogen, fluorine, fluorophenyl, or

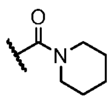 or 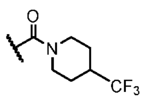.

**8.** The compound according to claim 2, wherein,
$R_7$ is hydrogen, $-CF_3$, phenyl, chlorophenyl, fluorophenyl, $-CF_3$-substituted phenyl, fluoropyridyl, or

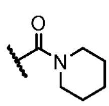, 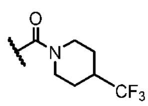 or 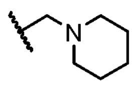.

**9.** The compound according to claim 2, wherein,

$R_6$ and $R_7$ are connected to form a 5-8-membered heterocycle containing 2 N atoms, or $C_1$-$C_6$ alkyl-substituted 5-8-membered heterocycle, and preferably, $R_6$ and $R_7$ are connected to form a pyrimidine; or

$R_6$ and $R_7$ are connected to form a 5-8-membered heterocycle containing 1 N atom, or $C_1$-$C_6$ alkyl-substituted 5-8-membered heterocycle, and preferably, $R_6$ and $R_7$ are connected to form a pyridine.

**10.** The compound according to claim 2, wherein the compound with the structural formula of formula (I) includes compounds with the structures of formulas I-1 to I-5 below, and the compound with the structural formula of formula (II) includes compounds with the structures of formulas II-1 to II-6 below:

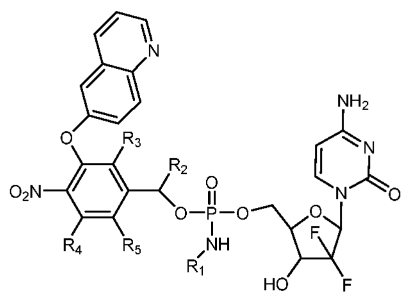
(I-1),

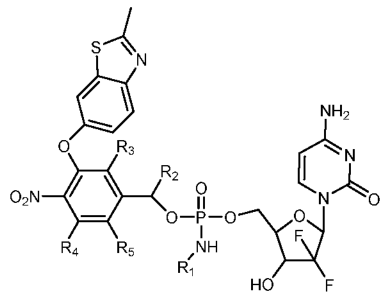
(I-2),

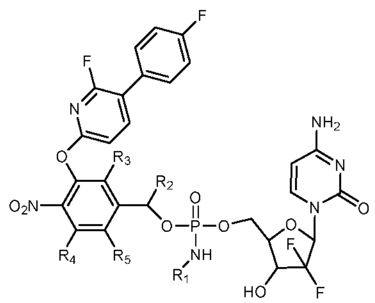
(I-3),

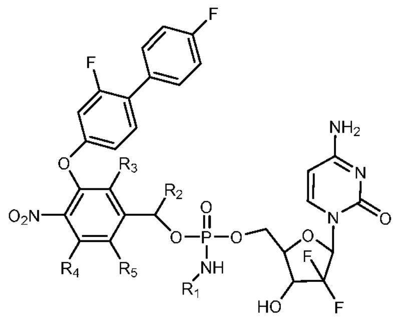
(I-4),

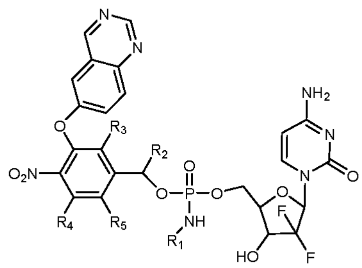
(I-5),

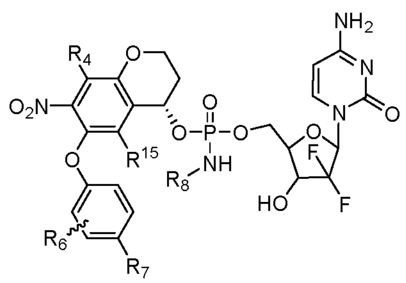
(II-1),

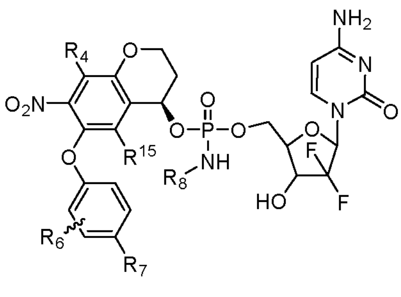
(II-2),

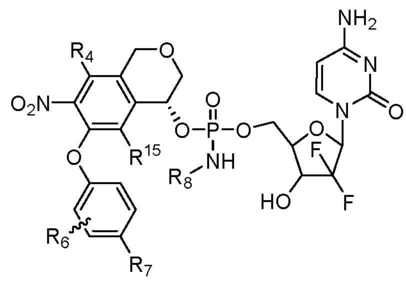
(II-3),

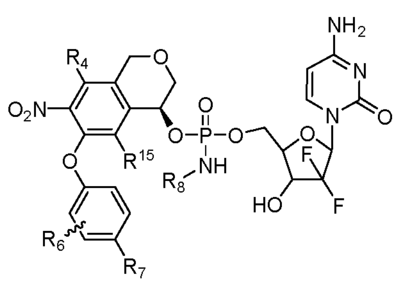

(II-4),

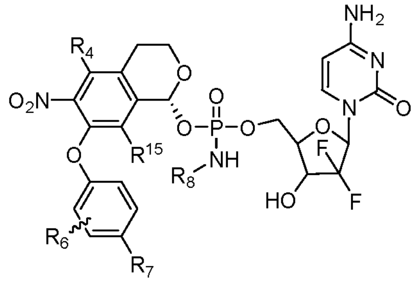

(II-5)

and

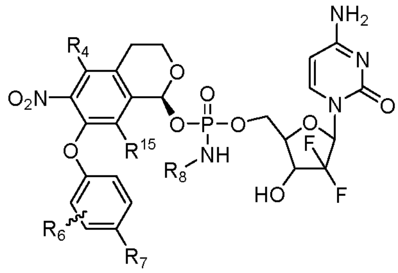

(II-6).

**11.** The compound according to claim 2, wherein the structure of formula (I) substituted by isotope deuterium includes compounds with the structures of formulas I-6 to I-13 below, and the structure of formula (II) substituted by isotope deuterium includes compounds with the structures of formulas II-7 to II-12 below:

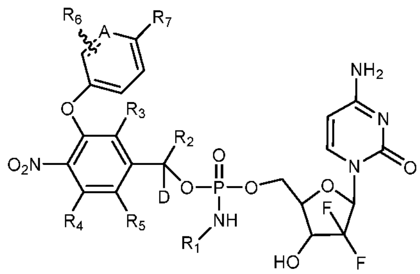

(I-6),

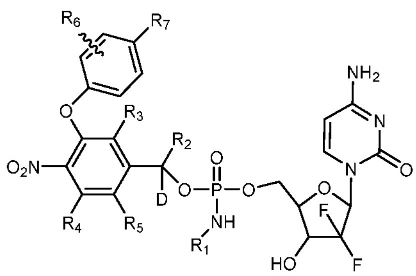

(I-7),

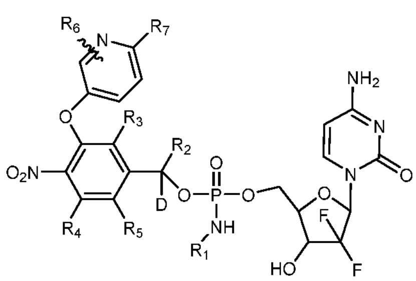

(I-8),

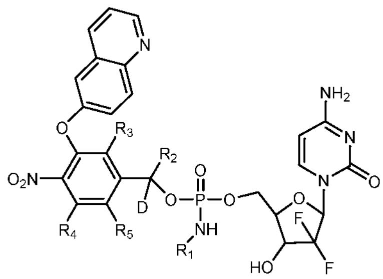

(I-9),

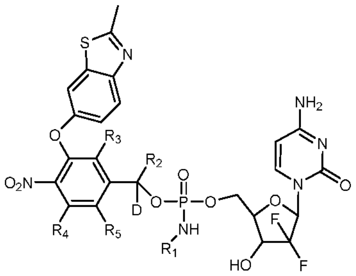 (I-10), 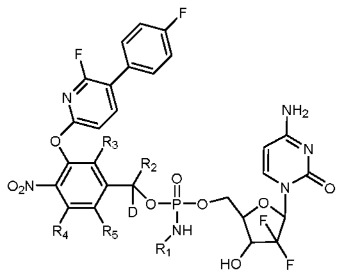 (I-11),

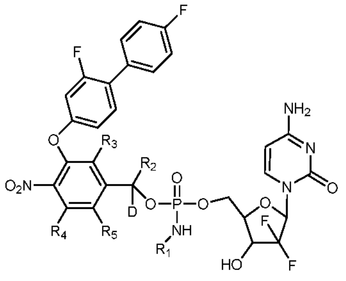 (I-12), 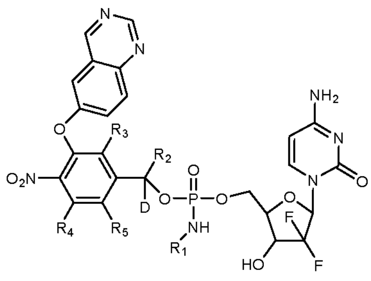 (I-13),

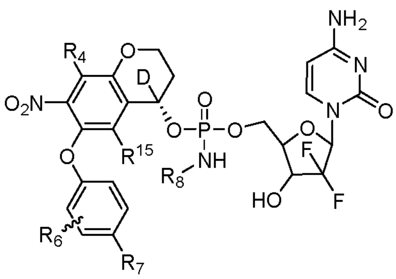 (II-7), 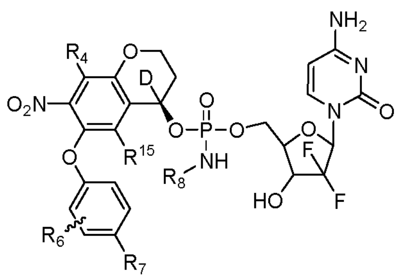 (II-8),

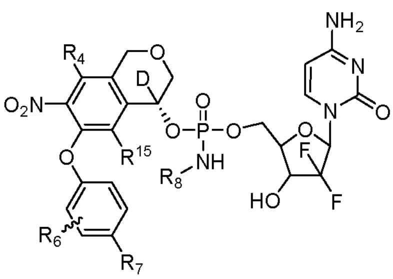 (II-9), 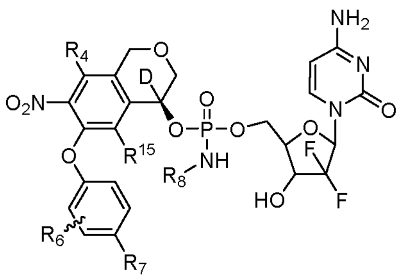 (II-10),

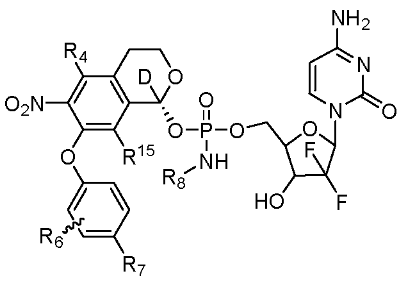 (II-11) and 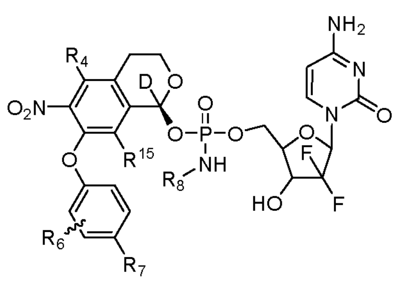 (II-12).

**12.** The compound according to claim 2 or 10 or 11, wherein, hydrogen atom(s) in $R_1$ are substituted by at least one deuterium.

**13.** The compound according to claim 2, wherein,

formula (I) is selected from compounds of the following structures:

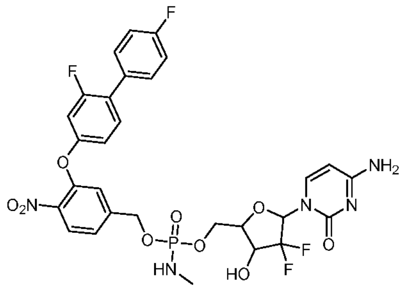 , 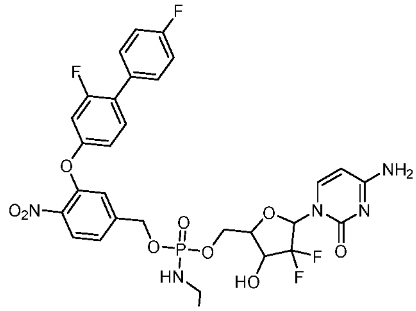 , 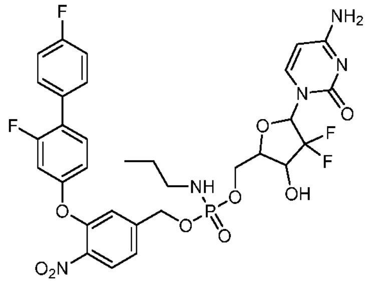 ,

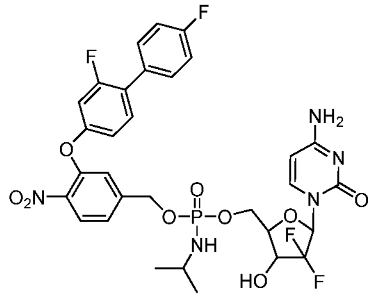 , 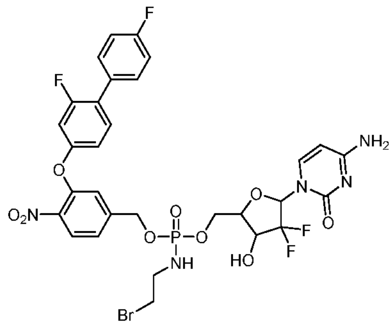 , 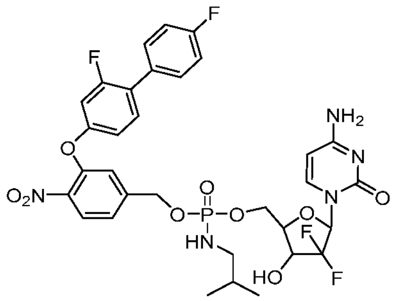 ,

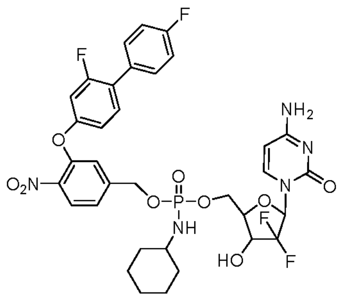 , 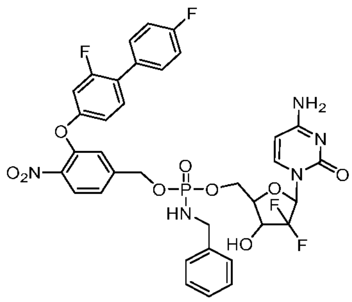 , 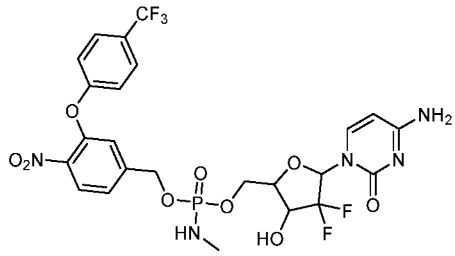 ,

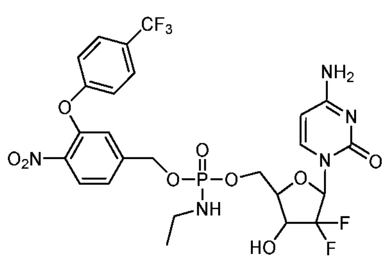 , 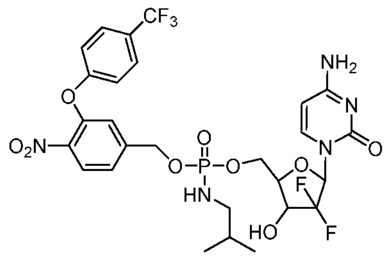 , 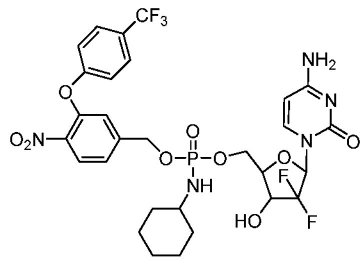 ,

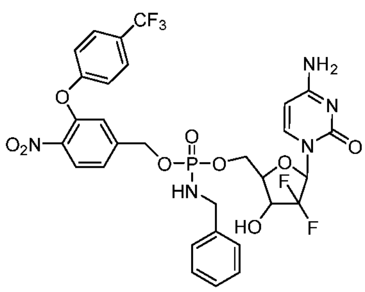,
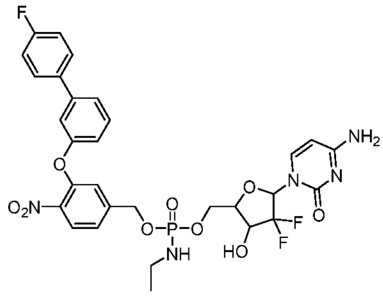,
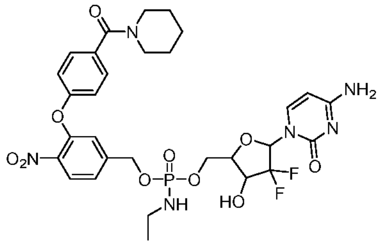,

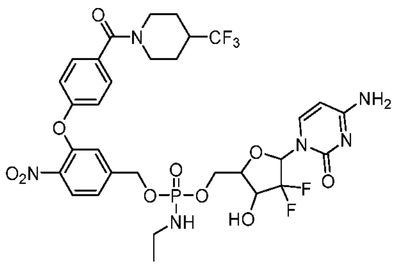,
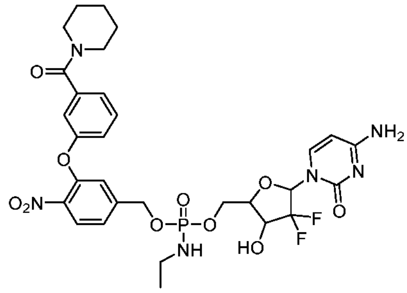,
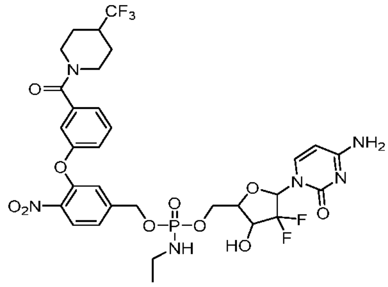,

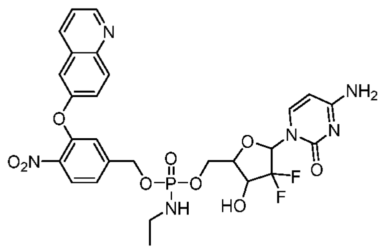,
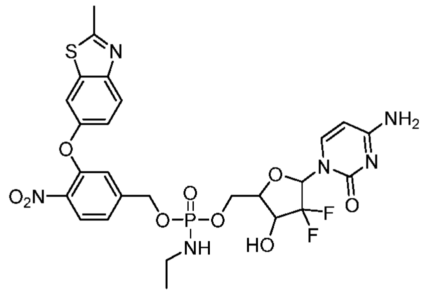,
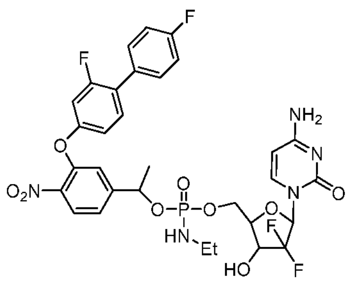,

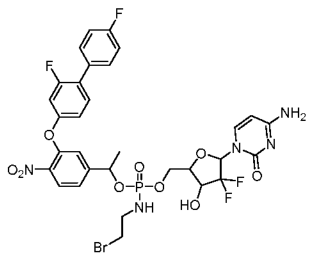,
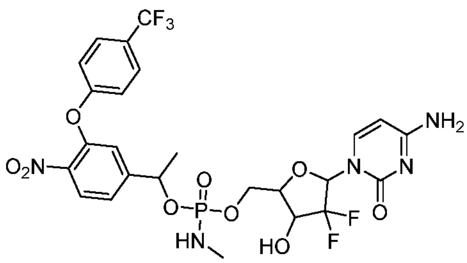,
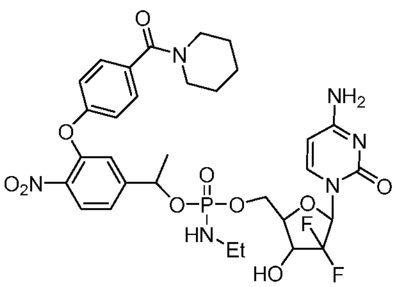,

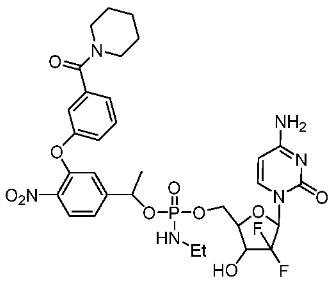,
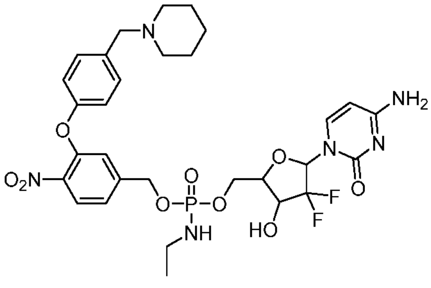,
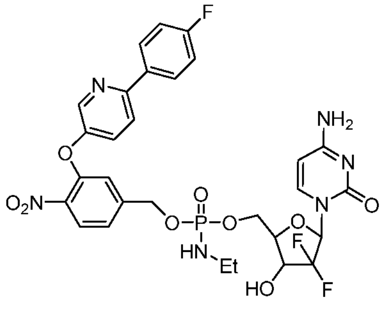,

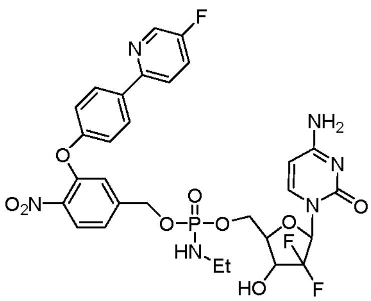 , 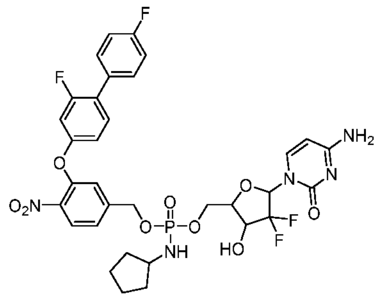 , 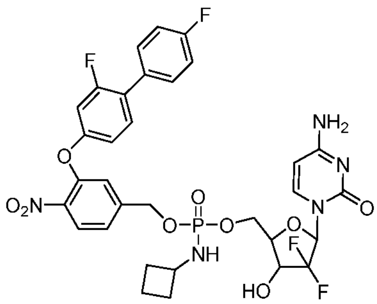 ,

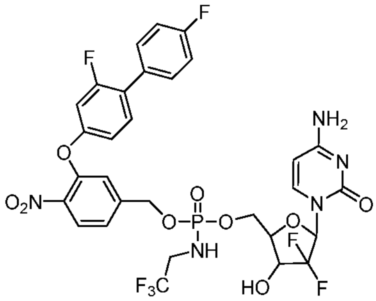 , 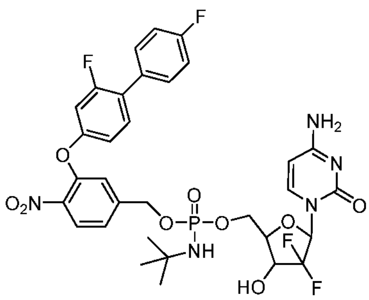 , 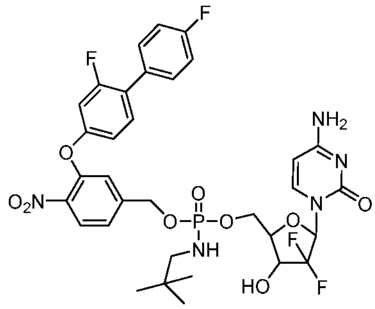 ,

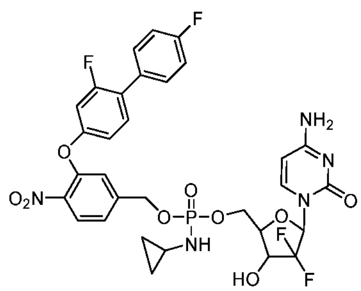 , 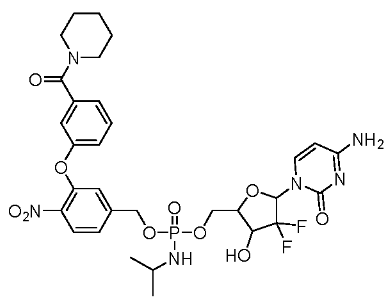 , 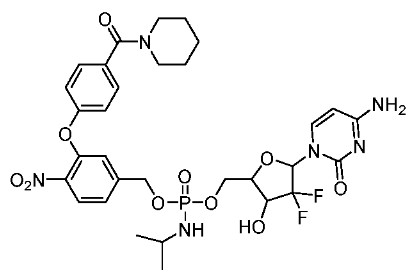 ,

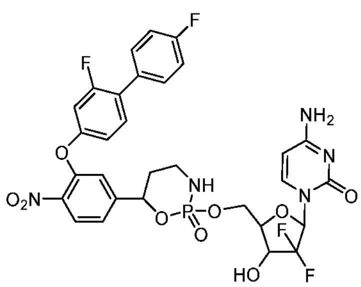 , 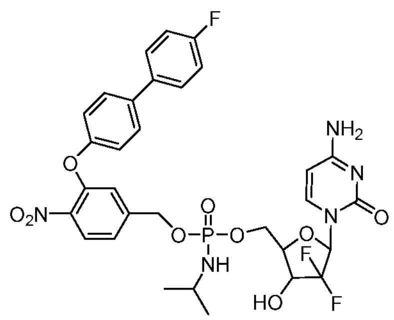 , 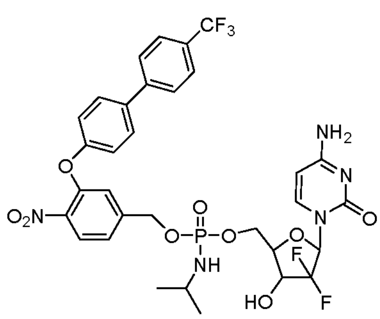 ,

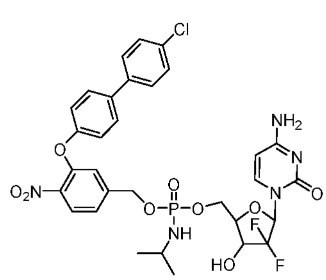 , 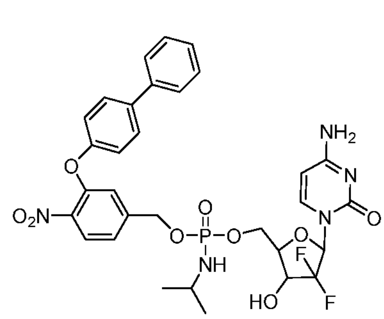 , 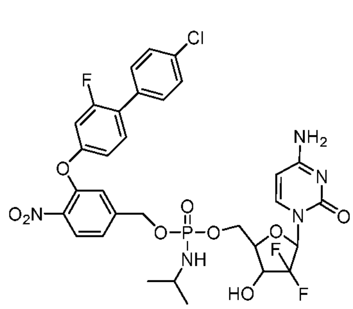 ,

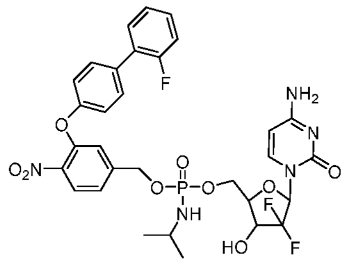 and 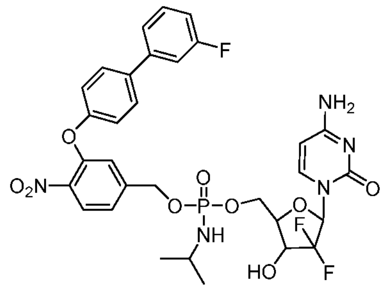 ;

formula (II) is selected from compounds of the following structures:

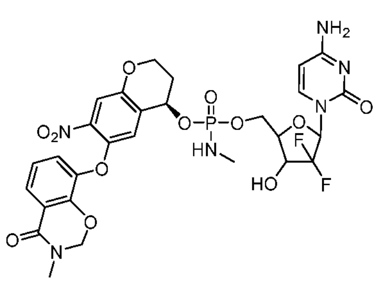 , 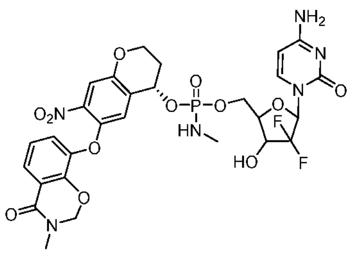 , 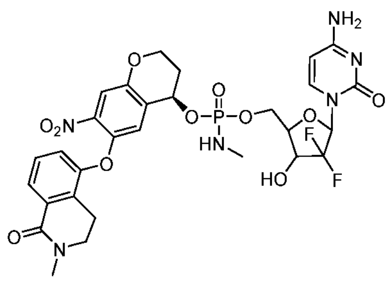 ,

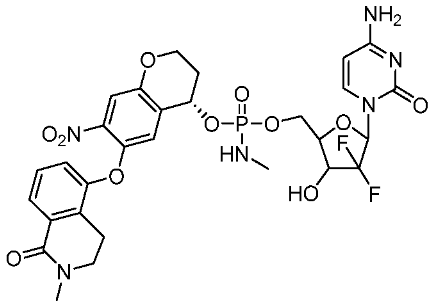 , 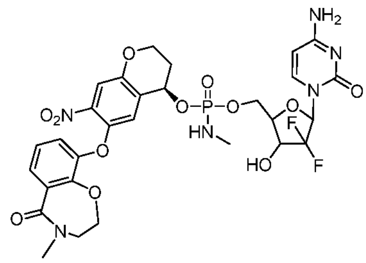 ,

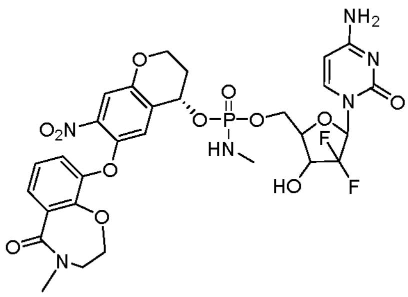 , 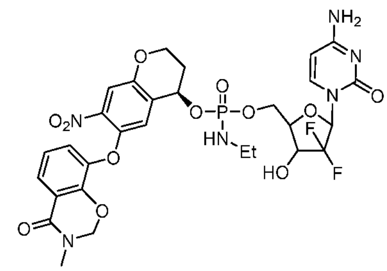 , 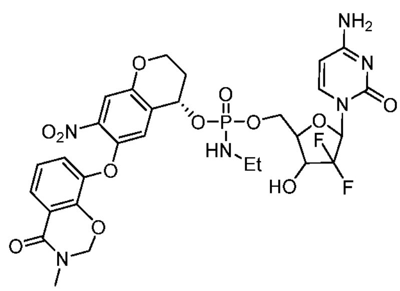 ,

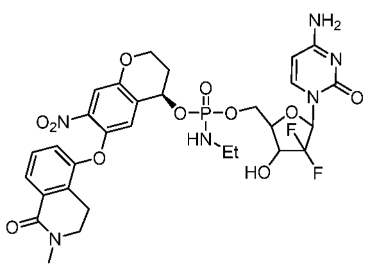 , 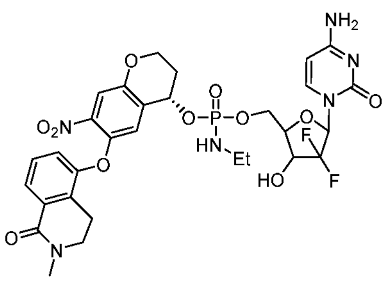 , 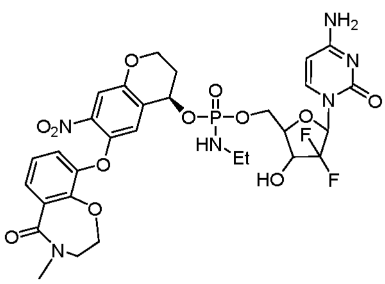 ,

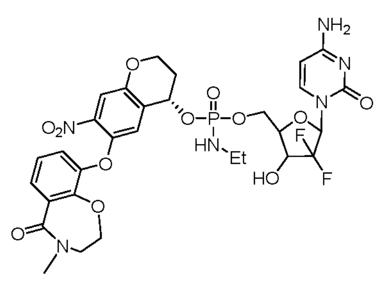
,
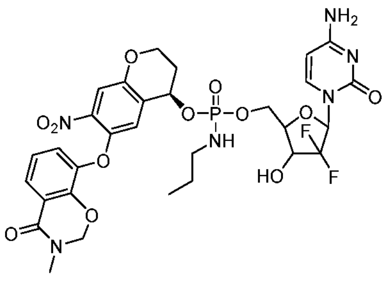
,
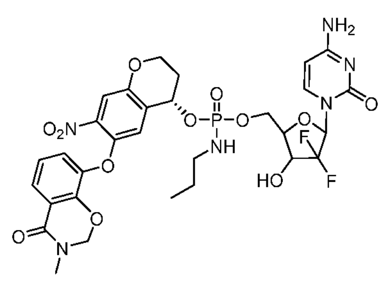
,

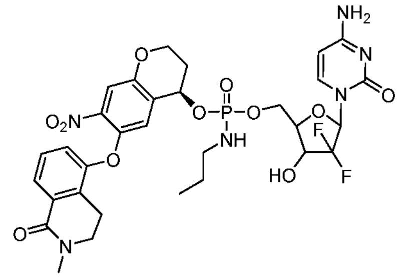
,
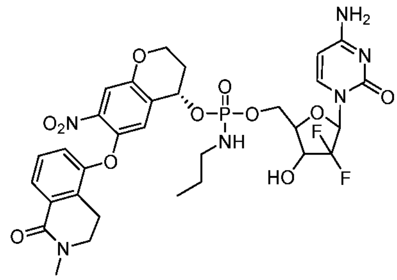
,
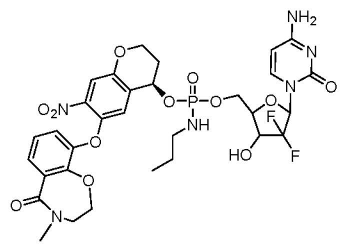
,

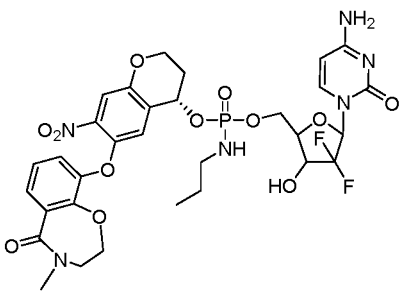
,
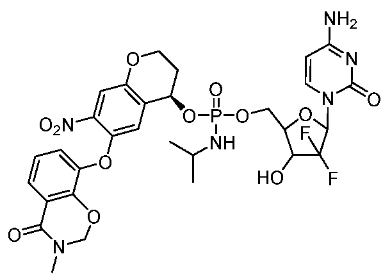
,
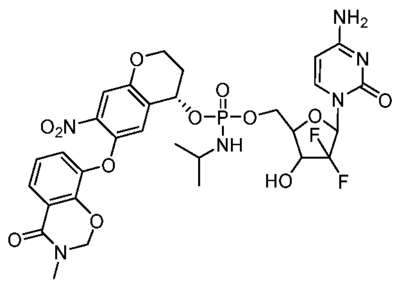
,

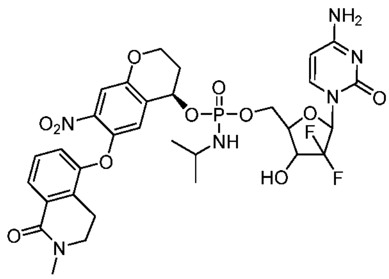
,
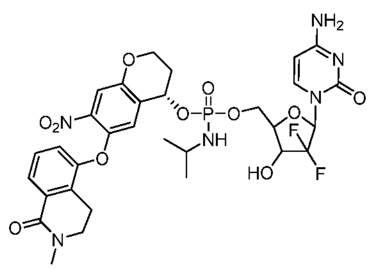
,
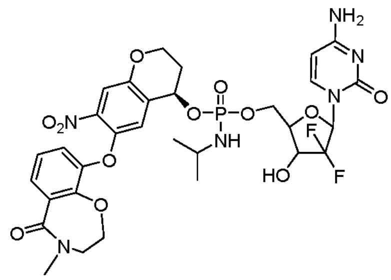
,

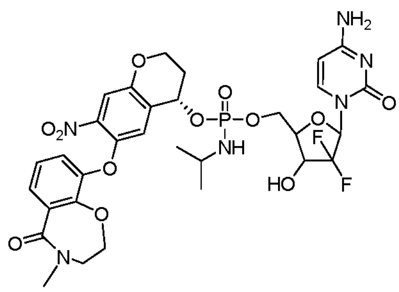
,
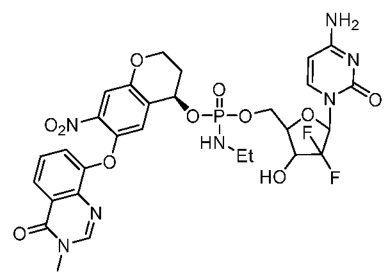
,
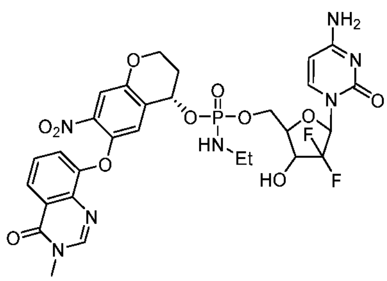
,

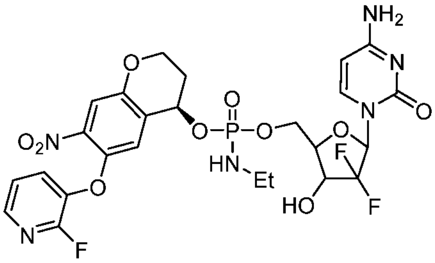,
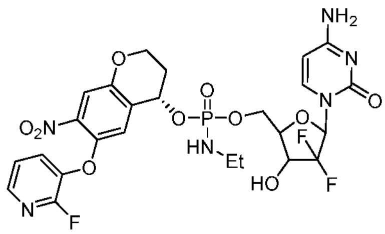,

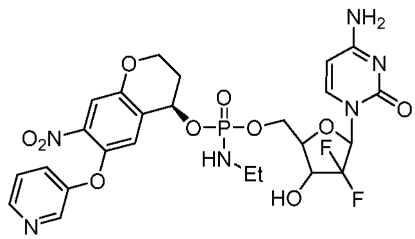,
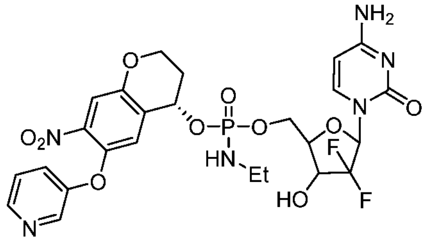,

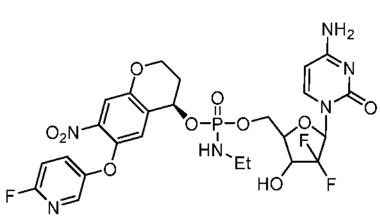,
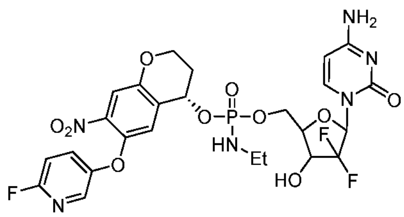,
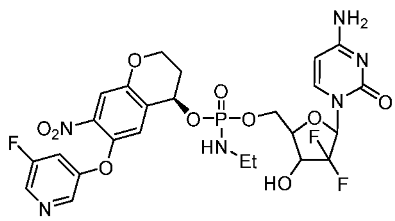,

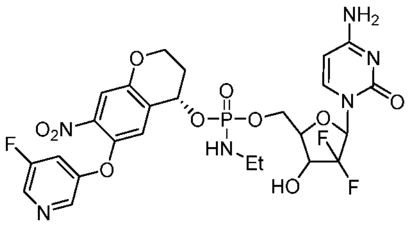,
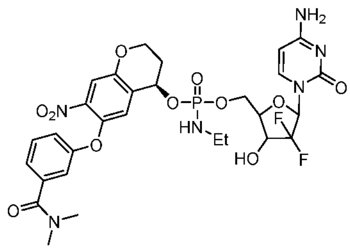,
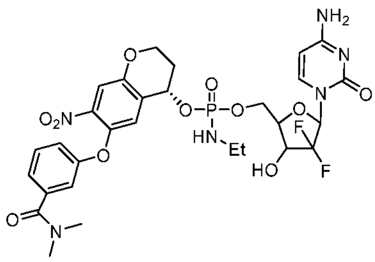,

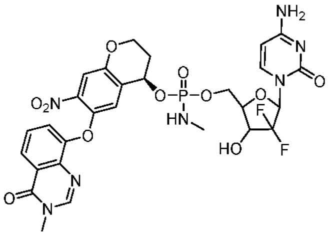,
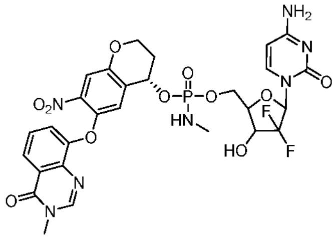,
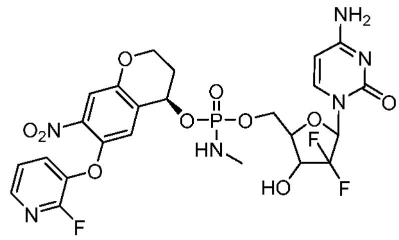,

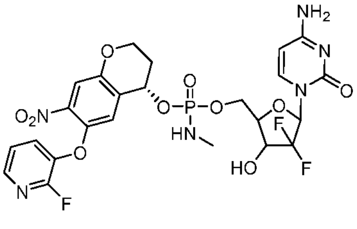,
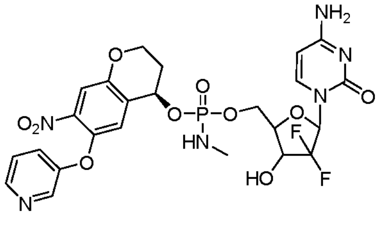,
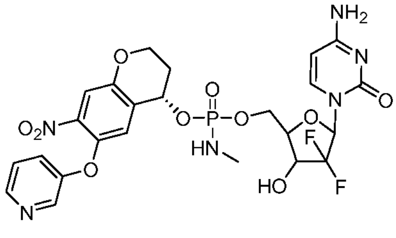,

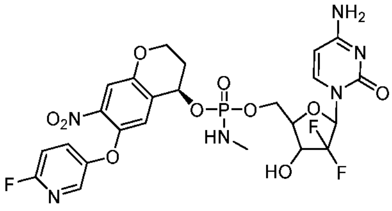,
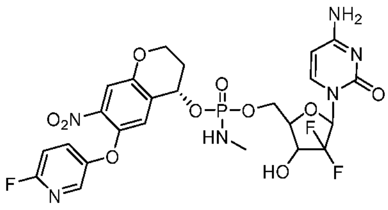,
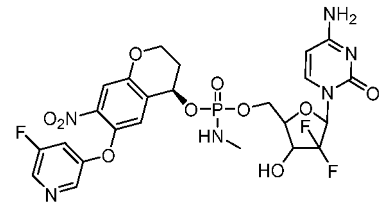,

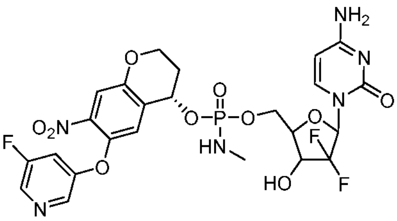,
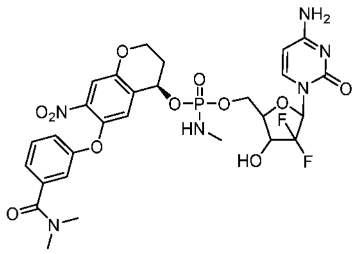,
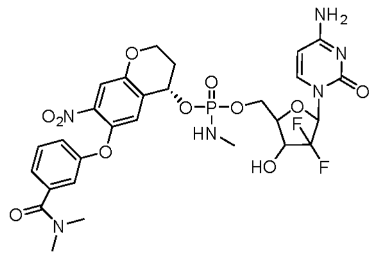,

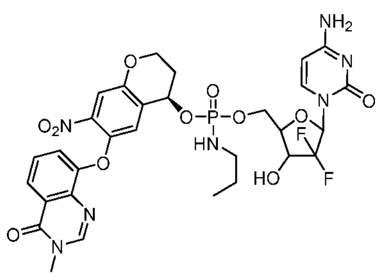,
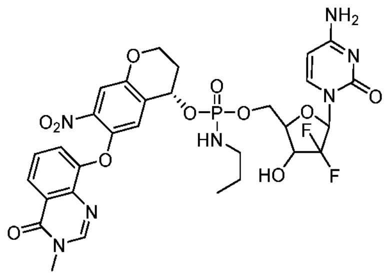,
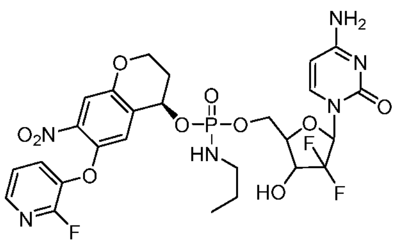,

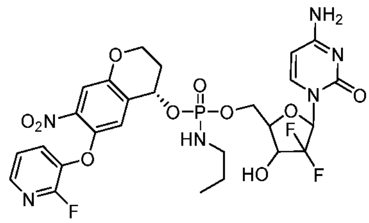,
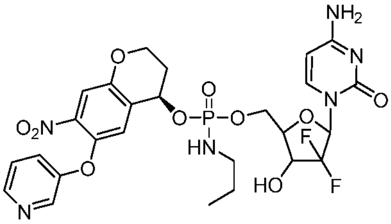,
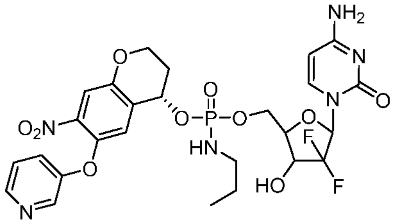,

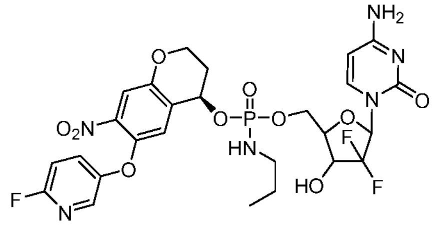,
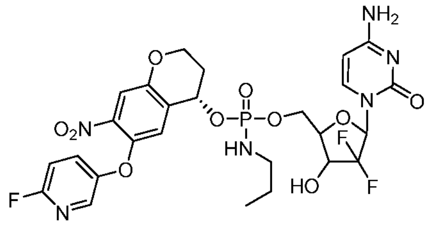,

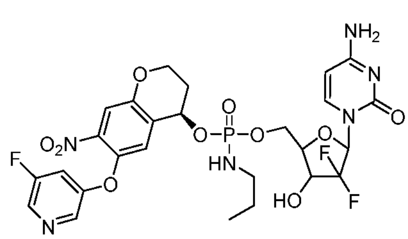,
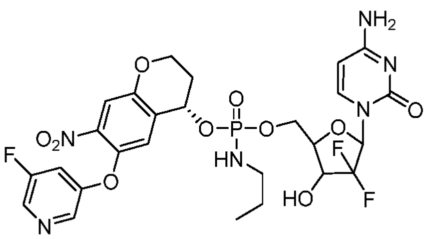,

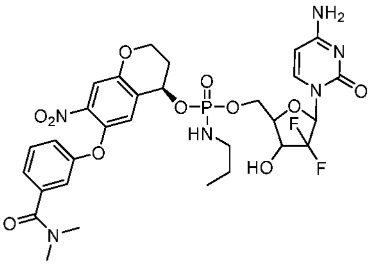,
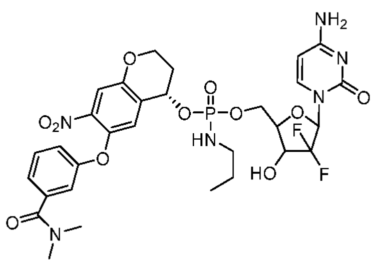,
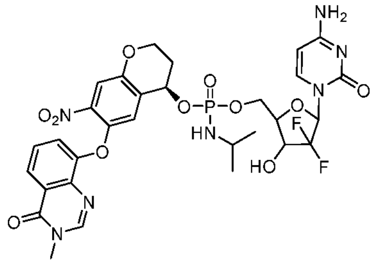,
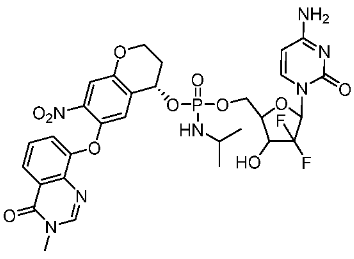,
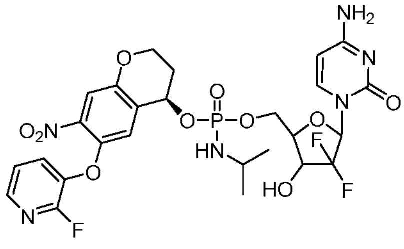,
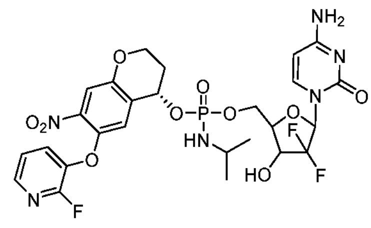,
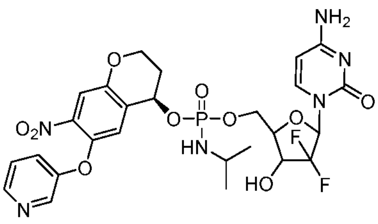,
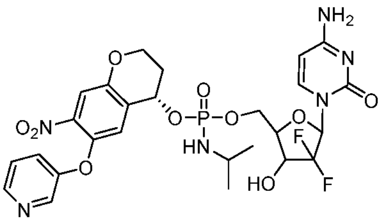,
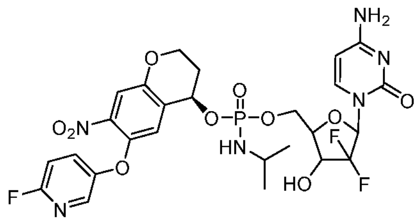,
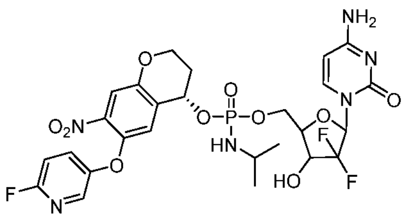,
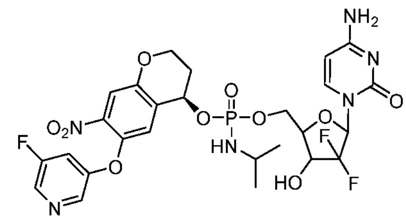,
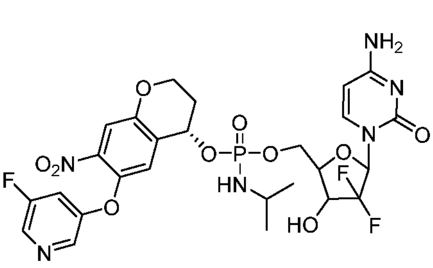,
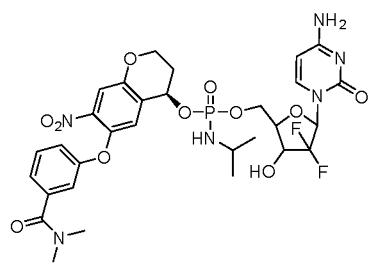

and

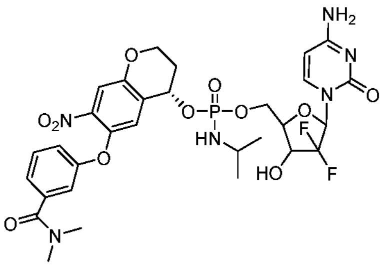

.

**14.** The compound according to any one of claims 1 to 13, wherein, the salt is a basic salt or an acid salt, and the solvate is a hydrate or alcoholate.

**15.** Use of the compound according to any one of claims 1 to 14 in the manufacture of a medicament for the treatment of tumors and cancers.

**16.** A medicament or formulation containing the compound according to any one of claims 1 to 14.

**17.** The medicament or formulation according to claim 16, wherein the medicament or formulation is used for the treatment of tumor and cancer diseases in patients, wherein the tumors and cancers include:
lung cancer, non-small cell lung cancer, liver cancer, pancreatic cancer, stomach cancer, bone cancer, esophagus cancer, breast cancer, prostate cancer, testicular cancer, colon cancer, ovarian cancer, bladder cancer, cervical cancer, melanoma, squamous cell carcinoma, basal cell carcinoma, adenocarcinoma, sweat gland carcinoma, sebaceous gland carcinoma, papillary carcinoma, papillary adenocarcinoma, cystic adenocarcinoma, cystic carcinoma, medullary carcinoma, bronchial carcinoma, osteocyte carcinoma, epithelial carcinoma, cholangiocarcinoma, choriocarcinoma, embryonal carcinoma, seminoma, Wilm's tumor, glioblastoma, astrocytoma, medulloblastoma, craniopharyngioma, ependymoma, pineal tumor, hemocytoblastoma, vocal cord neuroma, meningioma, neuroblastoma, optic neuroblastoma, retinoblastoma, neurofibroma, fibrosarcoma, fibroblastoma, fibroma, fibroadenoma, fibrochondroma, fibrocystoma, fibromyxoma, fibroosteoma, fibromyxosarcoma, fibropapilloma, myxosarcoma, myxocystoma, myxochondroma, myxochondrosarcoma, myxochondrofibrosarcoma, myxadenoma, myxoblastoma, liposarcoma, lipoma, lipoadenoma, lipoblastoma, lipochondroma, lipofibroma, lipoangioma, myxolipoma, chondrosarcoma, chondroma, chondromyoma, chordoma, chorioadenoma, chorioepithelioma, chorioblastoma, osteosarcoma, osteoblastoma, osteochondrofibroma, osteochondrosarcoma, osteochondroma, osteocystoma, osteodentinoma, osteofibroma, fibrosarcoma of bone, angiosarcoma, hemangioma, angiolipoma, angiochondroma, hemangioblastoma, angiokeratoma, angioglioma, angioendothelioma, angiofibroma, angiomyoma, angiolipoma, angiolymphangioma, angiolipoleiomyoma, angiomyolipoma, angiomyoneuroma, angiomyxoma, angioreticuloma, lymphangiosarcoma, lymphogranuloma, lymphangioma, lymphoma, lymphomyxoma, lymphosarcoma, lymphangiofibroma, lymphocytoma, lymphoepithelioma, lymphoblastoma, endothelioma, endothelioblastoma, synovioma, synovial sarcoma, mesothelioma, connective tissue tumor, Ewing's tumor, leiomyoma, leiomyosarcoma, leiomyoblastoma, leiomyofibroma, rhabdomyoma, rhabdomyosarcoma, rhabdomyomyxoma, acute lymphatic leukemia, acute myelogenous leukemia, anemia of chronic disease, polycythemia, lymphoma, endometrial cancer, glioma, colorectal cancer, thyroid cancer, urothelial cancer or multiple myeloma.

**18.** A method for treating cancer or tumor, comprising a step of applying the medicament or formulation according to claim 16; and a step of determining an AKR1C3 reductase content or expression level of cancer cells or tissues in a patient, and
administering the medicament or formulation according to claim 16 to the patient if the measured AKR1C3 reductase content or expression level is equal to or greater than a predetermined value.

**19.** A method for treating cancer or tumor, comprising a step of applying the medicament or formulation according to claim 16; and a step of adjusting an AKR1C3 reductase content or expression level, and
administering the medicament or formulation according to claim 16 to the patient when the AKR1C3 reductase content or expression level is adjusted to be equal to or greater than a predetermined value.

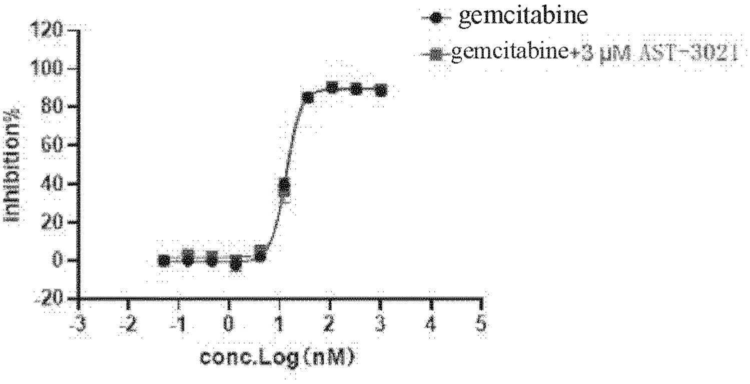


Figure 1

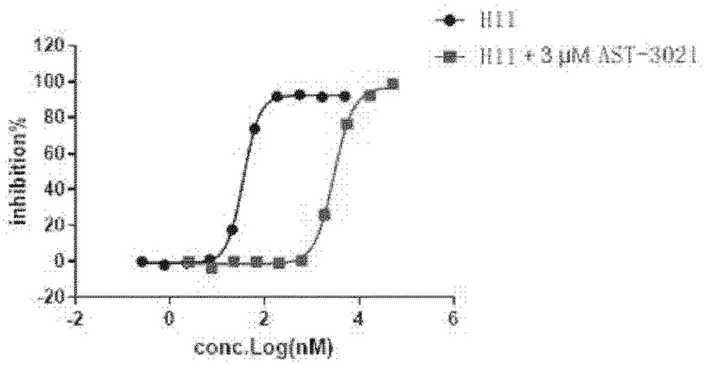


Figure 2

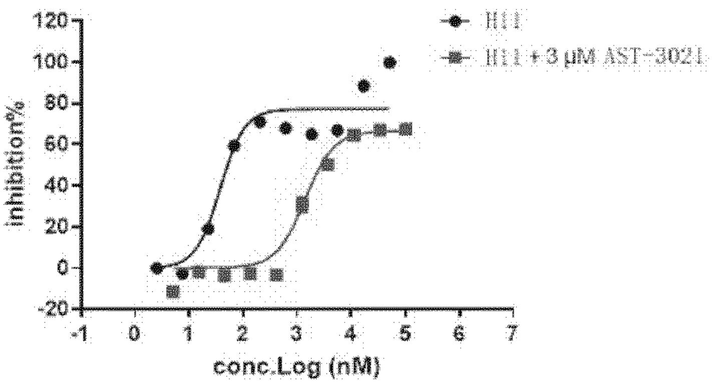


Figure 3

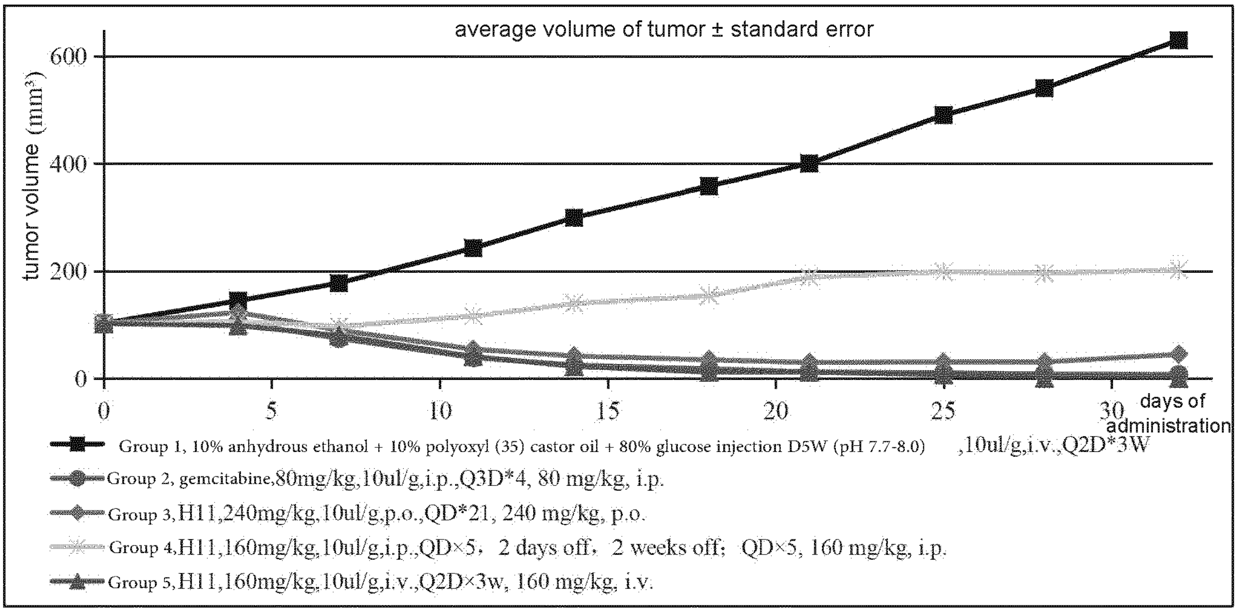


Figure 4

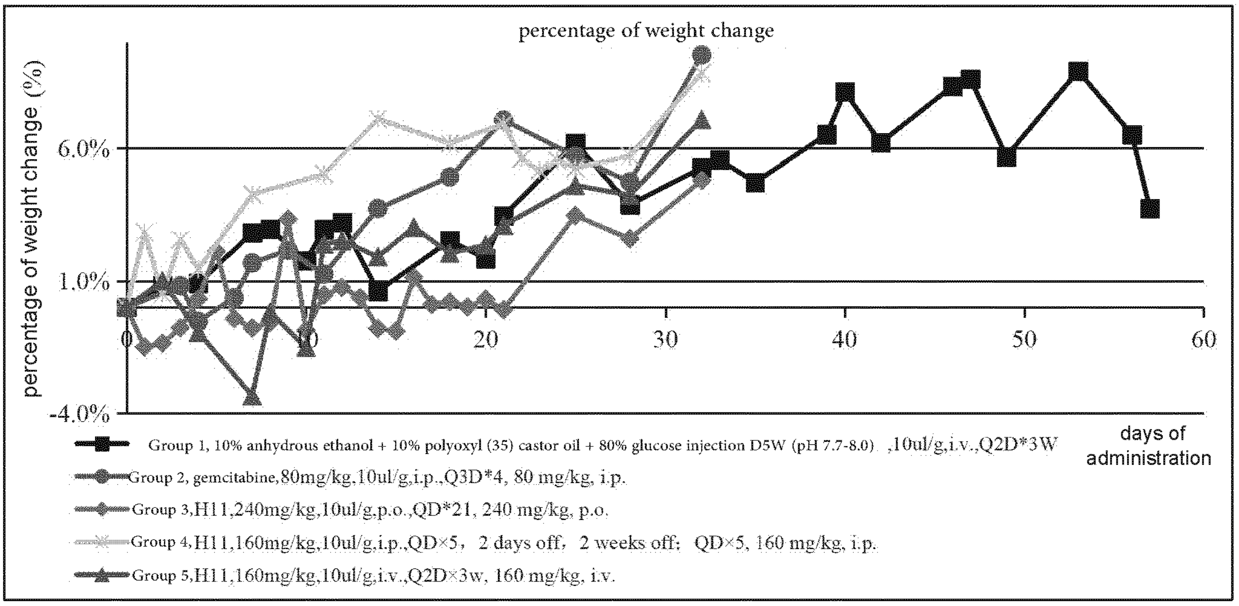


Figure 5

## INTERNATIONAL SEARCH REPORT

International application No.
**PCT/CN2022/098082**

**A. CLASSIFICATION OF SUBJECT MATTER**

C07H 19/073(2006.01)i; C07H 1/00(2006.01)i; A61K 31/7064(2006.01)i; A61P 35/00(2006.01)i

According to International Patent Classification (IPC) or to both national classification and IPC

**B. FIELDS SEARCHED**

Minimum documentation searched (classification system followed by classification symbols)

C07H; A61K; A61P

Documentation searched other than minimum documentation to the extent that such documents are included in the fields searched

Electronic data base consulted during the international search (name of data base and, where practicable, search terms used)

CNTXT; VEN; USTXT; EPTXT; WOTXT; STN-REGISTRY; STN-CAPLUS; CNKI: 深圳艾欣达伟医药科技有限公司, 李安蓉, 齐天阳, 段建新, 孟繁英, 张梦云, 孟滕, 吉西他滨, 癌, 瘤, gemcitabine, cancer, tumor, 式III结构式

**C. DOCUMENTS CONSIDERED TO BE RELEVANT**

| Category* | Citation of document, with indication, where appropriate, of the relevant passages | Relevant to claim No. |
|---|---|---|
| X | CN 108136214 A (THRESHOLD PHARMACEUTICALS, INC.) 08 June 2018 (2018-06-08) claims 1-20, and description, and paragraphs 41 and 213-220 | 1, 14-19 |
| A | CN 108136214 A (THRESHOLD PHARMACEUTICALS, INC.) 08 June 2018 (2018-06-08) claims 1-20, and description, and paragraphs 41 and 213-220 | 2-13 |
| X | WO 2021110085 A1 (ASCENTAWITS PHARMACEUTICALS, LTD.) 10 June 2021 (2021-06-10) claims 1-20 | 1, 14-19 |
| A | WO 2021110085 A1 (ASCENTAWITS PHARMACEUTICALS, LTD.) 10 June 2021 (2021-06-10) claims 1-20 | 2-13 |
| A | CN 107286190 A (LIU, Pei) 24 October 2017 (2017-10-24) entire document | 1-19 |
| A | CN 108290911 A (THRESHOLD PHARMACEUTICALS, INC.) 17 July 2018 (2018-07-17) entire document | 1-19 |

☐ Further documents are listed in the continuation of Box C.    ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **18 August 2022** | **30 August 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/098082**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **18, 19**
because they relate to subject matter not required to be searched by this Authority, namely:

[1] Claims 18 and 19 set forth a method for treating cancers or tumors, comprising administering the drug or preparation according to claim 16 to a patient, which comprises the step of administering a drug to a living human or animal body to treat cancers or tumors. The subject matter belongs to a method for the treatment of a human or animal body, and belongs to the cases listed in PCT Rule 39.1(iv) for which no international search is required.

[2] The search and written opinion for claims 18 and 19 were formed on the basis of the following reasonably expected amendment, i.e., amending the subject matter of claims 18 and 19 to be "an application of the drug or preparation according to claim 16 in the preparation of a drug for treating cancers or tumors".

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2022/098082**

| Patent document cited in search report | Publication date (day/month/year) | Patent family member(s) | Publication date (day/month/year) |
|---|---|---|---|
| CN 108136214 A | 08 June 2018 | JP 2019178172 A | 17 October 2019 |
| | | US 2021017120 A1 | 21 January 2021 |
| | | KR 20170127463 A | 21 November 2017 |
| | | WO 2016161342 A2 | 06 October 2016 |
| | | HK 1250959 A1 | 18 January 2019 |
| | | JP 2018511612 A | 26 April 2018 |
| | | US 2018086693 A1 | 29 March 2018 |
| | | CA 2981494 A1 | 06 October 2016 |
| | | EP 3277381 A2 | 07 February 2018 |
| | | TW 201706267 A | 16 February 2017 |
| | | AU 2016244000 A1 | 12 October 2017 |
| | | BR 112017021167 A2 | 03 July 2018 |
| | | KR 20190075145 A | 28 June 2019 |
| | | WO 2016161342 A3 | 10 November 2016 |
| | | SG 11201707375 A1 | 30 October 2017 |
| | | IN 201717031567 A | 01 December 2017 |
| | | EP 3277381 A4 | 05 December 2018 |
| | | KR 102001910 B1 | 19 July 2019 |
| | | JP 6612892 B2 | 27 November 2019 |
| | | IL 254769 A | 31 May 2020 |
| | | AU 2016244000 B2 | 02 July 2020 |
| | | CN 108136214 B | 28 August 2020 |
| | | US 10829437 B2 | 10 November 2020 |
| | | CN 112142692 A | 29 December 2020 |
| | | IN 363180 B | 02 April 2021 |
| | | TW 730957 B1 | 21 June 2021 |
| | | SG 11201707375 B | 25 June 2021 |
| | | KR 102276681 B1 | 13 July 2021 |
| | | JP 6947781 B2 | 13 October 2021 |
| | | SG 10201913709 A1 | 30 March 2020 |
| | | IN 202018004395 A | 13 March 2020 |
| WO 2021110085 A1 | 10 June 2021 | TW 202128187 A | 01 August 2021 |
| | | CN 112904026 A | 04 June 2021 |
| | | CN 114746753 A | 12 July 2022 |
| | | CA 3163127 A1 | 10 June 2021 |
| | | AU 2020396113 A1 | 14 July 2022 |
| CN 107286190 A | 24 October 2017 | None | |
| CN 108290911 A | 17 July 2018 | JP 2018517710 A | 05 July 2018 |
| | | TW 201726695 A | 01 August 2017 |
| | | ES 2781398 T3 | 01 September 2020 |
| | | EP 3390415 A1 | 24 October 2018 |
| | | CA 2990696 A1 | 26 May 2017 |
| | | KR 20170130615 A | 28 November 2017 |
| | | AU 2016357728 A1 | 30 November 2017 |
| | | WO 2017087428 A1 | 26 May 2017 |
| | | BR 112017025778 A2 | 14 August 2018 |
| | | HK 1250988 A1 | 18 January 2019 |
| | | SG 11201709123 A1 | 28 December 2017 |
| | | IN 201817016988 A | 10 August 2018 |
| | | US 2018258116 A1 | 13 September 2018 |

Form PCT/ISA/210 (patent family annex) (January 2015)

**INTERNATIONAL SEARCH REPORT**
**Information on patent family members**

International application No.
**PCT/CN2022/098082**

| Patent document cited in search report | Publication date (day/month/year) | Patent family member(s) | | | Publication date (day/month/year) |
|---|---|---|---|---|---|
| | | EP | 3390415 | A4 | 22 May 2019 |
| | | KR | 101985778 | B1 | 04 June 2019 |
| | | US | 10409869 | B2 | 10 September 2019 |
| | | EP | 3390415 | B1 | 26 February 2020 |
| | | CN | 108290911 | B | 08 May 2020 |
| | | JP | 6695360 | B2 | 20 May 2020 |
| | | TW | 702222 | B1 | 21 August 2020 |
| | | AU | 2016357728 | B2 | 27 August 2020 |
| | | IL | 256569 | A | 31 December 2020 |
| | | BR | 112017025778 | B1 | 16 March 2021 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2016025665 W
- WO 2016161342 A1
- CN 201680020013
- CN 108136214 A
- JP 2021145582 A
- US 2016062114 W
- WO 2017087428 A
- CN 2016800446081
- CN 108290911 A

### Non-patent literature cited in the description

- **JIAN-XIN DUAN et al.** *NITROBENZYL DERIVATIVES OF ANTI-CANCER AGENTS*
- **FLANAGAN et al.** *Bioorganic and Medicinal Chemistry,* 2014, 962-977
- *Cancer Chemotherapy and Pharmacology,* vol. 38 (4), 335-42